(19) Europäisches Patentamt
European Patent Office
Office européen des brevets

(11) EP 3 960 741 A1

(12) # EUROPEAN PATENT APPLICATION

published in accordance with Art. 153(4) EPC

(43) Date of publication:
**02.03.2022 Bulletin 2022/09**

(21) Application number: **20795447.0**

(22) Date of filing: **24.04.2020**

(51) International Patent Classification (IPC):
***C07D 471/04*** (2006.01) ***C07D 498/04*** (2006.01)
***C07D 519/00*** (2006.01) ***A61K 31/47*** (2006.01)
***A61K 31/4725*** (2006.01) ***A61K 31/4745*** (2006.01)
***A61P 31/12*** (2006.01) ***A61P 1/00*** (2006.01)

(52) Cooperative Patent Classification (CPC):
**A61K 31/47; A61K 31/4725; A61K 31/4745; A61P 1/00; A61P 31/12; C07D 471/04; C07D 498/04; C07D 519/00**

(86) International application number:
**PCT/CN2020/086837**

(87) International publication number:
**WO 2020/216350 (29.10.2020 Gazette 2020/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2019 CN 201910348714**

(71) Applicant: **Academy Of Military Medical Sciences
Haidian District
Beijing
100850 (CN)**

(72) Inventors:
- **ZHONG, Wu**
  **Beijing 100850 (CN)**
- **HAO, Tianlong**
  **Beijing 100850 (CN)**
- **CAO, Ruiyuan**
  **Beijing 100850 (CN)**
- **CHENG, Tong**
  **Xiamen, Fujian 361005 (CN)**
- **LI, Yuexiang**
  **Beijing 100850 (CN)**
- **FAN, Shiyong**
  **Beijing 100850 (CN)**
- **LI, Wei**
  **Beijing 100850 (CN)**
- **WANG, Shixu**
  **Beijing 100850 (CN)**
- **XIA, Ningshao**
  **Xiamen, Fujian 361005 (CN)**
- **LI, Song**
  **Beijing 100850 (CN)**

(74) Representative: **Inspicos P/S
Kogle Allé 2
2970 Hørsholm (DK)**

(54) **QUINOLINE COMPOUNDS AND PHARMACEUTICAL COMPOSITIONS AND USES THEREOF**

(57) Provided are quinoline derivative compounds of Formulae (I), (II) and (III) with an inhibitory effect on mTOR and applications of their pharmaceutically acceptable salts, their stereoisomers, their hydrates or their solvates in preparation of medicine for preventing and/or treating diseases caused by enteroviruses.

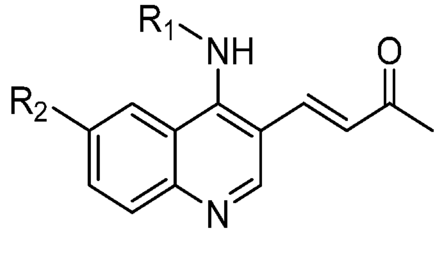

Formula I

EP 3 960 741 A1

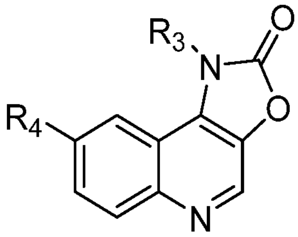

Formula II

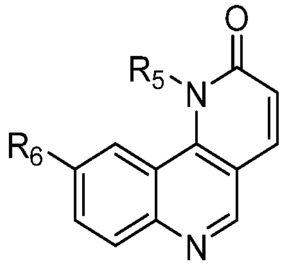

Formula III.

## Description

The present application is based on and claims the benefit of priority from Chinese application No. 201910348714.4, filed on April 26, 2019, the disclosures of which are incorporated herein by reference in its entirety.

## Technical Field

The present application relates to the field of medical technology, specifically to the quinoline derivative compounds represented by the following Formulas I, II and III and a pharmaceutically acceptable salt, a stereoisomer, a hydrate or a solvate thereof, and use thereof in the manufacture of a medicament for the prevention and/or first aid and/or treatment of a disease caused by enterovirus.

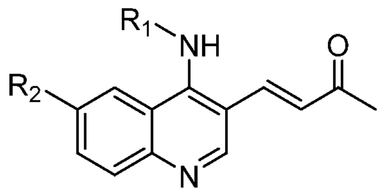

Formula I

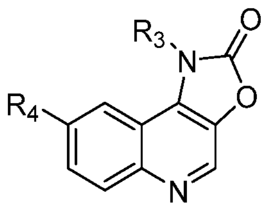

Formula II

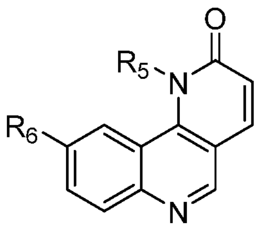

Formula III

## Background Art

Enterovirus 71 (EV71) is the main pathogen that causes severe hand-foot-and-mouth disease, belongs to the *Enterovirus* genus of the *Picornaviridae* family, is a non-enveloped positive-sense single-stranded RNA virus; it has a genome of 7.5 kb, encodes a polyprotein precursor of about 2200 amino acids, and can be further hydrolyzed to form 4 structural proteins (VP1 to VP4) and 7 non-structural proteins (2A to 2C and 3A to 3D), which play viral function together. EV71 generally replicates in the intestinal tract, and can also invade the central nervous system through the blood-brain barrier or through retrograde axon transport. Therefore, in addition to simple hand-foot-and-mouth disease in children with EV71 infection, the central nervous system is often involved and accompanied with severe complication. It is found through statistics that among the deaths from hand-foot-and-mouth disease, the proportion of EV71-infected children was >90%. Since 1969, the outbreaks of hand-foot- and-mouth disease caused by EV-71 are frequent. In recent years, a series of EV-71 epidemics in the Asia-Pacific region have aroused people's great attention. Traditional antiviral drugs mainly target the protein structure of the virus, but during the long-term treatment process, the virus will develop resistance through its own mutation.

In recent years, finding antiviral targets based on host cells has become a research hotspot. Intracellular signal

pathways can not only control the initiation of viral transcription, but also control the apoptosis of infected cells and cell autophagy. During the process of viral transcription and transmission, the signal pathways in host cells play an extremely important role.

Mammalian target of rapamycin (mTOR) is a serine/threonine protein kinase with high molecular weight (289 kDa). It has two subtypes mTORC1 (mTOR complex 1) and mTORC2 (mTOR complex 2). It has been found through studies that mTOR plays an important role in cell growth, apoptosis, and autophagy. After the virus infects host cells, mTOR is mainly activated from three aspects. On the first hand, through activation of phosphatidylinositol-3-kinase (PI3K), the virus destroys the balance of phosphatidylinositol-4,5-bisphosphate (PIP2) and phosphatidylinositol-3,4,5-triphosphate (PIP3) and promotes the conversion of PIP2 into PIP3, so that protein kinase B (Akt) and phosphoinositide-dependent kinase-1 (PDK1) move toward the cell membrane and PDK1 and Akt are activated. The activated Akt will affect the virus in two ways: ① accelerating cell's metabolism, growth, and material synthesis, and providing a material basis for the large-scale synthesis of viral proteins; ②continuously activating downstream mTORC1, and then through the action of the downstream factors eukaryotic initiation factor 4E binding protein 1 (4EBP1) and eukaryotic translation initiation factor 4E (eIF4E), binding mRNA cap structure to viral RNA polymerase to promote the initiation of viral transcription. On the second hand, through the activation of the Raf-MEK-ERK pathway, the virus can enhance the PI3K-Akt-mTOR signal to promote the activation of mTORC1. One the third hand, after the virus infects host cells, the acceleration of intracellular metabolism will lead to a lack of energy and oxygen, which activates the expression of regulated in development and DNA damage response 1 (REDD1) and AMP-activated protein kinase (AMPK) and inhibits the activation of mTORC1. In order to promote self-replication, through the inhibition of the translation process of mRNA of host cell, the virus inhibits the expression of REDD1 and AMPK genes and reduces the host cell's autonomous defense effect against the virus. Therefore, the purpose of suppressing the virus can be achieved by inhibiting the mTOR protein of the host cell.

In the present application, three types of new compounds with different structures were synthesized on the basis of quinoline core design, and the detection of inhibitory activity on mTOR kinase and in vitro activity of anti-EV71 virus was carried out, in which some of the compounds showed good inhibitory activity on mTOR kinase and in vitro activity of anti-EV71 virus, and exhibited a certain application prospect.

**Contents of the Application**

The purpose of the present application is to disclose a new quinoline compound, a pharmaceutically acceptable salt, and its application in the treatment of anti-enterovirus.

The technical solution of the present application is as follows.

The first aspect of the present application provides a compound represented by Formula I, a pharmaceutically acceptable salt, a stereoisomer, a hydrate or a solvate thereof,

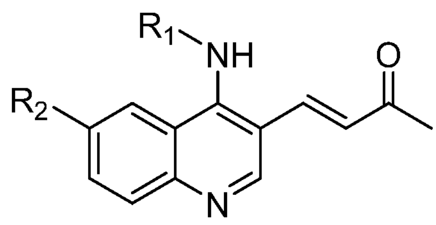

Formula I

wherein:

$R_1$ is $C_{1-10}$ alkyl, 3- to 14-membered substituted or unsubstituted cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 6-to 14-membered substituted or unsubstituted heteroaryl;

$R_2$ is hydrogen, or $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 7- to 12-membered substituted or unsubstituted bridged ring group, amino, 6- to 14-membered substituted or unsubstituted arylimino.

Another aspect of the present application provides a compound represented by Formula II, a pharmaceutically

acceptable salt, a stereoisomer, a hydrate or a solvate thereof,

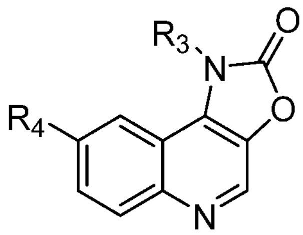

Formula II

wherein:

$R_3$ is $C_{1-10}$ alkyl, 3- to 14-membered substituted or unsubstituted cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 6-to 14-membered substituted or unsubstituted heteroaryl;

$R_4$ is hydrogen, or $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 7- to 12-membered substituted or unsubstituted bridged ring group, amino, 6- to 14-membered substituted or unsubstituted arylimino.

Another aspect of the application provides a compound represented by Formula III, a pharmaceutically acceptable salt, a stereoisomer, a hydrate or a solvate thereof,

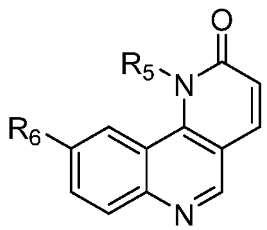

Formula III

$R_5$ is $C_{1-10}$ alkyl, 3- to 14-membered substituted or unsubstituted cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 6-to 14-membered substituted or unsubstituted heteroaryl;

$R_6$ is hydrogen, or $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 7- to 12-membered substituted or unsubstituted bridged ring group, amino, 6- to 14-membered substituted or unsubstituted arylimino.

In some embodiments, the compound of Formula III is not

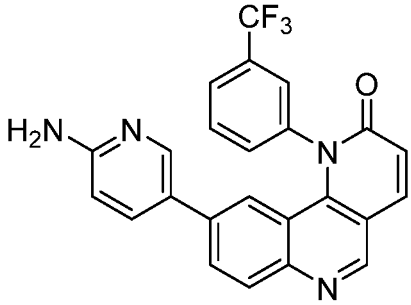

.

In some embodiments, in the compound of Formula I described in the present application, $R_1$ can be optionally substituted with one or more $R^a$, and each $R^a$ is independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino)), halogen or amino.

In some embodiments, in the compound of Formula I described in the present application, $R_1$ is 5- to 6-membered cycloalkyl, 5- to 6-membered heterocyclyl, 5- to 6-membered aryl or 5-to 6-membered heteroaryl, $R_1$ can be optionally substituted by one or more $R^a$, each $R^a$ is independently hydrogen, $C_{1-6}$ alkyl, $C_{1-6}$ haloalkyl, $C_{1-6}$ alkoxy, alkyl-amino (e.g. $C_{1-6}$ alkyl-amino), hydroxy, nitro, cyano, $C_{1-6}$ alkylthio, dialkyl-amino (e.g. di($C_{1-6}$ alkyl)-amino), halogen or amino.

In some embodiments, in the compound of Formula I described in the present application, $R_1$ is phenyl, and $R_1$ can be optionally substituted with one or more $R^a$, and each $R^a$ is independently hydrogen, trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino.

In some embodiments, in the compound of Formula I described in the present application, each $R^a$ is independently hydrogen, trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino.

In some embodiments, in the compound of Formula I described in the present application, each $R^a$ is independently trifluoromethyl.

In some embodiments, in the compound of Formula I described in the present application, each $R^a$ is independently methyl.

In some embodiments, in the compound of Formula I described in the present application, each $R^a$ is independently ethyl.

In some embodiments, in the compound of Formula I described in the present application, each $R^a$ is independently n-propyl.

In some embodiments, in the compound of Formula I described in the present application, each $R^a$ is independently isopropyl.

In some embodiments, in the compound of Formula I described in the present application, each $R^a$ is independently n-butyl.

In some embodiments, in the compound of Formula I described in the present application, each $R^a$ is independently methoxy.

In some embodiments, in the compound of Formula I described in the present application, each $R^a$ is independently ethoxy.

In some embodiments, in the compound of Formula I described in the present application, each $R^a$ is independently propoxy.

In some embodiments, in the compound of Formula I described in the present application, each $R^a$ is independently methylamino.

In some embodiments, in the compound of Formula I described in the present application, each $R^a$ is independently ethylamino.

In some embodiments, in the compound of Formula I described in the present application, each $R^a$ is independently dimethylamino.

In some embodiments, in the compound of Formula I described in the present application, each $R^a$ is independently diethylamino.

In some embodiments, in the compound of Formula I described in the present application, each $R^a$ is independently hydroxyl.

In some embodiments, in the compound of Formula I described in the present application, each $R^a$ is independently nitro.

In some embodiments, in the compound of Formula I described in the present application, each $R^a$ is independently cyano.

In some embodiments, in the compound of Formula I described in the present application, each $R^a$ is independently methylthio.

In some embodiments, in the compound of Formula I described in the present application, each $R^a$ is independently ethylthio.

In some embodiments, in the compound of Formula I described in the present application, each $R^a$ is independently fluorine.

In some embodiments, in the compound of Formula I described in the present application, each $R^a$ is independently chlorine.

In some embodiments, in the compound of Formula I described in the present application, each $R^a$ is independently bromine.

In some embodiments, in the compound of Formula I described in the present application, each $R^a$ is independently iodine.

In some embodiments, in the compound of Formula I described in the present application, each $R^a$ is independently amino.

In some embodiments, in the compounds of Formula I described in the present application, $R_1$ is

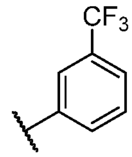

.

In some embodiments, in the compound of Formula I described in the present application, $R_2$ is optionally substituted with one or more $R^d$, and each $R^d$ is independently $C_{1-6}$ alkyl, $C_{1-6}$ haloalkyl, $C_{1-6}$ alkoxy, alkyl-amino, hydroxyl, nitro, cyano, $C_{1-6}$ alkylthio, dialkyl-amino, halogen, amino, $NH_2C(O)$-, R'OC(O)NH-, wherein R' is benzyl, phenyl, or $C_{1-6}$ alkyl.

In some embodiments, in the compound of Formula I described in the present application, $R_2$ is pyridyl, phenyl, furyl, imidazolyl, pyrazolyl, thienyl, or quinolyl.

In some embodiments, in the compound of Formula I described in the present application, $R_2$ is pyridyl, phenyl, furyl, imidazolyl, pyrazolyl, thienyl, or quinolyl, and $R_2$ is optionally substituted by one or more $R^d$, each $R^d$ is independently $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen, amino, $NH_2C(O)$-, R'OC(O)NH-, wherein R' is benzyl, phenyl or $C_{1-6}$ alkyl.

In some embodiments, in the compound of Formula I described in the present application, $R_2$ is pyridyl, phenyl, furyl, pyrazolyl, thienyl, quinolyl, and $R_2$ is optionally substituted with one or more $R^d$, each $R^d$ is independently $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen, amino, $NH_2C(O)$-, R'OC(O)NH-, wherein R' is benzyl, phenyl or $C_{1-6}$ alkyl.

In some embodiments, in the compound of Formula I described in the present application, each $R^d$ is independently trifluoromethyl, methyl, ethyl, n-propyl, isopropyl, n-butyl, isobutyl, sec-butyl, tert-butyl, n-pentyl, n-hexyl, methoxy, ethoxy, propoxy, methylamino, ethylamino, dimethylamino, diethylamino, methylthio, ethylthio, fluorine, chlorine, bromine, iodine, hydroxyl, nitro, cyano, amino, $NH_2C(O)$-, R'OC(O)NH-, wherein R' is defined as described in the present application.

In some embodiments, in the compound of Formula I described in the present application, $R_2$ is pyrazolyl,

, 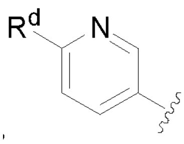 , 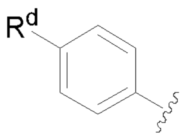 ,

wherein the definition of $R^d$ is as described in the present application.

In some embodiments, in the compound of Formula I described in the present application, $R_2$ is pyridyl, and $R_2$ can be optionally substituted with one or more $R^d$, wherein $R^d$ is defined as described in the present application.

In some embodiments, in the compound of Formula I described in the present application, $R_2$ is phenyl, and $R_2$ can be optionally substituted with one or more $R^d$, wherein $R^d$ is defined as described in the present application.

In some embodiments, in the compound of Formula I described in the present application, $R_2$ is furyl, and $R_2$ can be optionally substituted with one or more $R^d$, wherein $R^d$ is defined as described in the present application.

In some embodiments, in the compound of Formula I described in the present application, $R_2$ is pyrazolyl, and $R_2$ can be optionally substituted with one or more $R^d$, wherein $R^d$ is defined as described in the present application.
In some embodiments, in the compound of Formula I described in the present application, $R_2$ is thienyl, and $R_2$ can be optionally substituted with one or more $R^d$, wherein $R^d$ is defined as described in the present application.
In some embodiments, in the compound of Formula I described in the present application, $R_2$ is quinolyl, and $R_2$ can be optionally substituted with one or more $R^d$, wherein $R^d$ is defined as described in the present application.
In some embodiments, in the compound of Formula I described in the present application, R2 is

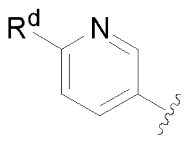,

wherein $R^d$ is as described in the present application.
In some embodiments, in the compound of Formula I described in the present application, R2 is

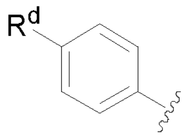,

wherein $R^d$ is as described in the present application.
In some embodiments, in the compound of Formula I described in the present application, each $R^d$ is independently $C_{1-6}$ alkyl.
In some embodiments, in the compound of Formula I described in the present application, each $R^d$ is independently $C_{1-6}$ haloalkyl.
In some embodiments, in the compound of Formula I described in the present application, each $R^d$ is independently a $C_{1-6}$ alkoxy.
In some embodiments, in the compound of Formula I described in the present application, each $R^d$ is independently alkyl-amino (e.g., $C_{1-6}$ alkyl-amino).
In some embodiments, in the compound of Formula I described in the present application, each $R^d$ is independently hydroxyl.
In some embodiments, in the compound of Formula I described in the present application, each $R^d$ is independently nitro.
In some embodiments, in the compound of Formula I described in the present application, each $R^d$ is each independently cyano.
In some embodiments, in the compound of Formula I described in the present application, each $R^d$ is independently $C_{1-6}$ alkylthio.
In some embodiments, in the compound of Formula I described in the present application, each $R^d$ is each independently a dialkyl-amino group (e.g., di($C_{1-6}$ alkyl)-amino group).
In some embodiments, in the compound of Formula I described in the present application, each $R^d$ is independently halogen, such as fluorine, chlorine, bromine, or iodine.
In some embodiments, in the compound of Formula I described in the present application, each $R^d$ is independently amino.
In some embodiments, in the compound of Formula I described in the present application, each $R^d$ is independently $NH_2C(O)$-.
In some embodiments, in the compound of Formula I described in the present application, each $R^d$ is independently R'OC(O)NH-, wherein R' is benzyl, phenyl, or $C_{1-6}$ alkyl.
In some embodiments, in the compound of Formula I described in the present application, each $R^d$ is independently benzyl-OC(O)NH-.
In some embodiments, in the compound of Formula I described in the present application, each $R^d$ is independently phenyl-OC(O)NH-.
In some embodiments, in the compound of Formula I described in the present application, each $R^d$ is independently $C_{1-6}$ alkyl-OC(O)NH-.
In some embodiments, in the compound of Formula I described in the present application, each $R^d$ is independently trifluoromethyl.

In some embodiments, in the compound of Formula I described in the present application, each $R^d$ is independently methyl.

In some embodiments, in the compound of Formula I described in the present application, each $R^d$ is independently ethyl.

In some embodiments, in the compound of Formula I described in the present application, each $R^d$ is independently n-propyl.

In some embodiments, in the compound of Formula I described in the present application, each $R^d$ is independently isopropyl.

In some embodiments, in the compound of Formula I described in the present application, each $R^d$ is independently n-butyl.

In some embodiments, in the compound of Formula I described in the present application, each $R^d$ is independently isobutyl.

In some embodiments, in the compound of Formula I described in the present application, each $R^d$ is each independently sec-butyl.

In some embodiments, in the compound of Formula I described in the present application, each $R^d$ is independently tert-butyl.

In some embodiments, in the compound of Formula I described in the present application, each $R^d$ is independently n-pentyl.

In some embodiments, in the compound of Formula I described in the present application, each $R^d$ is independently n-hexyl.

In some embodiments, in the compound of Formula I described in the present application, each $R^d$ is each independently methoxy.

In some embodiments, in the compound of Formula I described in the present application, each $R^d$ is independently ethoxy.

In some embodiments, in the compound of Formula I described in the present application, each $R^d$ is independently propoxy.

In some embodiments, in the compound of Formula I described in the present application, each $R^d$ is independently methylamino.

In some embodiments, in the compound of Formula I described in the present application, each $R^d$ is independently ethylamino.

In some embodiments, in the compound of Formula I described in the present application, each $R^d$ is independently dimethylamino.

In some embodiments, in the compound of Formula I described in the present application, each $R^d$ is independently diethylamino.

In some embodiments, in the compound of Formula I described in the present application, each $R^d$ is independently methylthio.

In some embodiments, in the compound of Formula I described in the present application, each $R^d$ is independently ethylthio.

In some embodiments, in the compound of Formula I described in the present application, each $R^d$ is independently fluorine.

In some embodiments, in the compound of Formula I described in the present application, each $R^d$ is independently chlorine.

In some embodiments, in the compound of Formula I described in the present application, each $R^d$ is independently bromine.

In some embodiments, in the compound of Formula I described in the present application, each $R^d$ is independently iodine.

In some embodiments, in the compound of Formula I described in the present application, R2 is

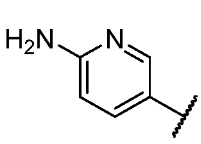 , 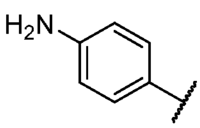 , 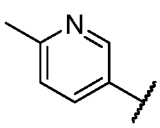 , 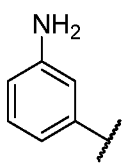 , 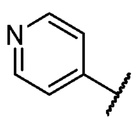 , 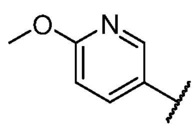 ,

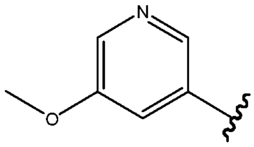, 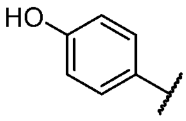, 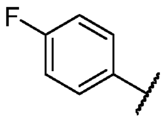, 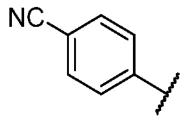, 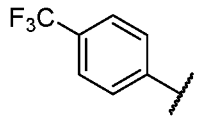,

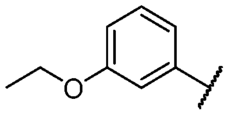, 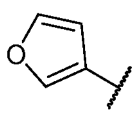, 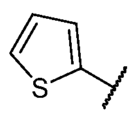, 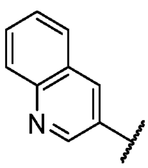, 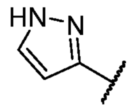, 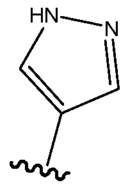, 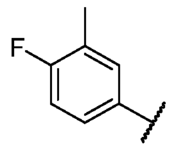,

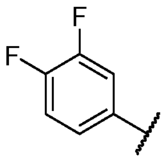, 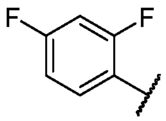, 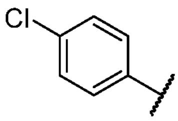, 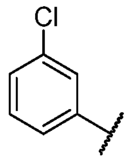, 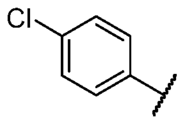, 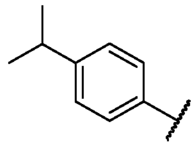,

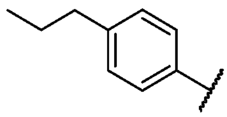, 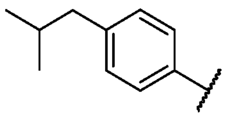, 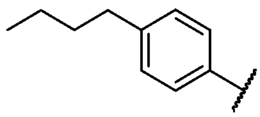, 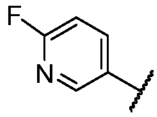, 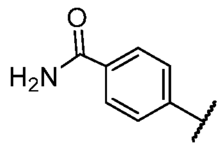,

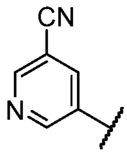, or 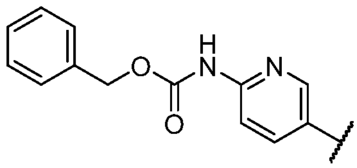.

In some embodiments, in the compound of Formula I described in the present application, $R_2$ is

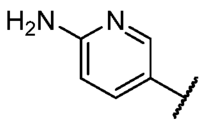, 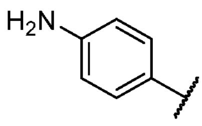, 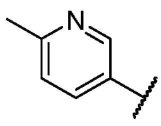, 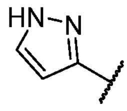,

or

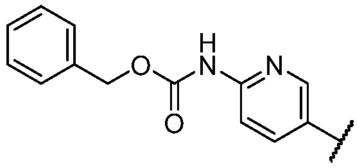.

In some embodiments, in the compound of Formula II described in the present application, $R_3$ can be optionally substituted with one or more $R^b$, and each $R^b$ is independently hydrogen, $C_{1-6}$ alkyl, $C_{1-6}$ haloalkyl, $C_{1-6}$ alkoxy, alkyl-amino, hydroxy, nitro, cyano, $C_{1-6}$ alkylthio, dialkyl-amino, halogen or amino.

In some embodiments, in the compound of Formula II described in the present application, $R_3$ is 5- to 6-membered cycloalkyl, 5- to 6-membered heterocyclyl, 5- to 6-membered aryl or 5-to 6-membered heteroaryl, $R_3$ can be optionally substituted with one or more $R^b$, each $R^b$ is independently hydrogen, $C_{1-6}$ alkyl, $C_{1-6}$ haloalkyl, $C_{1-6}$ alkoxy, alkyl-amino (e.g. $C_{1-6}$ alkyl-amino-), hydroxy, nitro, cyano, $C_{1-6}$ alkylthio, dialkyl-amino (e.g., di($C_{1-6}$ alkyl)-amino-), hal-

ogen or amino.

In some embodiments, in the compound of Formula II described in the present application, $R_3$ is phenyl, $R_3$ can be optionally substituted with one or more $R^b$, and each $R^b$ is independently hydrogen, trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino.

In some embodiments, in the compound of Formula II described in the present application, each $R^b$ is independently trifluoromethyl.

In some embodiments, in the compound of Formula II described in the present application, each $R^b$ is independently methyl.

In some embodiments, in the compound of Formula II described in the present application, each $R^b$ is independently ethyl.

In some embodiments, in the compound of Formula II described in the present application, each $R^b$ is independently n-propyl.

In some embodiments, in the compound of Formula II described in the present application, each $R^b$ is independently isopropyl.

In some embodiments, in the compound of Formula II described in the present application, each $R^b$ is independently n-butyl.

In some embodiments, in the compound of Formula II described in the present application, each $R^b$ is independently methoxy.

In some embodiments, in the compound of Formula II described in the present application, each $R^b$ is independently ethoxy.

In some embodiments, in the compound of Formula II described in the present application, each $R^b$ is independently propoxy.

In some embodiments, in the compound of Formula II described in the present application, each $R^b$ is independently methylamino.

In some embodiments, in the compound of Formula II described in the present application, each $R^b$ is independently ethylamino.

In some embodiments, in the compound of Formula II described in the present application, each $R^b$ is independently dimethylamino.

In some embodiments, in the compound of Formula II described in the present application, each $R^b$ is independently diethylamino.

In some embodiments, in the compound of Formula II described in the present application, each $R^b$ is independently hydroxyl.

In some embodiments, in the compound of Formula II described in the present application, each $R^b$ is independently nitro.

In some embodiments, in the compound of Formula II described in the present application, each $R^b$ is independently cyano.

In some embodiments, in the compound of Formula II described in the present application, each $R^b$ is independently methylthio.

In some embodiments, in the compound of Formula II described in the present application, each $R^b$ is independently ethylthio.

In some embodiments, in the compound of Formula II described in the present application, each $R^b$ is independently fluorine.

In some embodiments, in the compound of Formula II described in the present application, each $R^b$ is independently chlorine.

In some embodiments, in the compound of Formula II described in the present application, each $R^b$ is independently bromine.

In some embodiments, in the compound of Formula II described in the present application, each $R^b$ is independently iodine.

In some embodiments, in the compound of Formula II described in the present application, each $R^b$ is independently amino.

In some embodiments, in the Formula II described in the present application, $R_3$ is

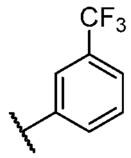

.

In some embodiments, in the compound of Formula II described in the present application, $R_4$ is optionally substituted with one or more $R^e$, and each $R^e$ is independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxyl, nitro, cyano, $C_{1-6}$ alkylthio, dialkyl-amino (e.g., di($C_{1-6}$ alkyl)-amino), halogen, amino, $NH_2C(O)$-, $NH_2C(O)NH$-, R'OC(O)- (wherein R' is benzyl, phenyl, or $C_{1-6}$ alkyl), R"OC(O)NH- (wherein R" is benzyl, phenyl, or $C_{1-6}$ alkyl), R"'C(O)NH- (wherein R'" is benzyl, phenyl, or $C_{1-6}$ alkyl).

In some embodiments, in the compound of Formula II described in the present application, $R_4$ is pyridyl, phenyl, furyl, imidazolyl, pyrazolyl, thienyl, quinolyl, vinyl, propenyl, or butenyl.

In some embodiments, in the compound of Formula II described in the present application, $R_4$ is pyridyl, phenyl, furyl, imidazolyl, pyrazolyl, thienyl, quinolyl, vinyl, propenyl, or butenyl, $R_4$ is optionally substituted by one or more $R^e$, each $R^e$ is independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen, amino, $NH_2C(O)$-, $NH_2C(O)NH$-, R'OC(O)- (wherein R' is benzyl, phenyl, or $C_{1-6}$ alkyl), R"OC(O)NH- (wherein R" is benzyl, phenyl, or $C_{1-6}$ alkyl), R"'C(O)NH- (wherein R'" is benzyl, phenyl, or $C_{1-6}$ alkyl).

In some embodiments, in the compound of Formula II described in the present application, $R_4$ is pyridyl, phenyl, furyl, pyrazolyl, thienyl, quinolyl, vinyl, propenyl, or butenyl, and $R_4$ is optionally substituted by one or more $R^e$, each $R^e$ is independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen, amino, $NH_2C(O)$-, $NH_2C(O)NH$-, R'OC(O)-(wherein R' is benzyl, phenyl, or $C_{1-6}$ alkyl), R"OC(O)NH- (wherein R" is benzyl, phenyl, or $C_{1-6}$ alkyl), R"'C(O)NH- (wherein R'" is benzyl, phenyl, or $C_{1-6}$ alkyl).

In some embodiments, in the compound of Formula II described in the present application, each $R^e$ is independently hydrogen, trifluoromethyl, methyl, ethyl, n-propyl, isopropyl, n-butyl, isobutyl, sec-butyl, tert-butyl, n-pentyl, n-hexyl, methoxy, ethoxy, propoxy, methylamino, ethylamino, dimethylamino, diethylamino, methylthio, ethylthio, fluorine, chlorine, bromine, iodine, hydroxyl, nitro, cyano, amino, $NH_2C(O)$-, $NH_2C(O)NH$-, R'OC(O)-, R"OC(O)NH-, R"'C(O)NH-, wherein R', R", R'" are defined as described in the present application.

In some embodiments, in the compound of Formula II described in the present application, $R_4$ is

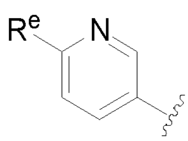 , 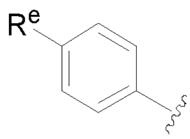 ,

wherein $R^e$ is as described in the present application.

In some embodiments, in the compound of Formula II described in the present application, $R_4$ is pyridyl, and $R_4$ can be optionally substituted with one or more $R^e$, wherein $R^e$ is defined as described in the present application.

In some embodiments, in the compound of Formula II described in the present application, $R_4$ is phenyl, and $R_4$ can be optionally substituted with one or more $R^e$, wherein $R^e$ is defined as described in the present application.

In some embodiments, in the compound of Formula II described in the present application, $R_4$ is furyl, and $R_4$ can be optionally substituted with one or more $R^e$, wherein $R^e$ is defined as described in the present application.

In some embodiments, in the compound of Formula II described in the present application, $R_4$ is pyrazolyl, and $R_4$ can be optionally substituted with one or more $R^e$, wherein $R^e$ is defined as described in the present application.

In some embodiments, in the compound of Formula II described in the present application, $R_4$ is thienyl, and $R_4$ can be optionally substituted with one or more $R^e$, wherein $R^e$ is defined as described in the present application.

In some embodiments, in the compound of Formula II described in the present application, $R_4$ is quinolyl, and $R_4$ can be optionally substituted with one or more $R^e$, wherein $R^e$ is defined as described in the present application.

In some embodiments, in the compound of Formula II described in the present application, $R_4$ is vinyl, and $R_4$ can be optionally substituted with one or more $R^e$, wherein $R^e$ is defined as described in the present application.

In some embodiments, in the compound of Formula II described in the present application, $R_4$ is propenyl, and $R_4$ can be optionally substituted with one or more $R^e$, wherein $R^e$ is defined as described in the present application.

In some embodiments, in the compound of Formula II described in the present application, $R_4$ is butenyl, and $R_4$ can be optionally substituted with one or more $R^e$, wherein $R^e$ is defined as described in the present application.

In some embodiments, in the compound of Formula II described in the present application, each $R^e$ is independently hydrogen.

In some embodiments, in the compound of Formula II described in the present application, each $R^e$ is independently $C_{1-6}$ alkyl.

In some embodiments, in the compound of Formula II described in the present application, each $R^e$ is independently $C_{1-6}$ haloalkyl.

In some embodiments, in the compound of Formula II described in the present application, each $R^e$ is independently $C_{1-6}$ alkoxy.

In some embodiments, in the compound of Formula II described in the present application, each $R^e$ is independently alkyl-amino (e.g., $C_{1-6}$ alkyl-amino).

In some embodiments, in the compound of Formula II described in the present application, each $R^e$ is independently hydroxyl.

In some embodiments, in the compound of Formula II described in the present application, each $R^e$ is independently nitro.

In some embodiments, in the compound of Formula II described in the present application, each $R^e$ is independently cyano.

In some embodiments, in the compound of Formula II described in the present application, each $R^e$ is independently $C_{1-6}$ alkylthio.

In some embodiments, in the compound of Formula II described in the present application, each $R^e$ is independently dialkyl-amino (e.g., di($C_{1-6}$ alkyl)-amino).

In some embodiments, in the compound of Formula II described in the present application, each $R^e$ is independently halogen.

In some embodiments, in the compound of Formula II described in the present application, each $R^e$ is independently amino.

In some embodiments, in the compound of Formula II described in the present application, each $R^e$ is independently $NH_2C(O)$-.

In some embodiments, in the compound of Formula II described in the present application, each $R^e$ is independently $NH_2C(O)NH$-.

In some embodiments, in the compound of Formula II described in the present application, each $R^e$ is independently R'OC(O)-, wherein R' is defined as described in the present application.

In some embodiments, in the compound of Formula II described in the present application, each $R^e$ is independently R"OC(O)NH-, wherein R" is defined as described in the present application.

In some embodiments, in the compound of Formula II described in the present application, each $R^e$ is independently R'"C(O)NH-, wherein R'" is defined as described in the present application.

In some embodiments, in the compound of Formula II described in the present application, R', R" and R'" are each independently benzyl.

In some embodiments, in the compound of Formula II described in the present application, R', R" and R'" are each independently phenyl.

In some embodiments, in the compound of Formula II described in the present application, R', R" and R'" are each independently $C_{1-6}$ alkyl.

In some embodiments, in the compound of Formula II described in the present application, R', R" and R'" are each independently methyl, ethyl, n-propyl, isopropyl, n-butyl, iso-butyl, sec-butyl, tert-butyl, n-pentyl, n-hexyl.

In some embodiments, in the compound of Formula II described in the present application, R', R" and R'" are each independently methyl.

In some embodiments, in the compound of Formula II described in the present application, R', R" and R'" are each independently ethyl.

In some embodiments, in the compound of Formula II described in the present application, R', R" and R'" are each independently n-propyl.

In some embodiments, in the compound of Formula II described in the present application, R', R" and R'" are each independently isopropyl.

In some embodiments, in the compound of Formula II described in the present application, R', R" and R'" are each independently n-butyl.

In some embodiments, in the compound of Formula II described in the present application, R', R" and R'" are each independently isobutyl.

In some embodiments, in the compound of Formula II described in the present application, R', R" and R'" are each independently sec-butyl.

In some embodiments, in the compound of Formula II described in the present application, R', R" and R'" are each independently tert-butyl.

In some embodiments, in the compound of Formula II described in the present application, R', R" and R'" are each independently n-pentyl.

In some embodiments, in the compound of Formula II described in the present application, R', R" and R'" are each independently n-hexyl.

In some embodiments, in Formula II described in the present application, $R_4$ is

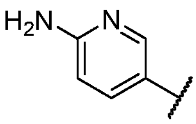, 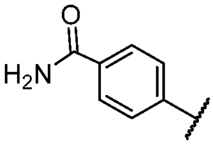, 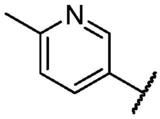, 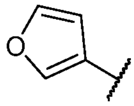, 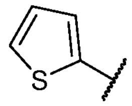, 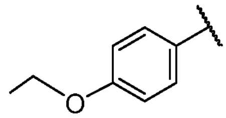,

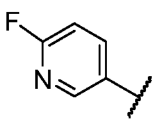, 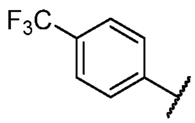, 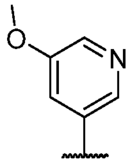, 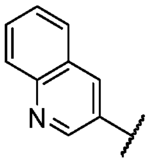, 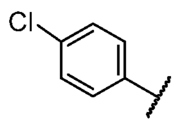, 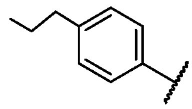,

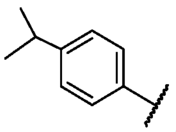, 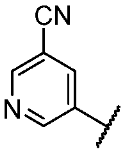, 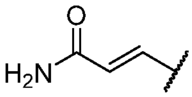, 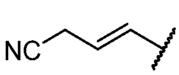, 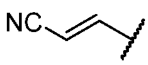, 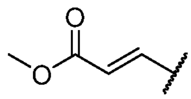,

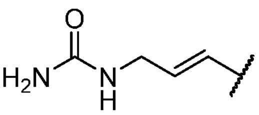, 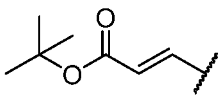, 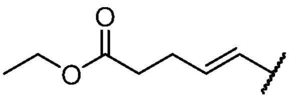, 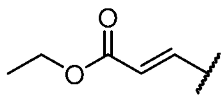,

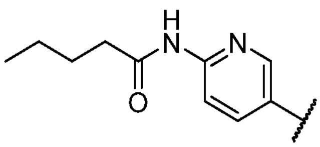, or 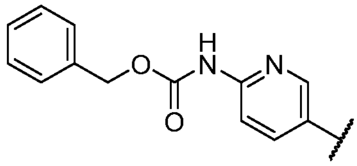.

In some embodiments, in Formula II described in the present application, $R_4$ is

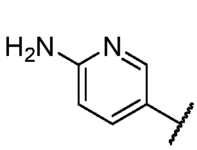, 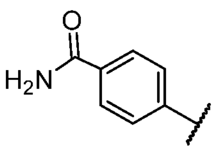, 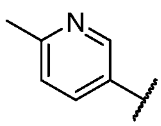, or 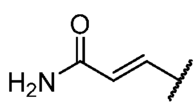.

In some embodiments, in Formula II described in the present application, $R_4$ is

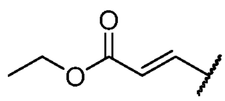.

In some embodiments, in the compound of Formula III described in the present application, $R_5$ can be optionally substituted with one or more $R^c$, and each $R^c$ is independently $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen or amino.

In some embodiments, in the compound of Formula III described in the present application, $R_5$ is 5- to 6-membered cycloalkyl, 5- to 6-membered heterocyclyl, 5- to 6-membered aryl or 5-to 6-membered heteroaryl, $R_5$ can be

optionally substituted by one or more $R^c$, each $R^c$ is independently $C_{1-6}$ alkyl, $C_{1-6}$ haloalkyl, $C_{1-6}$ alkoxy, alkyl-amino (e.g. $C_{1-6}$ alkyl-amino), hydroxy, nitro, cyano, $C_{1-6}$ alkylthio, dialkyl-amino (e.g. di($C_{1-6}$ alkyl)-amino), halogen or amino.

In some embodiments, in the compound of Formula III described in the present application, $R_5$ is phenyl, $R_5$ can be optionally substituted with one or more $R^c$, and each $R^c$ is independently trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino.

In some embodiments, in the compound of Formula III described in the present application, each $R^c$ is independently trifluoromethyl.

In some embodiments, in the compound of Formula III described in the present application, each $R^c$ is independently methyl.

In some embodiments, in the compound of Formula III described in the present application, each $R^c$ is independently ethyl.

In some embodiments, in the compound of Formula III described in the present application, each $R^c$ is independently n-propyl.

In some embodiments, in the compound of Formula III described in the present application, each $R^c$ is independently isopropyl.

In some embodiments, in the compound of Formula III described in the present application, each $R^c$ is independently n-butyl.

In some embodiments, in the compound of Formula III described in the present application, each $R^c$ is independently methoxy.

In some embodiments, in the compound of Formula III described in the present application, each $R^c$ is independently ethoxy.

In some embodiments, in the compound of Formula III described in the present application, each $R^c$ is independently propoxy.

In some embodiments, in the compound of Formula III described in the present application, each $R^c$ is independently methylamino.

In some embodiments, in the compound of Formula III described in the present application, each $R^c$ is independently ethylamino.

In some embodiments, in the compound of Formula III described in the present application, each $R^c$ is independently dimethylamino.

In some embodiments, in the compound of Formula III described in the present application, each $R^c$ is independently diethylamino.

In some embodiments, in the compound of Formula III described in the present application, each $R^c$ is independently hydroxyl.

In some embodiments, in the compound of Formula III described in the present application, each $R^c$ is independently nitro.

In some embodiments, in the compound of Formula III described in the present application, each $R^c$ is independently cyano.

In some embodiments, in the compound of Formula III described in the present application, each $R^c$ is independently methylthio.

In some embodiments, in the compound of Formula III described in the present application, each $R^c$ is independently ethylthio.

In some embodiments, in the compound of Formula III described in the present application, each $R^c$ is independently fluorine.

In some embodiments, in the compound of Formula III described in the present application, each $R^c$ is independently chlorine.

In some embodiments, in the compound of Formula III described in the present application, each $R^c$ is independently bromine.

In some embodiments, in the compound of Formula III described in the present application, each $R^c$ is independently iodine.

In some embodiments, in the compound of Formula III described in the present application, each $R^c$ is independently amino.

In some embodiments, in Formula III described in the present application, $R_5$ is

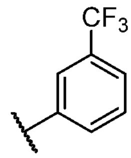.

In some embodiments, in the compound of Formula III described in the present application, $R_6$ is

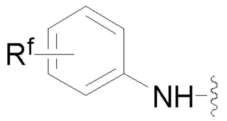, 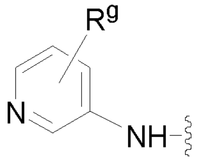, 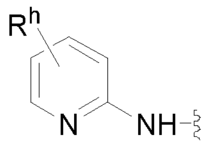, or 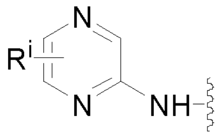,

Wherein $R^f$, $R^g$, $R^h$, $R^i$ are each independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxyl, nitro, cyano, $C_{1-6}$ alkylthio, dialkyl-amino (e.g., di($C_{1-6}$ alkyl)-amino), halogen, amino, or $C_{1-6}$ alkyl-C(O)NH-; or

$R_6$ is pyridyl, phenyl, quinolyl, 2-oxoindolyl, pyrimidyl, isoxazolyl, 1,4-dioxaspiro[4.5]dec-7-ene group, or pyrazolyl, $R_6$ is any optionally substituted by one or more $R^j$, each $R^j$ is independently hydrogen, $C_{1-6}$ alkyl, $C_{1-6}$ alkoxy, halogen, alkyl-amino (e.g., $C_{1-6}$ alkyl-amino), dialkyl-amino (e.g., di($C_{1-6}$ alkyl)-amino), 4-methyl-piperazinyl, morpholinyl, amino, $R^k$-C(O)NH- (wherein $R^k$- is benzyl, phenyl, p-methoxybenzyl, phenoxy or $C_{1-6}$ alkyl), pyrrolidinyl, allylamino or propargylamino; or

$R_6$ is $C_{2-6}$ alkenyl, $R_6$ is optionally substituted by one or more $R^m$, each $R^m$ is independently $NH_2C(O)$-, $NH_2C(O)NH$-, $R^n$-OC(O)- (wherein $R^n$ is $C_{1-6}$ alkyl).

In some embodiments, in the compound of Formula III described in the present application, $R_6$ is

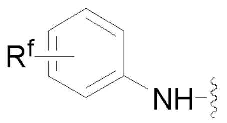, 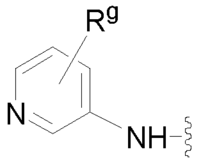, 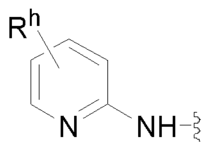, or 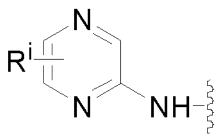,

wherein, $R^f$, $R^g$, $R^h$, and $R^i$ are each independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxyl, nitro, cyano, $C_{1-6}$ alkylthio, dialkyl-amino (e.g. di($C_{1-6}$ alkyl)-amino), halogen, amino, $C_{1-6}$ alkyl-C(O)NH-.

In some embodiments, in the compound of Formula III described in the present application, $R^f$, $R^g$, $R^h$ and $R^i$ are each independently hydrogen, trifluoromethyl, methyl, ethyl, n-propyl, isopropyl, n-butyl, isobutyl, sec-butyl, tert-butyl, n-pentyl, n-hexyl, methoxy, ethoxy, propoxy, methylamino, ethylamino, dimethylamino, diethylamino, methylthio, ethylthio, fluorine, chlorine, bromine, iodine, hydroxyl, nitro, cyano, amino, $CH_3$-C(O)NH-, $C_2H_5$-C(O)NH-, or $CH_3(CH_2)_2$-C(O)NH-.

In some embodiments, in the compound of Formula III described in the present application, $R_6$ is

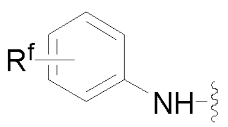,

wherein $R^f$ is defined as described in the present application.

In some embodiments, in the compound of Formula III described in the present application, $R_6$ is

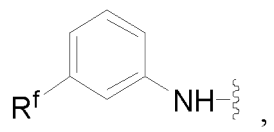

wherein $R^f$ is defined as described in the present application.

In some embodiments, in the compound of Formula III described in the present application, $R^f$ is amino, $C_{1-6}$ alkyl-amino, di($C_{1-6}$ alkyl)-amino, or $C_{1-6}$ alkyl-C(O)NH-.

In some embodiments, in the compound of Formula III described in the present application, $R^f$ is amino.

In some embodiments, in the compound of Formula III described in the present application, $R^f$ is $C_{1-6}$ alkyl-C(O)NH-.

In some embodiments, in the compound of Formula III described in the present application, $R^f$ is $C_{1-6}$ alkyl-amino.

In some embodiments, in the compound of Formula III described in the present application, $R^f$ is di($C_{1-6}$ alkyl)-amino.

In some embodiments, in the compound of Formula III described in the present application, $R^f$ is amino, $CH_3$-C(O)NH-, $C_2H_5$-C(O)NH-, or $CH_3(CH_2)_2$-C(O)NH-.

In some embodiments, in the compound of Formula III described in the present application, $R^f$ is $C_2H_5$-C(O)NH-.

In some embodiments, in the compound of Formula III described in the present application, $R^f$ is $CH_3(CH_2)_2$-C(O)NH-.

In some embodiments, in the compound of Formula III described in the present application, $R^f$ is amino or $CH_3$-C(O)NH-.

In some embodiments, in the compound of Formula III described in the present application, $R^f$ is amino, $C_{1-4}$ alkyl-amino, di($C_{1-4}$ alkyl)-amino, or $C_{1-6}$ alkyl-C(O)NH-.

In some embodiments, in the compound of Formula III described in the present application, $R^f$ is amino, methylamino, ethylamino, dimethylamino, or diethylamino.

In some embodiments, in the compound of Formula III described in the present application, $R^f$ is methylamino, ethylamino, dimethylamino, or diethylamino.

In some embodiments, in the compound of Formula III described in the present application, $R_6$ is

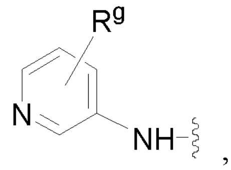

wherein $R^g$ is defined as described in the present application.

In some embodiments, in the compound of Formula III described in the present application, R6 is

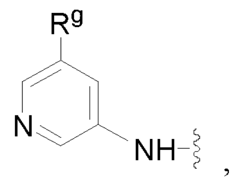

wherein $R^g$ is defined as described in the present application.

In some embodiments, in the compound of Formula III described in the present application, $R^g$ is amino, $C_{1-6}$ alkyl-amino, di($C_{1-6}$ alkyl)-amino, or $C_{1-6}$ alkyl-C(O)NH-.

In some embodiments, in the compound of Formula III described in the present application, $R^g$ is amino.

In some embodiments, in the compound of Formula III described in the present application, $R^g$ is $C_{1-6}$ alkyl-C(O)NH-.

In some embodiments, in the compound of Formula III described in the present application, $R^g$ is $C_{1-6}$ alkyl-amino.

In some embodiments, in the compound of Formula III described in the present application, $R^g$ is di($C_{1-6}$ alkyl)-amino.

In some embodiments, in the compound of Formula III described in the present application, $R^g$ is amino,

$CH_3$-C(O)NH-, $C_2H_5$-C(O)NH-, or $CH_3(CH_2)_2$-C(O)NH-.

In some embodiments, in the compound of Formula III described in the present application, $R^g$ is $C_2H_5$-C(O)NH-.

In some embodiments, in the compound of Formula III described in the present application, $R^g$ is $CH_3(CH_2)_2$-C(O)NH-.

In some embodiments, in the compound of Formula III described in the present application, $R^g$ is amino or $CH_3$-C(O)NH-.

In some embodiments, in the compound of Formula III described in the present application, $R^g$ is amino, $C_{1-4}$ alkyl-amino, di($C_{1-4}$ alkyl)-amino, or $C_{1-6}$ alkyl-C(O)NH-.

In some embodiments, in the compound of Formula III described in the present application, $R^g$ is amino, methylamino, ethylamino, dimethylamino, or diethylamino.

In some embodiments, in the compound of Formula III described in the present application, $R^g$ is methylamino, ethylamino, dimethylamino, or diethylamino.

In some embodiments, in the compound of Formula III described in the present application, R6 is

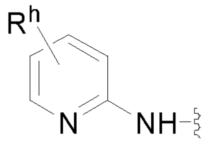

, wherein $R^h$ is defined as described in the present application.

In some embodiments, in the compound of Formula III described in the present application, $R_6$ is

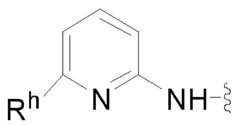

wherein $R^h$ is defined as described in the present application.

In some embodiments, in the compound of Formula III described in the present application, $R^h$ is amino, $C_{1-6}$ alkyl-amino, di($C_{1-6}$ alkyl)-amino, or $C_{1-6}$ alkyl-C(O)NH-.

In some embodiments, in the compound of Formula III described in the present application, $R^h$ is amino.

In some embodiments, in the compound of Formula III described in the present application, $R^h$ is $C_{1-6}$ alkyl-C(O)NH-.

In some embodiments, in the compound of Formula III described in the present application, $R^h$ is $C_{1-6}$ alkyl-amino.

In some embodiments, in the compound of Formula III described in the present application, $R^h$ is di($C_{1-6}$ alkyl)-amino.

In some embodiments, in the compound of Formula III described in the present application, $R^h$ is amino, $CH_3$-C(O)NH-, $C_2H_5$-C(O)NH-, or $CH_3(CH_2)_2$-C(O)NH-.

In some embodiments, in the compound of Formula III described in the present application, $R^h$ is $C_2H_5$-C(O)NH-.

In some embodiments, in the compound of Formula III described in the present application, $R^h$ is $CH_3(CH_2)_2$-C(O)NH-.

In some embodiments, in the compound of Formula III described in the present application, $R^h$ is amino or $CH_3$-C(O)NH-.

In some embodiments, in the compound of Formula III described in the present application, $R^h$ is amino, $C_{1-4}$ alkyl-amino, di($C_{1-4}$ alkyl)-amino, or $C_{1-6}$ alkyl-C(O)NH-.

In some embodiments, in the compound of Formula III described in the present application, $R^h$ is amino, methylamino, ethylamino, dimethylamino, or diethylamino.

In some embodiments, in the compound of Formula III described in the present application, $R^h$ is methylamino, ethylamino, dimethylamino, or diethylamino.

In some embodiments, in the compound of Formula III described in the present application, $R_6$ is

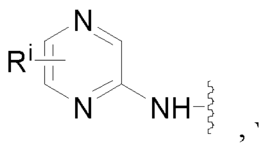

wherein $R^i$ is defined as described in the present application.

In some embodiments, in the compound of Formula III described in the present application, R6 is

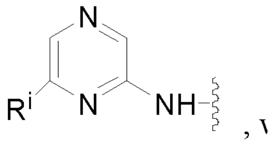

wherein $R^i$ is defined as described in the present application.

In some embodiments, in the compound of Formula III described in the present application, $R^i$ is amino, $C_{1-6}$ alkyl-amino, di($C_{1-6}$ alkyl)-amino, or $C_{1-6}$ alkyl-C(O)NH-.

In some embodiments, in the compound of Formula III described in the present application, $R^i$ is amino.

In some embodiments, in the compound of Formula III described in the present application, $R^i$ is $C_{1-6}$ alkyl-C(O)NH-.

In some embodiments, in the compound of Formula III described in the present application, $R^i$ is $C_{1-6}$ alkyl-amino.

In some embodiments, in the compound of Formula III described in the present application, $R^i$ is di($C_{1-6}$ alkyl)-amino.

In some embodiments, in the compound of Formula III described in the present application, $R^i$ is amino, $CH_3$-C(O)NH-, $C_2H_5$-C(O)NH-, or $CH_3(CH_2)_2$-C(O)NH-.

In some embodiments, in the compound of Formula III described in the present application, $R^i$ is $C_2H_5$-C(O)NH-.

In some embodiments, in the compound of Formula III described in the present application, $R^i$ is $CH_3(CH_2)_2$-C(O)NH-.

In some embodiments, in the compound of Formula III described in the present application, $R^i$ is amino or $CH_3$-C(O)NH-.

In some embodiments, in the compound of Formula III described in the present application, $R^i$ is amino, $C_{1-4}$ alkyl-amino, di($C_{1-4}$ alkyl)-amino, or $C_{1-6}$ alkyl-C(O)NH-.

In some embodiments, in the compound of Formula III described in the present application, $R^i$ is amino, methylamino, ethylamino, dimethylamino, or diethylamino.

In some embodiments, in the compound of Formula III described in the present application, $R^i$ is methylamino, ethylamino, dimethylamino, or diethylamino.

In some embodiments, in the compound of Formula III described in the present application, $R_6$ is pyridyl, phenyl, quinolyl, 2-oxoindolyl, pyrimidyl, isoxazolyl, 1,4-dioxa-spiro[4.5]dec-7-ene group or pyrazolyl, $R_6$ is optionally substituted by one or more $R^j$, each $R^j$ is independently hydrogen, $C_{1-6}$ alkyl, $C_{1-6}$ alkoxy, halogen, alkyl-amino (e.g., $C_{1-6}$ alkyl-amino), dialkyl-amino (e.g., di($C_{1-6}$ alkyl)-amino), 4-methyl-piperazinyl, morpholinyl, amino, $R^k$-C(O)NH- (wherein $R^k$ is benzyl, phenyl, p-methoxybenzyl, phenoxy or $C_{1-6}$ alkyl), pyrrolidinyl, allylamino or propargylamino.

In some embodiments, in the compound of Formula III described in the present application, $R_6$ is pyridyl, and $R_6$ can be optionally substituted with one or more $R^j$, wherein $R^j$ is defined as described in the present application.

In some embodiments, in the compound of Formula III described in the present application, $R_6$ is phenyl, and $R_6$ can be optionally substituted with one or more $R^j$, wherein $R^j$ is defined as described in the present application.

In some embodiments, in the compound of Formula III described in the present application, $R_6$ is quinolyl, and $R_6$ can be optionally substituted with one or more $R^j$, wherein $R^j$ is defined as described in the present application.

In some embodiments, in the compound of Formula III described in the present application, $R_6$ is 2-oxoindolyl, and $R_6$ can be optionally substituted with one or more $R^j$, wherein $R^j$ is defined as described in the present application.

In some embodiments, in the compound of Formula III as described in the present application, $R_6$ is pyrimidyl, and $R_6$ can be optionally substituted with one or more $R^j$, wherein $R^j$ is defined as described in the present application.

In some embodiments, in the compound of Formula III described in the present application, $R_6$ is isoxazolyl, and $R_6$ can be optionally substituted with one or more $R^j$, wherein $R^j$ is defined as described in the present application.

In some embodiments, in the compound of Formula III described in the present application, $R_6$ is 1,4-dioxa-spiro[4.5]dec-7-ene group, and $R_6$ can be optionally substituted with one or more $R^j$, wherein $R^j$ is defined as described in the present application.

In some embodiments, in the compound of Formula III described in the present application, $R_6$ is pyrazolyl,

and $R_6$ can be optionally substituted with one or more $R^j$, wherein $R^j$ is defined as described in the present application.

In some embodiments, in the compound of Formula III described in the present application, R6 is

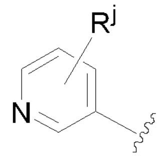,

wherein $R^j$ is defined as described in the present application.

In some embodiments, in the compound of Formula III described in the present application, each $R^j$ is independently $C_{1-6}$ alkyl.

In some embodiments, in the compound of Formula III described in the present application, each $R^j$ is independently hydrogen.

In some embodiments, in the compound of Formula III described in the present application, each $R^j$ is independently 4-methyl-piperazinyl.

In some embodiments, in the compound of Formula III described in the present application, each $R^j$ is independently morpholinyl.

In some embodiments, in the compound of Formula III described in the present application, each $R^j$ is independently $R^k$-C(O)NH- (wherein $R^k$ is benzyl, phenyl, p-methoxybenzyl, phenoxy or $C_{1-6}$ alkyl).

In some embodiments, in the compound of Formula III described in the present application, each $R^j$ is independently pyrrolidinyl.

In some embodiments, in the compound of Formula III described in the present application, each $R^j$ is independently allylamino.

In some embodiments, in the compound of Formula III described in the present application, each $R^j$ is independently a propargylamino.

In some embodiments, in the compound of Formula III described in the present application, each $R^j$ is independently $C_{1-6}$ alkoxy.

In some embodiments, in the compound of Formula III described in the present application, each $R^j$ is independently alkyl-amino (e.g., $C_{1-6}$ alkyl-amino).

In some embodiments, in the compound of Formula III described in the present application, each $R^j$ is independently a dialkyl-amino group (e.g., di($C_{1-6}$ alkyl)-amino group).

In some embodiments, in the compound of Formula III described in the present application, each $R^j$ is independently halogen.

In some embodiments, in the compound of Formula III described in the present application, each $R^j$ is independently amino.

In some embodiments, in the compound of Formula III described in the present application, each $R^j$ is independently methyl, ethyl, n-propyl, isopropyl, n-butyl, isobutyl, sec-butyl, tert-butyl, n-pentyl, n-hexyl, methoxy, ethoxy, propoxy, methylamino, ethylamino, dimethylamino, diethylamino, fluorine, chlorine, bromine or iodine.

In some embodiments, in the compound of Formula III described in the present application, each $R^j$ is independently methyl.

In some embodiments, in the compound of Formula III described in the present application, each $R^j$ is independently ethyl.

In some embodiments, in the compound of Formula III described in the present application, each $R^j$ is independently n-propyl.

In some embodiments, in the compound of Formula III described in the present application, each $R^j$ is independently isopropyl.

In some embodiments, in the compound of Formula III described in the present application, each $R^j$ is independently n-butyl.

In some embodiments, in the compound of Formula III described in the present application, each $R^j$ is independently isobutyl.

In some embodiments, in the compound of Formula III described in the present application, each $R^j$ is independently sec-butyl.

In some embodiments, in the compound of Formula III described in the present application, each $R^j$ is independently tert-butyl.

In some embodiments, in the compound of Formula III described in the present application, each $R^j$ is independently n-pentyl.

In some embodiments, in the compound of Formula III described in the present application, each $R^j$ is independently n-hexyl.

In some embodiments, in the compound of Formula III described in the present application, each $R^j$ is independently methoxy.

In some embodiments, in the compound of Formula III described in the present application, each $R^j$ is independently ethoxy.

In some embodiments, in the compound of Formula III described in the present application, each $R^j$ is independently propoxy.

In some embodiments, in the compound of Formula III described in the present application, each $R^j$ is independently methylamino.

In some embodiments, in the compound of Formula III described in the present application, each $R^j$ is independently ethylamino.

In some embodiments, in the compound of Formula III described in the present application, each $R^j$ is independently dimethylamino.

In some embodiments, in the compound of Formula III described in the present application, each $R^j$ is independently diethylamino.

In some embodiments, in the compound of Formula III described in the present application, each $R^j$ is independently fluorine.

In some embodiments, in the compound of Formula III described in the present application, each $R^j$ is independently chlorine.

In some embodiments, in the compound of Formula III described in the present application, each $R^j$ is independently bromine.

In some embodiments, in the compound of Formula III described in the present application, each $R^j$ is independently iodine.

In some embodiments, in the compound of Formula III described in the present application, $R^k$ is benzyl.

In some embodiments, in the compound of Formula III described in the present application, $R^k$ is phenyl.

In some embodiments, in the compound of Formula III described in the present application, $R^k$ is p-methoxybenzyl.

In some embodiments, in the compound of Formula III described in the present application, $R^k$ is phenoxy.

In some embodiments, in the compound of Formula III described in the present application, $R^k$ is $C_{1-6}$ alkyl.

In some embodiments, in the compound of Formula III described in the present application, $R_6$ is $C_{2-6}$ alkenyl, $R_6$ is optionally substituted with $R^m$, and $R^m$ is $NH_2C(O)$-, $NH_2C(O)NH$-, or $R^n$-OC(O)- (wherein $R^n$ is $C_{1-6}$ alkyl).

In some embodiments, in the compound of Formula III described in the present application, $R_6$ is $C_{2-4}$ alkenyl, $R_6$ is optionally substituted by $R^m$, and $R^m$ is $NH_2C(O)$-, $NH_2C(O)NH$-, or $R^n$-OC(O)- (wherein $R^n$ is $C_{1-6}$ alkyl).

In some embodiments, in the compound of Formula III described in the present application, $R_6$ is vinyl, $R_6$ is optionally substituted by $R^m$, and $R^m$ is $NH_2C(O)$-, $NH_2C(O)NH$-, or $R^n$-OC(O)- (wherein $R^n$ is $C_{1-6}$ alkyl).

In some embodiments, in the compound of Formula III described in the present application, $R_6$ is propenyl, $R_6$ is optionally substituted by $R^m$, and $R^m$ is $NH_2C(O)$-, $NH_2C(O)NH$-, or $R^n$-OC(O)- (wherein $R^n$ is $C_{1-6}$ alkyl).

In some embodiments, in the compound of Formula III described in the present application, $R^m$ is $NH_2C(O)$-.

In some embodiments, in the compound of Formula III described in the present application, $R^m$ is $NH_2C(O)NH$-.

In some embodiments, in the compound of Formula III described in the present application, $R^m$ is $R^n$-OC(O)-, wherein $R^n$ is $C_{1-6}$ alkyl.

In some embodiments, in the compound of Formula III described in the present application, $R^m$ is $R^n$-OC(O)-, wherein $R^n$ is $C_{1-4}$ alkyl.

In some embodiments, in the compound of Formula III described in the present application, $R^m$ is $R^n$-OC(O)-, wherein $R^n$ is methyl, ethyl, n-propyl, isopropyl, n-butyl, isobutyl, sec-butyl, tert-butyl, n-pentyl, or n-hexyl.

In some embodiments, in the compound of Formula III described in the present application, $R^m$ is $R^n$-OC(O)-, wherein $R^n$ is methyl, ethyl, n-propyl, isopropyl, n-butyl, isobutyl, sec-butyl, or tert-butyl.

In some embodiments, in the compound of Formula III described in the present application, $R^m$ is $R^n$-OC(O)-, wherein $R^n$ is methyl, ethyl, or n-propyl.

In some embodiments, in the Formula III described in the present application, $R_6$ is

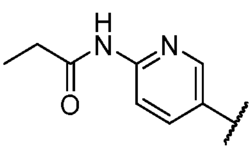, 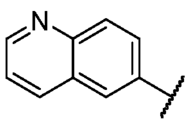, 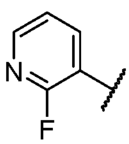, 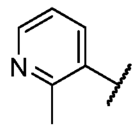, 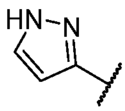, 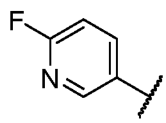,

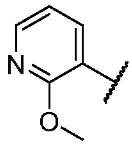, 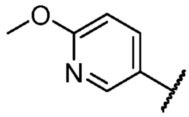, 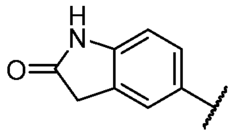, 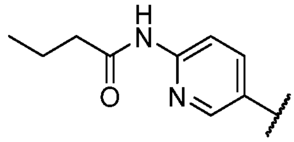, 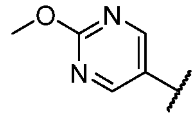,

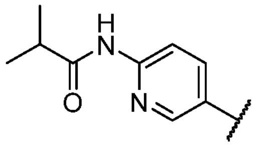, 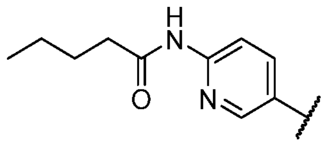, 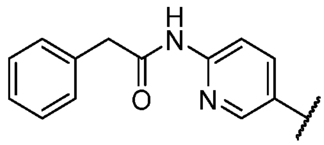,

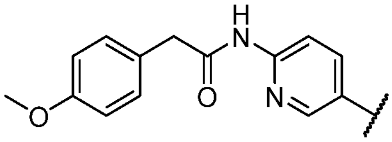, 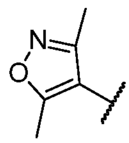, 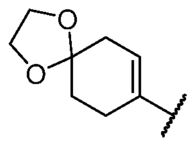, 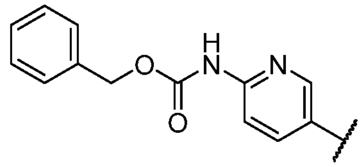,

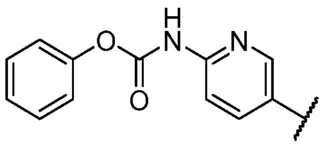, 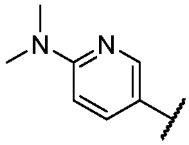, 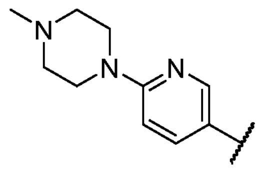, 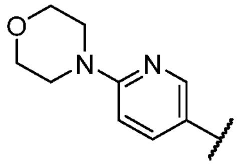,

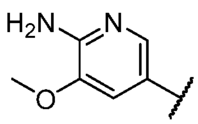, 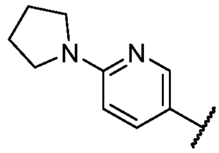, 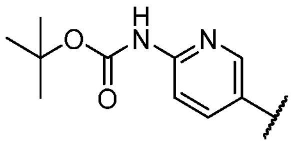, 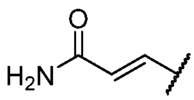,

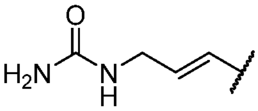, 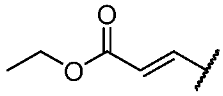, 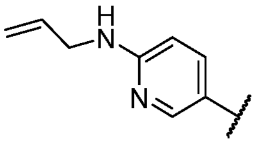, 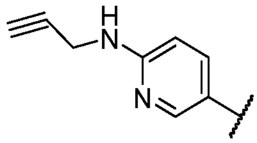,

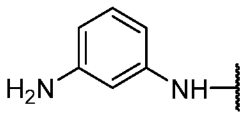, 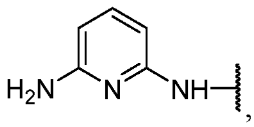, 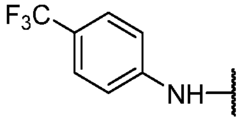, 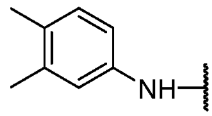,

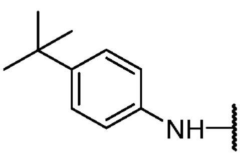, 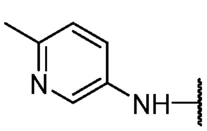, 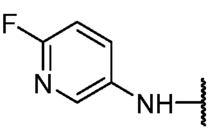, 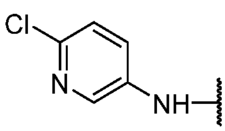, 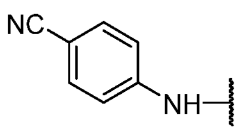,

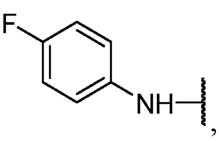, 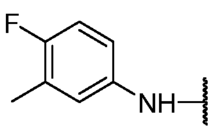, 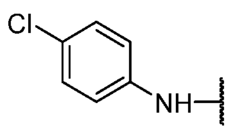, 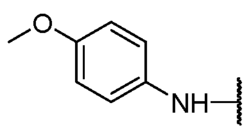,

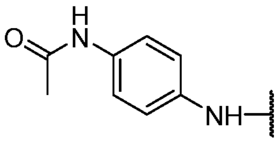, 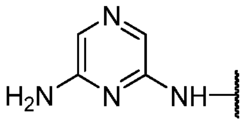, or 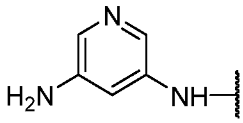.

In some embodiments, in the Formula III described in the present application, $R_6$ is

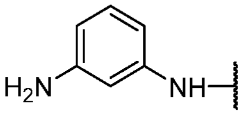, 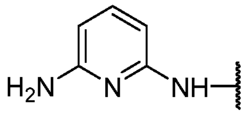, 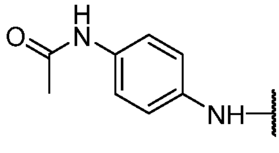, 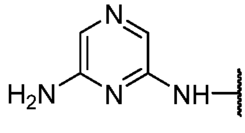, or

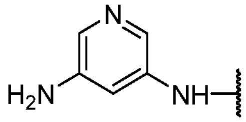.

In some embodiments, in Formula III described in the present application, $R_6$ is

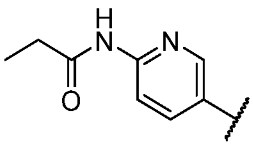, 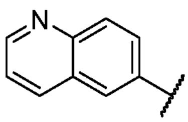, 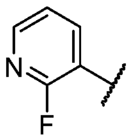, 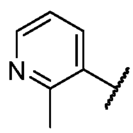, 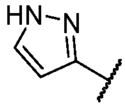, 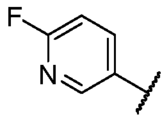,

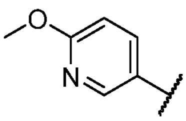, 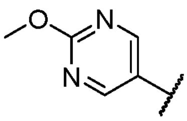, 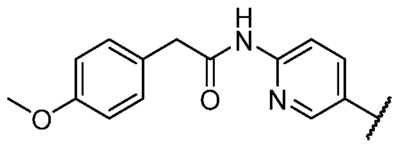, 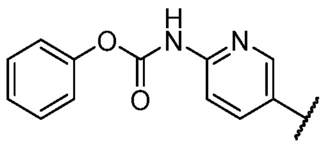,

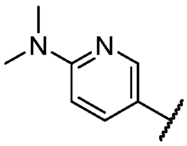, 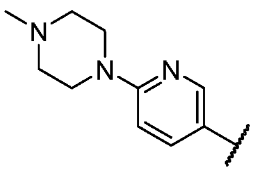, 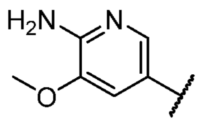, 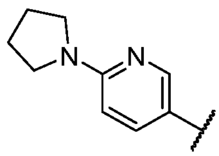,

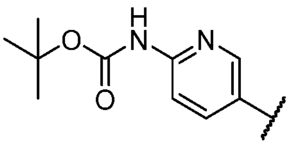, 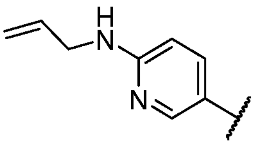, or 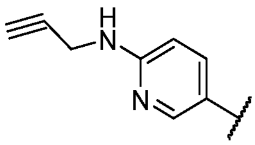.

In some embodiments, in the Formula III described in the present application, $R_6$ is

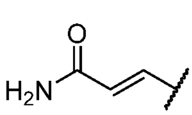, or 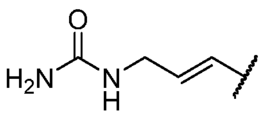.

The present application also provides a pharmaceutically acceptable salt of the quinoline compound having the above-mentioned Formula I, II or III, and the salt is preferably a conventional inorganic acid salt (e.g., hydrochloride, sulfate, phosphate) or organic acid salt (methanesulfonate, trifluoromethanesulfonate, acetate, trifluoroacetate, benzoate) of the quinoline compound, preferably hydrochloride.

The quinoline compound of the present application, a pharmaceutically acceptable salt, a stereoisomer, a hydrate or a solvate thereof has potential inhibitory activity against Enterovirus 71, making such compound can be useful as an active ingredient of a drug for the treatment of anti-enterovirus 71.

The use of the quinoline compound represented by Formula I, II and III, a pharmaceutically acceptable salt, a stereoisomer, a hydrate or a solvate thereof in the manufacture of drug for the treatment of anti-enterovirus 71 also falls into the protection scope of the present application.

The preferred compounds of Formula I, II and III in the present application are as follows:

| No. | Code | Structure | Molecular Formula |
|---|---|---|---|
| 1 | HTL-2-34 | 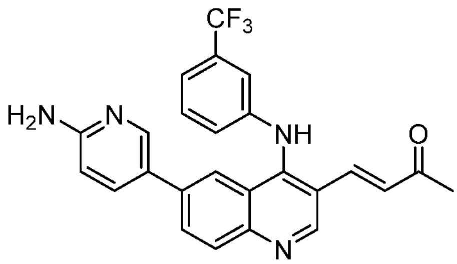 | $C_{25}H_{19}F_3N_4O$ |
| 2 | HTL-2-35 | 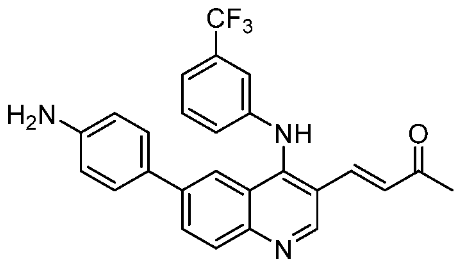 | $C_{26}H_{20}F_3N_3O$ |
| 3 | HTL-2-38 | 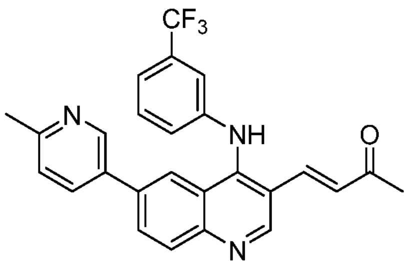 | $C_{26}H_{20}F_3N_3O$ |
| 4 | HTL-2-42 | 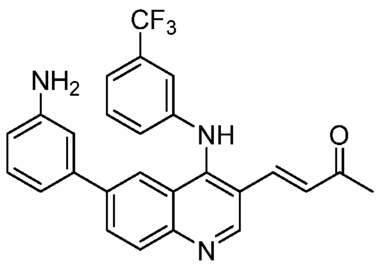 | $C_{26}H_{20}F_3N_3O$ |
| 5 | HTL-3-04 | 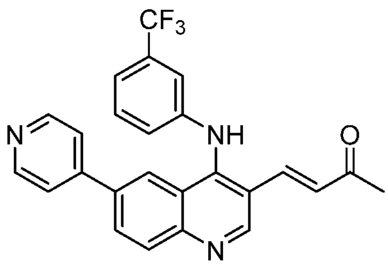 | $C_{25}H_{18}F_3N_3O$ |

(continued)

| No. | Code | Structure | Molecular Formula |
|---|---|---|---|
| 6 | HTL-3-07 | 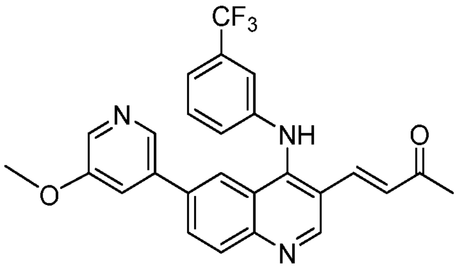 | $C_{26}H_{20}F_3N_3O_2$ |
| 7 | HTL-3-11 | 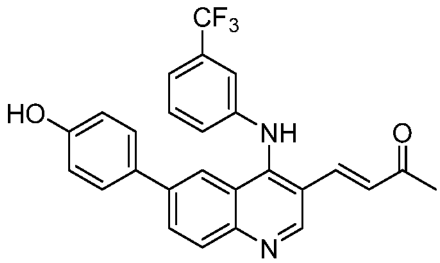 | $C_{26}H_{19}F_3N_2O_2$ |
| 8 | HTL-3-12 | 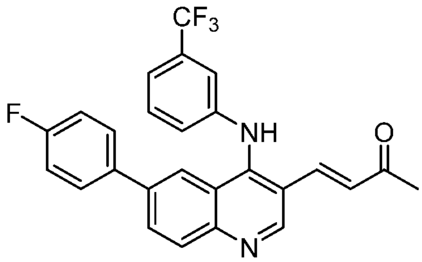 | $C_{26}H_{18}F_4N_2O$ |
| 9 | HTL-3-15 | 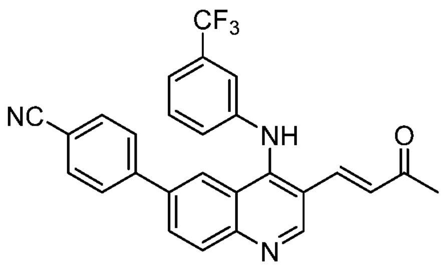 | $C_{27}H_{18}F_3N_3O$ |
| 10 | HTL-3-18 | 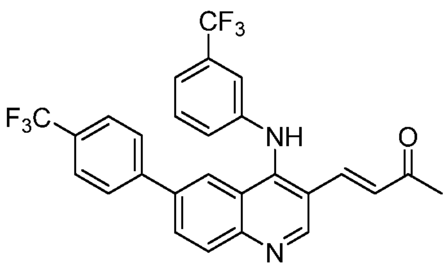 | $C_{27}H_{18}F_6N_2O$ |
| 11 | HTL-3-16 | 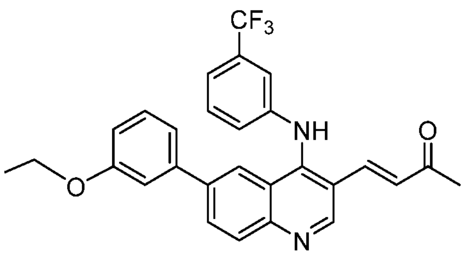 | $C_{28}H_{23}F_3N_2O_2$ |

(continued)

| No. | Code | Structure | Molecular Formula |
|---|---|---|---|
| 12 | HTL-3-22 | 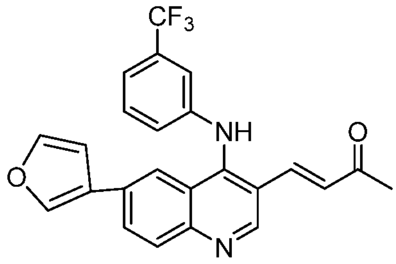 | $C_{24}H_{17}F_3N_2O_2$ |
| 13 | HTL-3-24 | 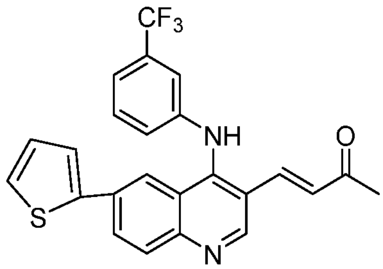 | $C_{24}H_{17}F_3N_2OS$ |
| 14 | HTL-3-25 | 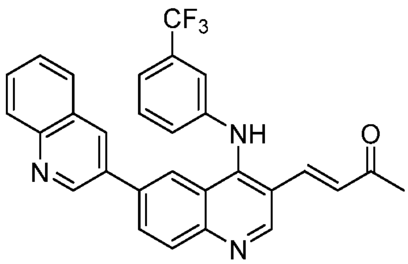 | $C_{29}H_{20}F_3N_3O$ |
| 15 | HTL-3-26 | 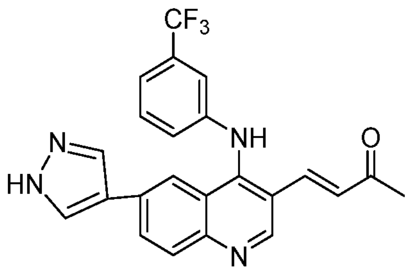 | $C_{23}H_{17}F_3N_4O$ |
| 16 | HTL-3-32 | 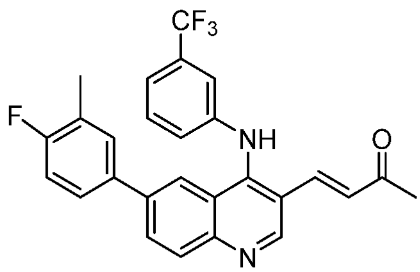 | $C_{27}H_{20}F_4N_2O$ |

(continued)

| No. | Code | Structure | Molecular Formula |
|---|---|---|---|
| 17 | HTL-3-33 | 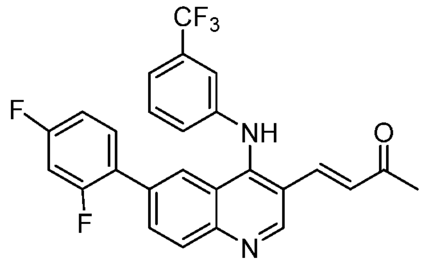 | $C_{26}H_{17}F_5N_2O$ |
| 18 | HTL-3-34 | 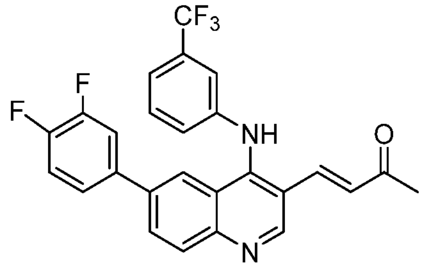 | $C_{26}H_{17}F_5N_2O$ |
| 19 | HTL-3-36 | 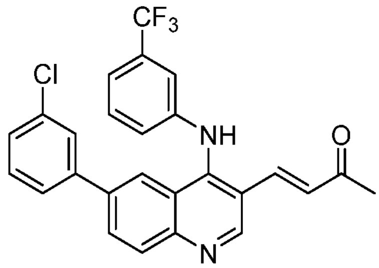 | $C_{26}H_{18}ClF_3N_2O$ |
| 20 | HTL-3-37 | 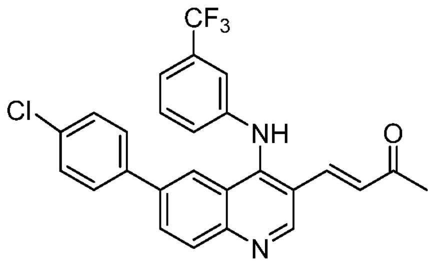 | $C_{26}H_{18}ClF_3N_2O$ |
| 21 | HTL-3-39 | 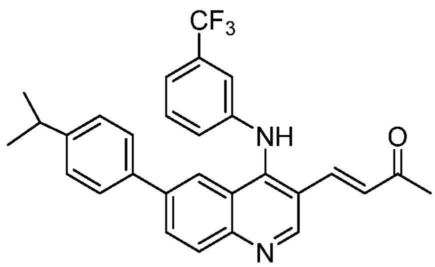 | $C_{29}H_{25}F_3N_2O$ |

(continued)

| No. | Code | Structure | Molecular Formula |
|---|---|---|---|
| 22 | HTL-3-40 | 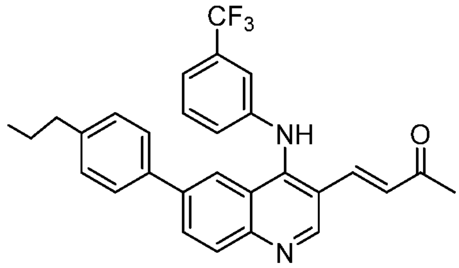 | $C_{29}H_{25}F_3N_2O$ |
| 23 | HTL-3-41 | 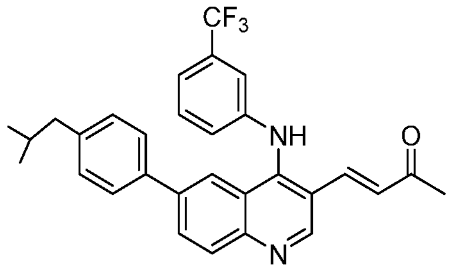 | $C_{30}H_{27}F_3N_2O$ |
| 24 | HTL-3-42 | 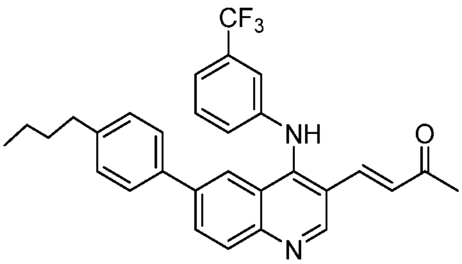 | $C_{30}H_{27}F_3N_2O$ |
| 25 | HTL-3-43 | 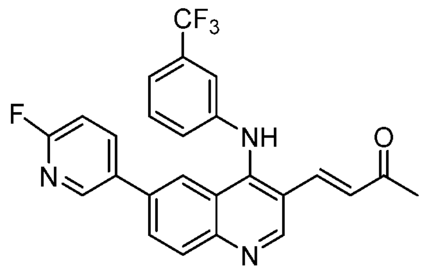 | $C_{25}H_{17}F_4N_3O$ |
| 26 | HTL-3-45 | 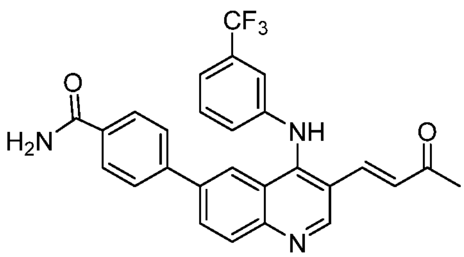 | $C_{27}H_{20}F_3N_3O_2$ |
| 27 | HTL-3-46 | 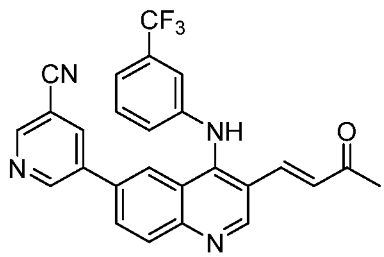 | $C_{26}H_{17}F_3N_4O$ |

(continued)

| No. | Code | Structure | Molecular Formula |
|---|---|---|---|
| 28 | HTL-4-32 | 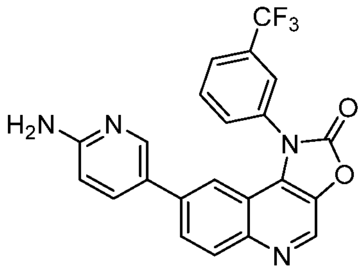 | $C_{22}H_{13}F_3N_4O_2$ |
| 29 | HTL-5-21 | 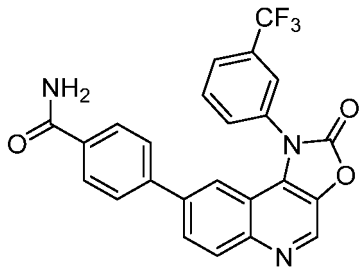 | $C_{24}H_{14}F_3N_3O_3$ |
| 30 | HTL-5-23 | 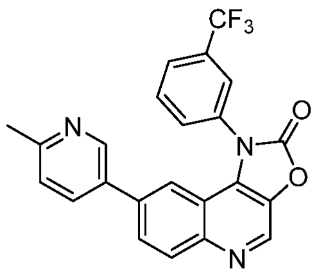 | $C_{23}H_{14}F_3N_3O_2$ |
| 31 | HTL-5-25 | 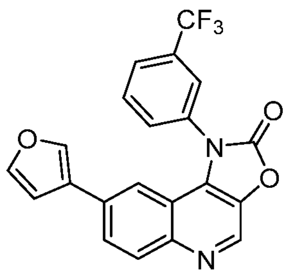 | $C_{21}H_{11}F_3N_2O_3$ |
| 32 | HTL-5-26 | 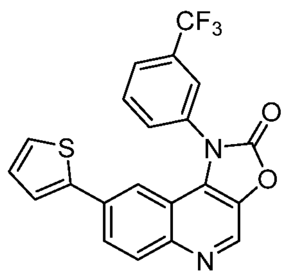 | $C_{21}H_{11}F_3N_2O_2S$ |

(continued)

| No. | Code | Structure | Molecular Formula |
|---|---|---|---|
| 33 | HTL-5-27 | 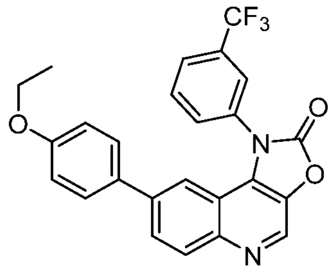 | $C_{25}H_{17}F_3N_2O_3$ |
| 34 | HTL-5-28 | 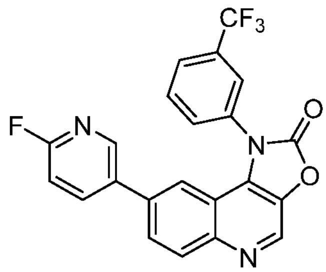 | $C_{22}H_{11}F_4N_3O_2$ |
| 35 | HTL-5-29 | 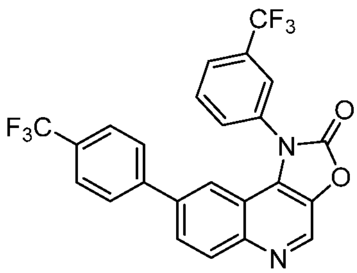 | $C_{24}H_{12}F_6N_2O_2$ |
| 36 | HTL-5-30 | 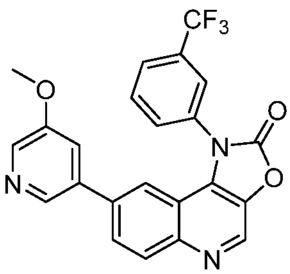 | $C_{23}H_{14}F_3N_3O_3$ |
| 37 | HTL-5-32 | 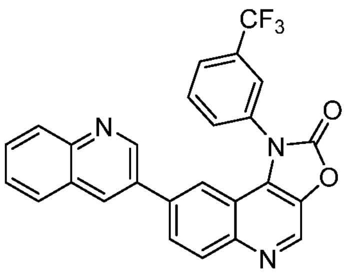 | $C_{26}H_{14}F_3N_3O_2$ |

(continued)

| No. | Code | Structure | Molecular Formula |
|---|---|---|---|
| 38 | HTL-5-33 | 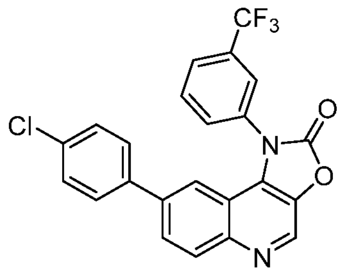 | $C_{23}H_{12}ClF_3N_2O_2$ |
| 39 | HTL-5-34 | 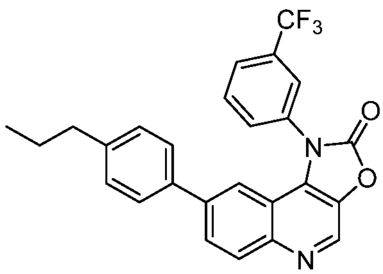 | $C_{26}H_{19}F_3N_2O_2$ |
| 40 | HTL-5-35 | 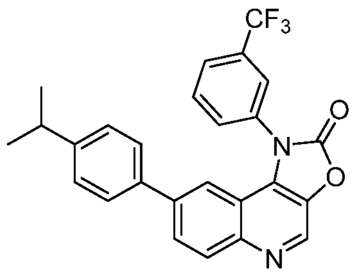 | $C_{26}H_{19}F_3N_2O_3$ |
| 41 | HTL-5-36 | 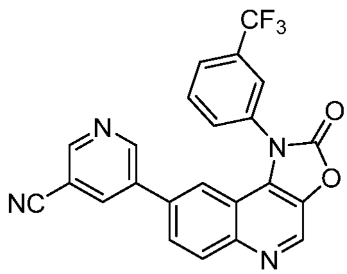 | $C_{23}H_{11}F_3N_4O_2$ |
| 42 | HTL-6-11 | 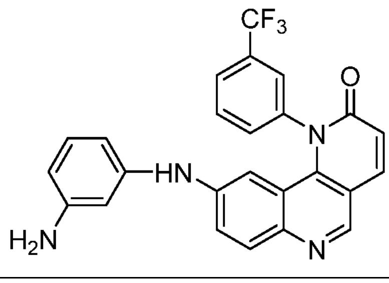 | $C_{25}H_{17}F_3N_4O$ |

(continued)

| No. | Code | Structure | Molecular Formula |
|---|---|---|---|
| 43 | HTL-6-12 | 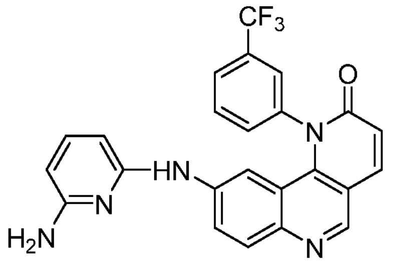 | $C_{24}H_{16}F_3N_5O$ |
| 44 | HTL-6-16 | 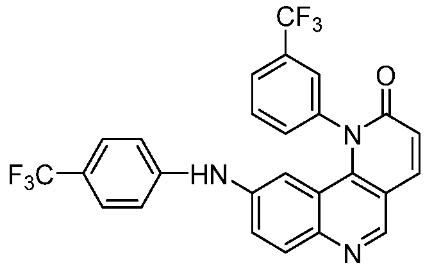 | $C_{26}H_{15}F_6N_3O$ |
| 45 | HTL-6-17 | 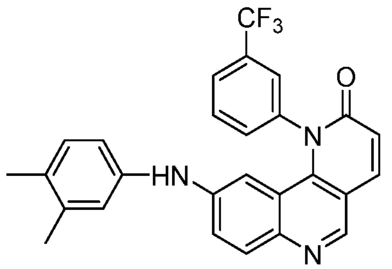 | $C_{27}H_{20}F_3N_3O$ |
| 46 | HTL-6-18 | 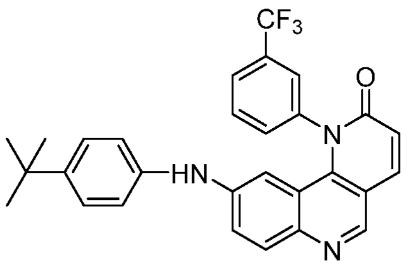 | $C_{29}H_{24}F_3N_3O$ |
| 47 | HTL-6-19 | 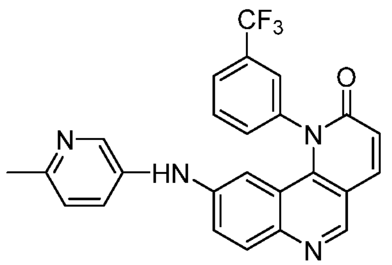 | $C_{25}H_{17}F_3N_4O$ |

(continued)

| No. | Code | Structure | Molecular Formula |
|---|---|---|---|
| 48 | HTL-6-20 | 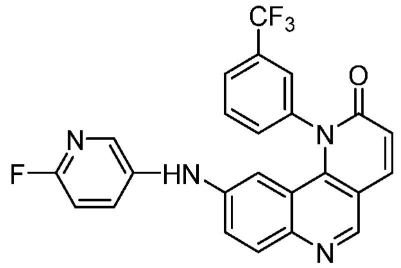 | $C_{24}H_{14}F_4N_4O$ |
| 49 | HTL-6-21 | 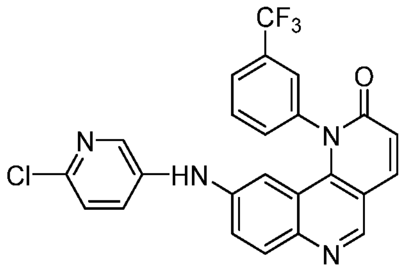 | $C_{24}H_{14}ClF_3N_4O$ |
| 50 | HTL-6-22 | 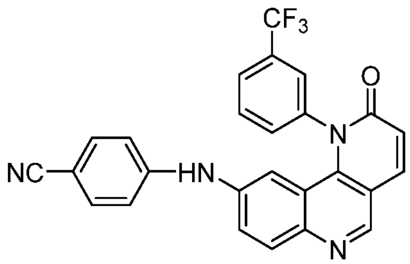 | $C_{26}H_{15}F_3N_4O$ |
| 51 | HTL-6-23 | 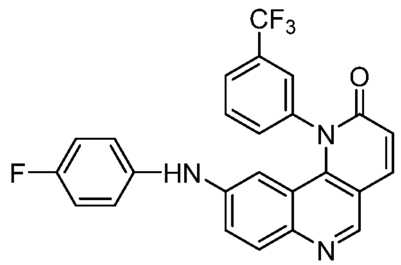 | $C_{25}H_{15}F_4N_3O$ |
| 52 | HTL-6-24 | 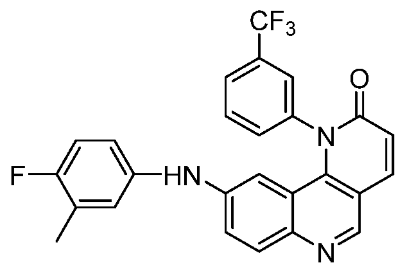 | $C_{26}H_{17}F_4N_3O$ |

(continued)

| No. | Code | Structure | Molecular Formula |
|---|---|---|---|
| 53 | HTL-6-25 | 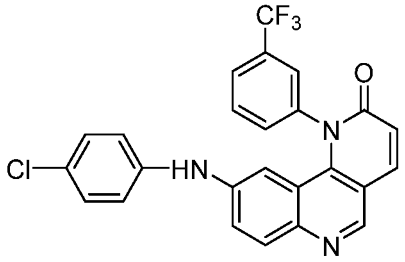 | $C_{24}H_{14}ClF_3N_3O$ |
| 54 | HTL-6-26 | 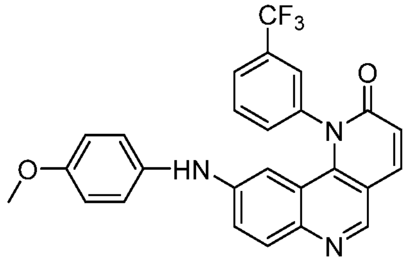 | $C_{26}H_{18}F_3N_3O_2$ |
| 55 | HTL-6-27 | 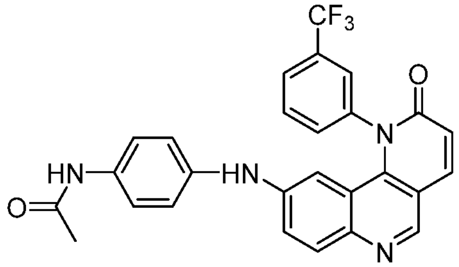 | $C_{27}H_{19}F_3N_4O_2$ |
| 56 | HTL-6-28 | 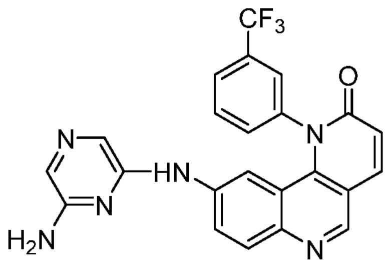 | $C_{23}H_{15}F_3N_6O$ |
| 57 | HTL-6-29 | 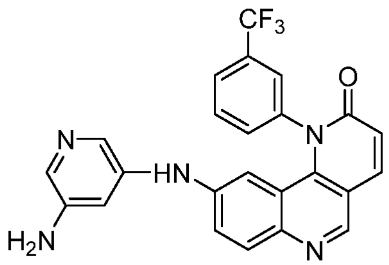 | $C_{24}H_{16}F_3N_5O$ |

(continued)

| No. | Code | Structure | Molecular Formula |
|---|---|---|---|
| 58 | HTL-6-30 | 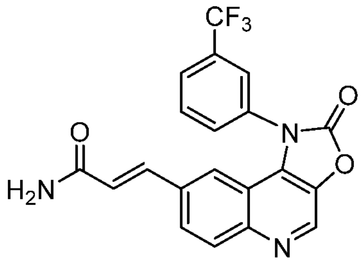 | $C_{20}H_{12}F_3N_3O_3$ |
| 59 | HTL-6-31 | 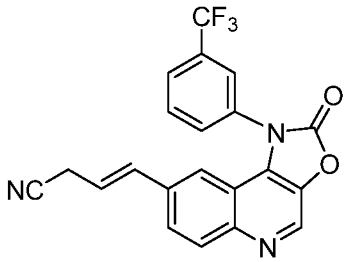 | $C_{21}H_{12}F_3N_3O_2$ |
| 60 | HTL-6-32 | 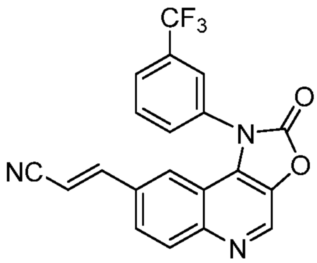 | $C_{20}H_{10}F_3N_3O_2$ |
| 61 | HTL-6-33 | 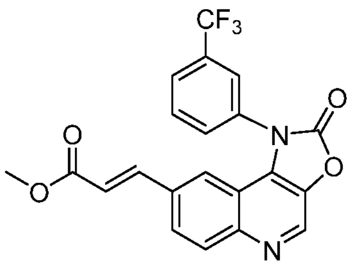 | $C_{21}H_{13}F_3N_2O_4$ |
| 62 | HTL-6-34 | 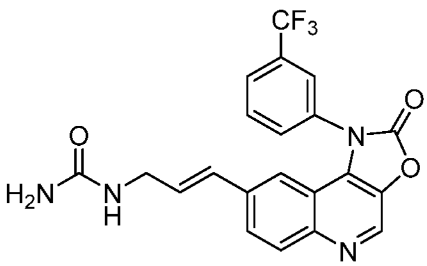 | $C_{21}H_{15}F_3N_4O_3$ |
| 63 | HTL-6-35 | 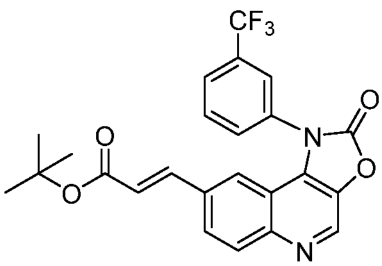 | $C_{24}H_{19}F_3N_2O_4$ |

(continued)

| No. | Code | Structure | Molecular Formula |
|---|---|---|---|
| 64 | HTL-6-38 | 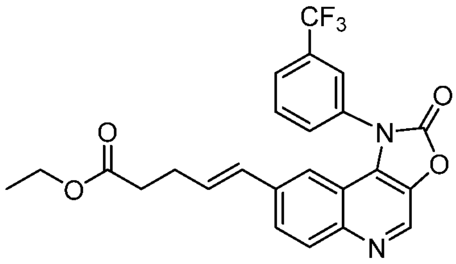 | $C_{24}H_{19}F_3N_2O_4$ |
| 65 | WSX-1-13 | 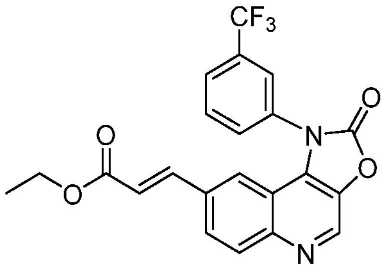 | $C_{22}H_{15}F_3N_2O_4$ |
| 66 | WSX-1-24 | 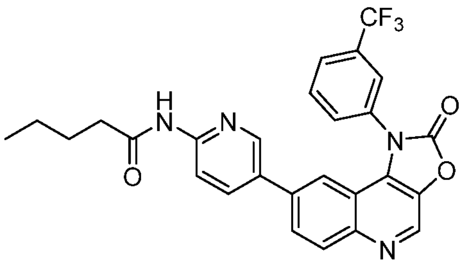 | $C_{27}H_{21}F_3N_4O_3$ |
| 67 | HTL-7-23 | 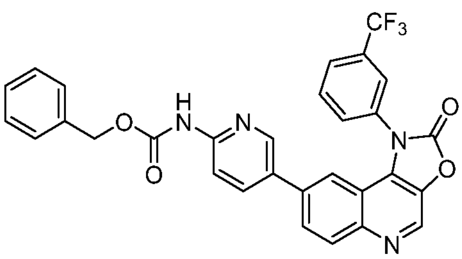 | $C_{30}H_{19}F_3N_4O_4$ |
| 68 | HTL-7-22 | 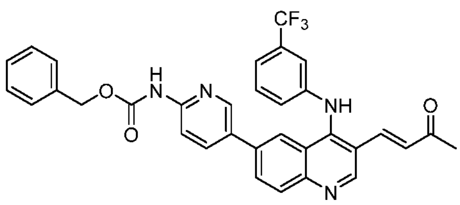 | $C_{23}H_{25}F_3N_4O_3$ |
| 69 | HTL-6-45 | 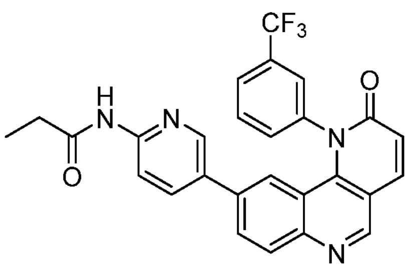 | $C_{27}H_{19}F_3N_4O_2$ |

(continued)

| No. | Code | Structure | Molecular Formula |
|---|---|---|---|
| 70 | HTL-6-47 | 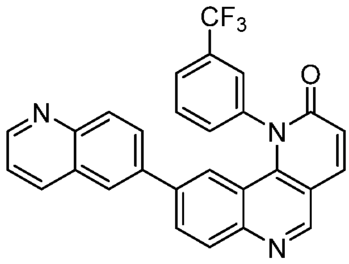 | $C_{28}H_{16}F_3N_3O$ |
| 71 | HTL-6-48 | 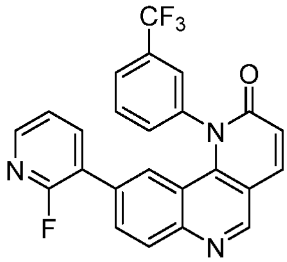 | $C_{24}H_{13}F_4N_3O$ |
| 72 | HTL-6-49 | 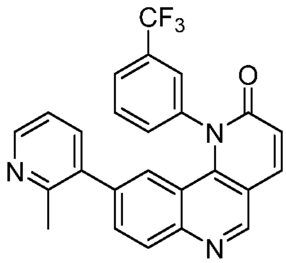 | $C_{25}H_{16}F_3N_3O$ |
| 73 | HTL-7-01 | 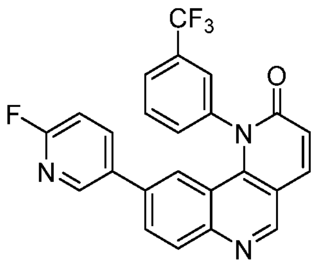 | $C_{24}H_{13}F_4N_3O$ |
| 74 | HTL-7-02 | 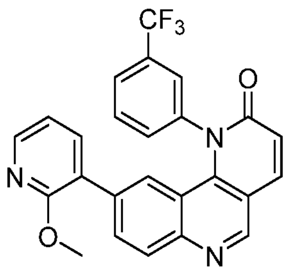 | $C_{25}H_{16}F_3N_3O_2$ |

(continued)

| No. | Code | Structure | Molecular Formula |
|---|---|---|---|
| 75 | HTL-7-03 | 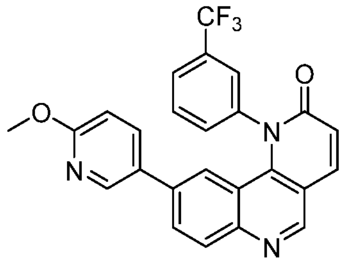 | $C_{25}H_{16}F_3N_3O_2$ |
| 76 | HTL-7-04 | 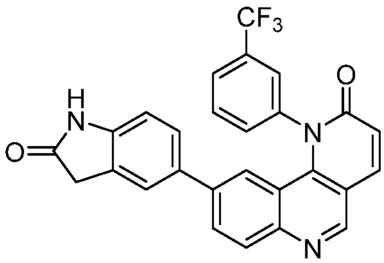 | $C_{27}H_{16}F_3N_3O_2$ |
| 77 | HTL-7-05 | 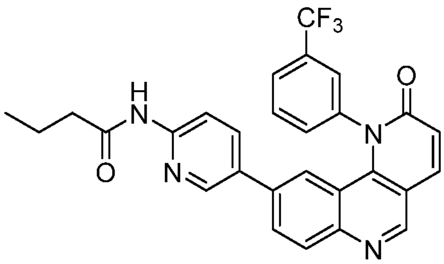 | $C_{28}H_{21}F_3N_4O_2$ |
| 78 | HTL-7-06 | 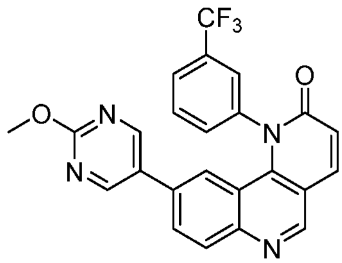 | $C_{24}H_{15}F_3N_4O_2$ |
| 79 | HTL-7-07 | 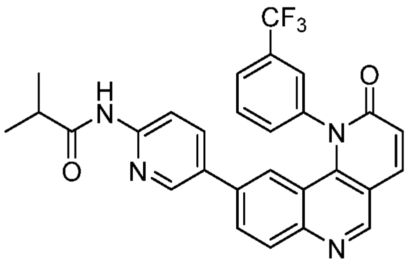 | $C_{28}H_{21}F_3N_4O_2$ |
| 80 | HTL-7-08 | 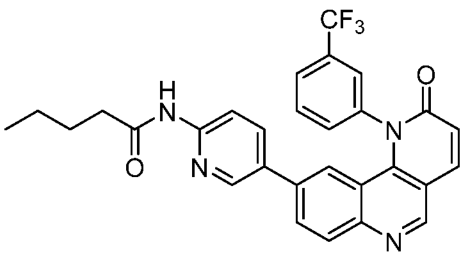 | $C_{29}H_{23}F_3N_4O_2$ |

(continued)

| No. | Code | Structure | Molecular Formula |
|---|---|---|---|
| 81 | HTL-7-10 | 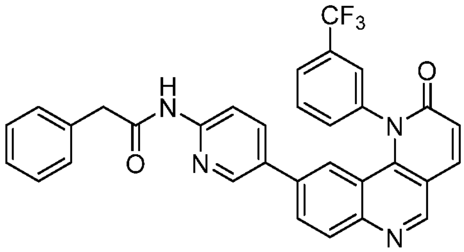 | $C_{32}H_{21}F_3N_4O_2$ |
| 82 | HTL-7-11 | 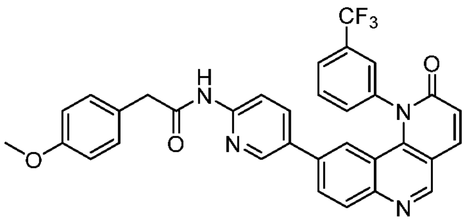 | $C_{33}H_{23}F_3N_4O_3$ |
| 83 | HTL-7-12 | 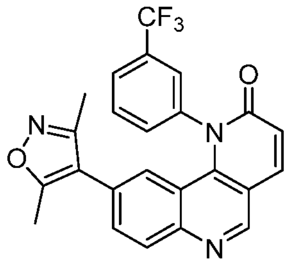 | $C_{24}H_{16}F_3N_3O_2$ |
| 84 | HTL-7-13 | 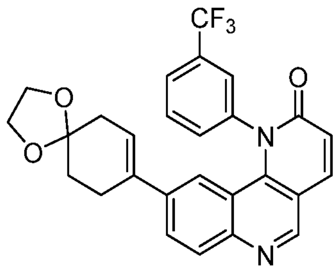 | $C_{27}H_{21}F_3N_2O_3$ |
| 85 | HTL-7-14 | 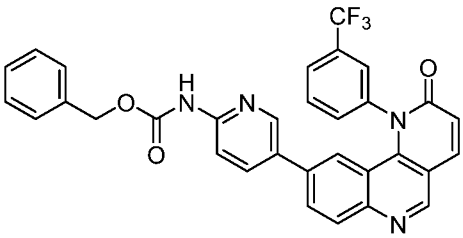 | $C_{32}H_{21}F_3N_4O_3$ |
| 86 | HTL-7-15 | 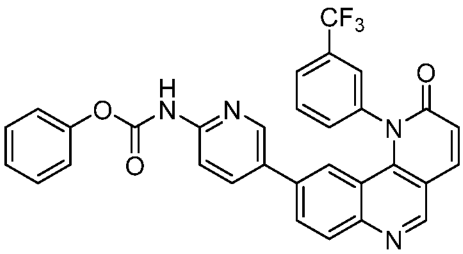 | $C_{31}H_{19}F_3N_4O_3$ |

(continued)

| No. | Code | Structure | Molecular Formula |
|---|---|---|---|
| 87 | HTL-7-16 | 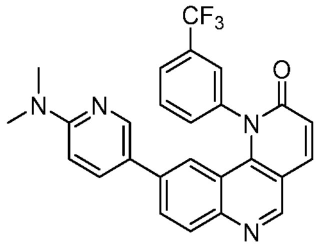 | $C_{26}H_{19}F_3N_4O$ |
| 88 | HTL-7-17 | 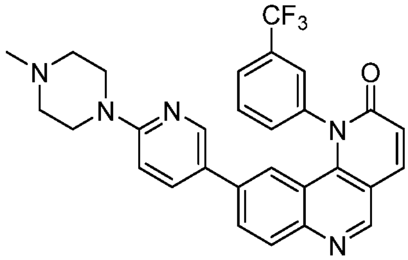 | $C_{29}H_{24}F_3N_5O$ |
| 89 | HTL-6-50 | 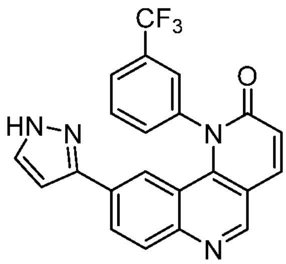 | $C_{22}H_{13}F_3N_4O$ |
| 90 | HTL-7-18 | 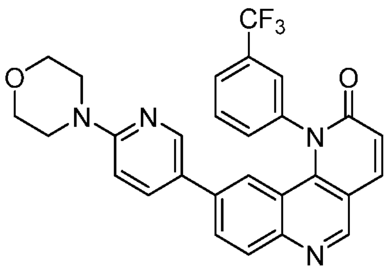 | $C_{28}H_{21}F_3N_4O_2$ |
| 91 | HTL-7-19 | 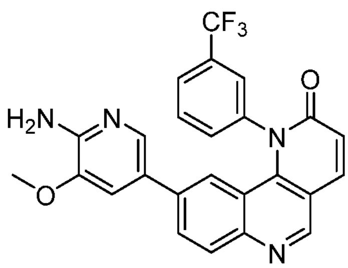 | $C_{25}H_{17}F_3N_4O_2$ |
| 92 | HTL-7-20 | 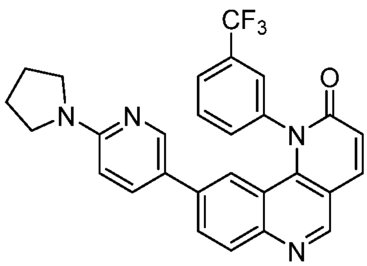 | $C_{28}H_{21}F_3N_4O$ |

(continued)

| No. | Code | Structure | Molecular Formula |
|---|---|---|---|
| 93 | HTL-7-21 | 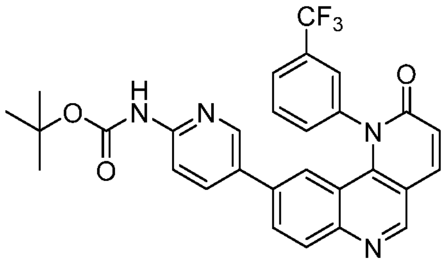 | $C_{29}H_{23}F_3N_4O_3$ |
| 94 | HTL-7-24 | 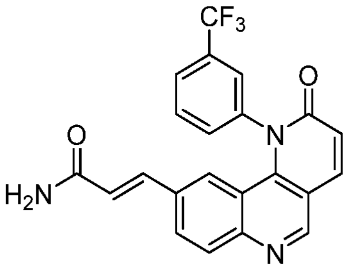 | $C_{22}H_{14}F_3N_3O_2$ |
| 95 | HTL-7-25 | 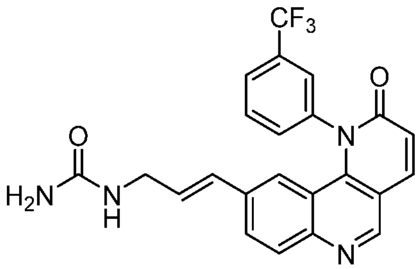 | $C_{23}H_{17}F_3N_4O_2$ |
| 96 | HTL-7-26 | 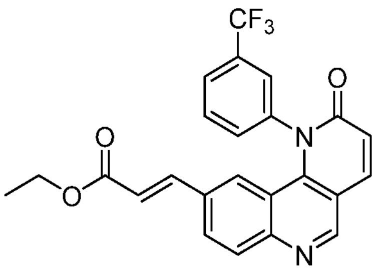 | $C_{24}H_{17}F_3N_2O_3$ |
| 97 | HTL-7-28 | 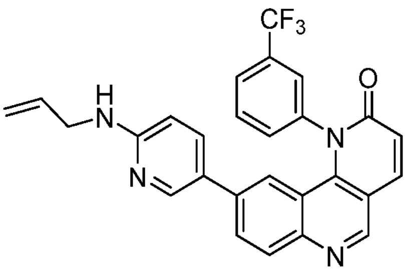 | $C_{27}H_{19}F_3N_4O$ |

(continued)

| No. | Code | Structure | Molecular Formula |
|---|---|---|---|
| 98 | HTL-7-29 | 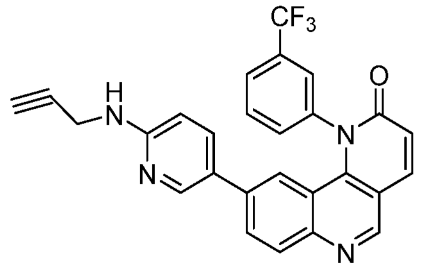 | $C_{27}H_{17}F_3N_4O$ |

The compounds of Formula I, Formula II or Formula III described in the present application can be prepared by conventional synthetic routes as required.

The present application also provides a pharmaceutical composition, which at least comprises the compound represented by Formula I, Formula II or Formula III, a pharmaceutically acceptable salt, a stereoisomer, a hydrate or a solvate thereof as described in the present application, and one or more pharmaceutically acceptable carriers or excipients.

The present application also provides use of the compound represented by Formula I, Formula II or Formula III, a pharmaceutically acceptable salt, a stereoisomer, a hydrate or a solvate thereof, or the pharmaceutical composition as described in the present application in the manufacture of a medicament for the treatment and/or prevention of a disease or condition associated with viral infection, or in the manufacture of a medicament for inhibiting the replication of an enterovirus (e.g., EV71) in a host cell (e.g., a cell of mammal). In some embodiments, the viral infection is an infection caused by an enterovirus (e.g., EV71). In some embodiments, the disease or condition associated with viral infection is hand-foot-and-mouth disease.

The present application also provides the compound represented by Formula I, Formula II or Formula III, a pharmaceutically acceptable salt, a stereoisomer, a hydrate or a solvate thereof, or the pharmaceutical combination described in the present application, for use in the treatment and/or prevention of a disease or condition associated with viral infection. In some embodiments, the viral infection is an infection caused by an enterovirus (e.g., EV71). In some embodiments, the disease or condition associated with viral infection is hand-foot-and-mouth disease.

The present application also provides the compound represented by Formula I, Formula II or Formula III, a pharmaceutically acceptable salt, a stereoisomer, a hydrate or a solvate thereof, or the pharmaceutical combination described in the present application, for use in inhibiting the replication of an enterovirus (e.g., EV71) in a host cell (e.g., a cell of mammal).

The present application also provides a method for the treatment and/or prevention of a disease or condition associated with viral infection, the method comprising administering to a subject in need a therapeutically and/or prophylactically effective amount of at least one of the compound represented by Formula I, Formula II or Formula III as described in the present application, a pharmaceutically acceptable salt, a stereoisomer, a hydrate or a solvate thereof, or the pharmaceutical composition as described in the present application. In some embodiments, the viral infection is an infection caused by an enterovirus (e.g., EV71). In some embodiments, the disease or condition associated with viral infection is hand-foot-and-mouth disease.

The present application also provides a method for inhibiting the replication of an enterovirus (e.g., EV71) in a mammal in need, the method comprising administering to the mammal in need a therapeutically and/or prophylactically effective amount of the compound represented by Formula I, Formula II or Formula III as described in the preset application, a pharmaceutically acceptable salt, a stereoisomer, a hydrate or a solvate thereof, or the pharmaceutical composition as described in the present application.

The various terms and phrases used in the present invention have the general meanings known to those skilled in the art. Even so, in the present invention, more detailed descriptions and explanations of these terms and phrases are still provided here. If the terms and phrases as mentioned are inconsistent with the known meanings, the meaning expressed in the present invention shall prevail.

The pharmaceutical composition as described in the present application can be administered through various routes, such as oral tablet, capsule, powder, oral liquid, injection and transdermal preparation. According to conventional pharmaceutical practices, the pharmaceutically acceptable carrier comprises diluent, filler, disintegrant, wetting agent, lubricant, coloring agent, flavoring agent or other conventional additive. Typical pharmaceutically acceptable carriers include, for example, microcrystalline cellulose, starch, crospovidone, povidone, polyvinylpyrrolidone, maltitol, citric acid, sodium lauryl sulfonate or magnesium stearate, etc..

In some embodiments, the mammal comprises bovidae, equidae, caprinae, suidae, canidae, felidae, glires, primate, among which the mammal is preferred to be human.

The pharmaceutical composition as described in the present application can be prepared into various forms according to different administration routes.

According to the present application, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the help of an explant reservoir, wherein the preferred administration route is oral, intraperitoneal or intravenous administration.

When orally administered, the compound of Formula I can be prepared into any form of orally acceptable preparation, including but not limited to a tablet, a capsule, an aqueous solution or an aqueous suspension. The carrier for use in a tablet generally includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned oral preparation forms.

When rectally administered, the compound of Formula I can generally be prepared in a form of suppository, which is prepared by mixing the drug with a suitable non-irritating excipient. The excipient is present in solid state at room temperature, but melts at the rectal temperature to release the drug. Such excipient includes cocoa butter, beeswax and polyethylene glycol.

When topically administered, especially for treatment of easily accessible diseased parts or organs such as eye, skin, or lower intestinal neurological disease by topical application, the compound of Formula I can be prepared in various forms of topical preparations according to different diseased parts or organs, the specific instructions are as follows:

When topically administered to eye, the compound of Formula I can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the compound of Formula I can be prepared into a suitable form such as an ointment, a lotion or a cream, in which the active ingredient is suspended or dissolved in one or more carriers. The carrier for use in an ointment includes, but is not limited to: mineral oil, liquid petrolatum, white petrolatum, propylene glycol, polyethylene oxide, polypropylene oxide, emulsifying wax, and water. The carrier for use in a lotion or a cream includes, but is not limited to: mineral oil, sorbitan monostearate, Tween-60, cetyl ester wax, hexadecenyl aryl alcohol, 2-octyldodecanol, benzyl alcohol and water.

When topically administered to lower intestinal tract, the compound of Formula I can be prepared into a form such as rectal suppository as described above or a suitable enema preparation form, in addition, a topical transdermal patch can also be used.

The compound of Formula I can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

The above various preparation forms of drugs can be prepared according to conventional methods in the pharmaceutical field.

As described herein, "therapeutically effective amount" or "prophylactically effective amount" refers to an amount that is sufficient to treat or prevent a patient's disease but is sufficiently low to avoid serious side effects (at a reasonable benefit/risk ratio) within a reasonable medical judgment. The therapeutically effective amount of the compound will change upon the factors such as the selected specific compound (for example, considering the potency, effectiveness and half-life of compound), the selected route of administration, the disease to be treated, the severity of the disease to be treated, and the conditions such as age, size, weight and physical disease of the patient to be treated, the medical history of the patient to be treated, the duration of treatment, the nature of concurrent therapy, the desired therapeutic effect and so on, but it can still be routinely determined by those skilled in the art.

In addition, it should be noted that the specific dosage and usage of the compound of Formula I described in the present application for different patients depends on many factors, including the patient's age, weight, gender, natural health status, nutritional status, the active strength of the compound, the time of administration, the metabolic rate, the severity of disease, and the subjective judgment of physician. Herein it is preferable to use a dose of 0.01 to 100 mg/kg body weight/day.

As described herein, the term "pharmaceutically acceptable", for example, when describing "pharmaceutically acceptable salt", means that the salt is not only physiologically acceptable to the subject, but also refers to it is a

pharmaceutically useful synthetic substance.

The term "alkyl" as used herein refers to a saturated linear or branched monovalent hydrocarbon group, preferably having 1 to 12 carbon atoms, more preferably having 1 to 10, 1 to 8, 1 to 6, 1 to 4 or 1 to 3 carbon atoms. The term "$C_{1-10}$ alkyl" or "$C_{1-6}$ alkyl" refers to an alkyl having a specified number of carbon atoms, which is a linear or branched alkyl, and which can include its subgroups, such as $C_{1-6}$ alkyl, $C_{1-4}$ alkyl, $C_{1-3}$ alkyl, $C_{1-2}$ alkyl, $C_{2-5}$ alkyl, $C_{2-4}$ alkyl, etc. Typical examples of "alkyl" include, but are not limited to, methyl, ethyl, n-propyl, isopropyl, n-butyl, isobutyl, sec-butyl, tert-butyl, n-pentyl, tert-pentyl, neo-pentyl, hexyl, heptyl, octyl, etc.

The term "haloalkyl" as used herein refers to an alkyl that is mono- or polysubstituted by halogen such as fluorine, chlorine, bromine or iodine. Preferred haloalkyl groups are chloromethyl, chloroethyl, dichloroethyl, trifluoromethyl, difluoromethyl, monofluoromethyl and the like.

As used herein, the terms "halogen", "halogen atom", "halo" and the like represent fluorine, chlorine, bromine or iodine, especially fluorine, chlorine or bromine.

The term "amino" as used herein refers to $-NH_2$.

The term "hydroxyl" as used herein refers to -OH.

The term "alkoxy" as used herein refers to an alkyl as defined above that is attached to a parent molecular moiety through an oxygen atom. The term "$C_{1-6}$ alkoxy" refers to an alkoxy having a specified number of carbon atoms, which can include its subgroups, such as $C_{1-6}$ alkoxy, $C_{1-4}$ alkoxy, $C_{1-3}$ alkoxy, $C_{1-2}$ alkoxy, $C_{2-5}$ alkoxy, $C_{2-4}$ alkoxy, etc. Typical examples of "alkoxy" include, but are not limited to, methoxy, ethoxy, n-propoxy, isopropoxy, n-butoxy, isobutoxy, tert-butoxy, sec-butoxy, n-pentyloxy, n-hexyloxy, 1,2-dimethylbutoxy, etc.

The term "$C_{1-6}$ haloalkyl" as used herein refers to a $C_{1-6}$ alkyl as defined above that is mono-or polysubstituted by halogen such as fluorine, chlorine, bromine or iodine. Representative examples of $C_{1-6}$ haloalkyl include, but are not limited to, chloromethyl, chloroethyl, dichloroethyl, trifluoromethyl, difluoromethyl, monofluoromethyl, and the like.

The term "alkyl-amino" as used herein refers to an amino group monosubstituted by an alkyl as defined above. The term "$C_{1-6}$ alkyl-amino" as used herein refers to an amino group monosubstituted by a $C_{1-6}$ alkyl as defined above. Typical examples of "$C_{1-6}$ alkyl-amino" include, but are not limited to, methylamino, ethylamino, propylamino, butylamino and the like.

The term "dialkyl-amino" as used herein refers to an amino group disubstituted with alkyl as defined above. The term "di($C_{1-6}$ alkyl)-amino" as used herein refers to an amino group disubstituted with $C_{1-6}$ alkyl as defined above. Typical examples of "di($C_{1-6}$ alkyl)-amino" include, but are not limited to, dimethylamino, diethylamino, dipropylamino, dibutylamino and the like.

The term "3- to 14-membered substituted or unsubstituted cycloalkyl" as used herein refers to a saturated cyclic hydrocarbonyl having 3 to 14 carbon atoms and having monocyclic or bicyclic or multiple rings (including fused or bridged ring system), for example, having 3 to 8, 5 to 8, 3 to 6 or 5 to 6 carbon atoms, and the group is unsubstituted or substituted. Typical examples of "3- to 14-membered cycloalkyl" include, but are not limited to, monocyclic structures such as cyclopropyl, cyclobutyl, cyclopentyl, cyclohexyl, cycloheptyl, cyclooctyl, 1-methylcyclopropyl, 2-methylcyclopentyl, 2-methylcyclooctyl, bicyclic structures such as bicyclo[2.2.1]heptyl, and polycyclic structures such as adamantyl, etc.

The term "3- to 14-membered substituted or unsubstituted heterocyclyl" as used herein refers to a saturated or partially saturated and non-aromatic cyclic group containing at least one heteroatom (e.g., containing 1, 2, 3, 4 or 5), having 3 to 14 ring atoms and having monocyclic ring or bicyclic ring or fused ring of multiple rings, in which the heteroatom is nitrogen atom, oxygen atom and/or sulfur atom, and the group is unsubstituted or substituted. The "3- to 14-membered substituted or unsubstituted heterocyclyl" can be oxo or thio. For example, specific examples of the "3- to 14-membered substituted or unsubstituted heterocyclyl" include but are not limited to: azetidinyl, 1,4-dioxanyl, 1,3-dioxanyl, 1,3-dioxolanyl, 1,4-dioxacyclohexadienyl, tetrahydrofuryl, dihydropyrrolyl, pyrrolidinyl, imidazolidinyl, 4,5-dihydroimidazolyl, pyrazolidyl, 4,5-dihydropyrazolyl, 2,5-dihydrothienyl, tetrahydrothienyl, piperazinyl, thiazinyl, piperidinyl, morpholinyl, etc.

The term "6- to 14-membered substituted or unsubstituted aryl" as used herein refers to an unsaturated aromatic carbocyclic group having 6 to 14 carbon atoms and having a monocyclic ring or fused ring of two or more rings, and the group is unsubstituted or substituted. The aryl has, for example, 5 to 8 or 5 to 6 carbon atoms. Typical examples of the aryl include, but are not limited to, phenyl, naphthyl, anthracenyl and the like.

The term "6- to 14-membered substituted or unsubstituted heteroaryl" as used herein refers to a heteroaromatic cyclic group having 6 to 14 ring members, comprising monocyclic heteroaromatic ring and polycyclic aromatic rings, and in the polycyclic aromatic ring, a monocyclic aromatic ring is fused with one or more other aromatic rings, and the group is unsubstituted or substituted. The "6- to 14-membered heteroaryl" has one or two or more heteroatoms selected from O, S and N. The term "heteroaryl" as used herein also includes groups in which an aromatic ring is fused with one or more non-aromatic rings (carbocyclic or heterocyclic rings), wherein the linking group or site is located on the aromatic ring or non-aromatic ring. Typical examples of "6- to 14-membered heteroaryl" include, but are not limited to, furyl, imidazolyl, triazolyl, indolyl, tetrazolyl, pyridyl, pteridyl, pyrimidyl, triazolyl, quinolyl, isoquinolyl, quinazolinyl, quinoxalinyl and the like.

The term "7- to 12-membered substituted or unsubstituted bridged-ring group" used in the present application

refers to a ring system group with 7 to 12 ring atoms formed by two or more cyclic structures that share two atoms that are not directly connected to each other, and the group is unsubstituted or substituted.

The term "$C_{2-10}$ alkanoyl" used in the present application refers to an "alkyl-C(O)-" having 2 to 12 carbon atoms.

The term "alkenyl" as used in the present application refers to a branched and unbranched unsaturated hydrocarbonyl containing at least one double bond.

The term "polyenyl" as used in the present application refers to a branched and unbranched unsaturated hydrocarbonyl containing at least two double bonds.

The term "$C_{2-12}$ alkenyl" used in the present application refers to an alkenyl having 2 to 12 carbon atoms, such as "$C_{2-6}$ alkenyl" having 2 to 6 carbon atoms. Specific examples include vinyl, 1-methyl-1-vinyl, 2,2-dimethyl-1-vinyl, 1-propenyl, 2-propenyl (allyl), 1-butenyl, 2-butenyl, 3-butenyl, 4-pentenyl, 1-methyl-4-pentenyl, 3-methyl-1-pentenyl, 1-hexenyl, 2-hexenyl and the like.

The term "$C_{2-12}$ polyenyl" used in the present application refers to a polyenyl group having 2 to 12 carbon atoms.

The term "$C_{2-12}$ alkenoyl" used in the present application refers to an "alkenyl-C(O)-" having 2 to 12 carbon atoms.

The term "$C_{2-12}$ polyenoyl" used in the present application refers to a "polyenyl-C(O)-" having 2 to 12 carbon atoms.

When the name of the compound as used herein is inconsistent with the chemical structural formula, the chemical structural formula shall prevail.

## Brief Description of the Drawings

Figure 1 shows the Western blot result of the compound.

## Specific Models for Carrying Out the Application

The content of the present application will be described in detail through the following examples. In the present application, the following examples are used to better illustrate the application, and not used to limit the scope of the application.

Example 1

Synthesis of (*E*)-6-[3-(6-amino)pyridyl]-4-[3-(trifluoromethyl)phenyl]aminoquinoline-3-butenone (HTL-2-34)

80mL microwave tube was taken, to which (*E*)-4-{6-bromo-4-[3-(trifluoromethylphenyl)amino]}quinoline-3-butenone (4.34 g, 10 mmol), $Pd(Ph_3P)_4$ (1.16 g, 1 mmol), $K_2CO_3$ (2.76 g, 20 mmol) and 6-aminopyridineboronic acid (1.66 g, 12 mmol) were added and dissolved in a solution of 1,4-dioxane. The microwave tube was sealed with cover, placed in a microwave reactor, the temperature was set to 100°C, the reaction was carried out for 40 minutes, the microwave tube was taken out, and cooled to room temperature. The reaction solution was transferred to a separatory funnel, to which water was added, and the resulting mixture was extracted with ethyl acetate. The organic layer was washed with saturated NaCl, dried with anhydrous $Na_2SO_4$, filtered, the filtrate was dried by a rotary evaporator and purified with column chromatography (n-hexane/ethyl acetate 5:1) to obtain 3.71 g of a yellow solid, with a yield of 83%. $^1$H-NMR (400 MHz, DMSO-$D_6$) $\delta$ (ppm): 9.40 (s, 1H), 9.05 (s, 1H), 8.35 (d, *J* = 10.6 Hz, 2H), 8.02 (d, *J* = 9.9 Hz, 2H), 7.81 (d, *J* = 8.5 Hz, 1H), 7.49 - 7.40 (m, 2H), 7.22 (d, *J* = 7.6 Hz, 1H), 7.13 (d, *J* = 8.1 Hz, 2H), 6.88 (d, *J* = 16.5 Hz, 1H), 6.56 (d, *J* = 8.6 Hz, 1H), 6.20 (s, 2H), 1.99 (d, *J* = 11.0 Hz, 3H). MS (ESI): m/z 449.15 $[M + H]^+$. Mp 172-175°C.

Example 2

Synthesis of (*E*)-6-(4-amino)phenyl-4-[3-(trifluoromethyl)phenyl]aminoquinoline-3-butenone (HTL-2-35)

The synthesis was carried out according to the synthetic method of HTL-2-34 to obtain a yellow solid, with a total yield of 29%. $^1$H-NMR (400 MHz, DMSO-$D_6$) $\delta$ (ppm): 9.46 (s, 1H), 9.06 (s, 1H), 8.37 (d, *J* = 1.6 Hz, 1H), 8.03 (d, *J* = 8.7 Hz, 1H), 7.96 (dd, *J* = 8.7, 1.8 Hz, 1H), 7.42 (t, *J* = 3.8 Hz, 1H), 7.38 (d, *J* = 4.4 Hz, 1H), 7.17 (d, *J* = 7.8 Hz, 1H), 7.13 - 7.07 (m, 3H), 6.92 (d, *J* = 1.7 Hz, 1H), 6.87 (s, 1H), 6.84 (d, *J* = 5.0 Hz, 1H), 6.59 (dd, *J* = 8.0, 1.4 Hz, 1H), 5.18 (s, 2H), 1.97 (s, 3H). MS (ESI): m/z 448.16 $[M + H]^+$. Mp 183-185°C.

Example 3

Synthesis of (*E*)-6-[3-(6-methyl)pyridyl]-4-[3-(trifluoromethyl)phenyl]aminoquinoline-3-butenone (HTL-2-3 8)

The synthesis was carried out according to the synthetic method of HTL-2-34 to obtain a yellow solid, with a total yield of 19%. $^1$H-NMR (400 MHz, DMSO-$D_6$) δ (ppm): 9.43 (s, 1H), 9.04 (s, 1H), 8.81 (d, *J* = 2.0 Hz, 1H), 8.47 (s, 1H), 8.10 - 7.98 (m, 3H), 7.45 - 7.30 (m, 3H), 7.19 (d, *J* = 7.8 Hz, 1H), 7.12 (d, *J* = 8.9 Hz, 2H), 6.83 (d, *J* = 16.5 Hz, 1H), 2.47 (s, 3H), 1.95 (s, 3H). MS (ESI): m/z 448.16 $[M + H]^+$. Mp 172-174°C.

Example 4

Synthesis of (*E*)-6-(3-aminophenyl)-4-[3-(trifluoromethyl)phenyl]aminoquinoline-3-butenone (HTL-2-42)

The synthesis was carried out according to the synthetic method of HTL-2-34 to obtain a yellow solid, with a total yield of 19%. $^1$H-NMR (400 MHz, DMSO-$D_6$) δ (ppm): 9.45 (s, 1H), 9.06 (s, 1H), 8.35 (s, 1H), 8.03 (d, *J* = 8.7 Hz, 1H), 7.96 (dd, *J* = 8.7, 1.5 Hz, 1H), 7.40 (dd, *J* = 15.8, 7.0 Hz, 2H), 7.18 (d, *J* = 7.7 Hz, 1H), 7.09 (dd, *J* = 7.9, 4.5 Hz, 3H), 6.86 (dd, *J* = 24.7, 8.4 Hz, 3H), 6.57 (d, *J* = 6.7 Hz, 1H), 5.18 (s, 2H), 1.97 (s, 3H). MS (ESI): m/ z 448.16 $[M + H]^+$. Mp 184-186°C.

Example 5

Synthesis of (*E*)-6-(4-pyridyl)-4-[3-(trifluoromethyl)phenyl]aminoquinoline-3-butenone (HTL-3-04)

The synthesis was carried out according to the synthetic method of HTL-2-34 to obtain a yellow solid, with a total yield of 21%. $^1$H-NMR (400 MHz, DMSO-$D_6$) δ (ppm): 9.51 (s, 1H), 9.08 (s, 1H), 8.69 - 8.61 (m, 3H), 8.18 (dd, *J* = 8.7, 1.9 Hz, 1H), 8.09 (d, *J* = 8.7 Hz, 1H), 7.79 (dd, *J* = 4.6, 1.5 Hz, 2H), 7.43 (dd, *J* = 10.2, 6.3 Hz, 1H), 7.34 (d, *J* = 16.5 Hz, 1H), 7.21 (d, *J* = 7.7 Hz, 1H), 7.17 - 7.11 (m, 2H), 6.83 (d, *J* = 16.5 Hz, 1H), 1.94 (s, 3H). MS (ESI): m/z 434.14 $[M + H]^+$. Mp 192-195°C.

Example 6

Synthesis of (*E*)-6-[3-(5-methoxy)pyridyl]-4-[3-(trifluoromethyl)phenyl]aminoquinoline-3-butenone (HTL-3-07)

The synthesis was carried out according to the synthetic method of HTL-2-34 to obtain a yellow solid, with a total yield of 20%. $^1$H-NMR (400 MHz, DMSO-$D_6$) δ (ppm): 9.49 (s, 1H), 9.10 (s, 1H), 8.56 (dd, *J* = 25.3, 1.4 Hz, 2H), 8.33 (d, *J* = 2.7 Hz, 1H), 8.19 (dd, *J* = 8.7, 1.7 Hz, 1H), 8.10 (d, *J* = 8.7 Hz, 1H), 7.72 - 7.64 (m, 1H), 7.52 - 7.36 (m, 2H), 7.24 (d, *J* = 7.7 Hz, 1H), 7.16 (d, *J* = 6.9 Hz, 2H), 6.88 (d, *J* = 16.4 Hz, 1H), 3.90 (s, 3H), 2.00 (s, 3H). MS (ESI): m/z 464.15 $[M + H]^+$. Mp 164-167°C.

Example 7

Synthesis of (*E*)-6-(4-hydroxy)phenyl-4-[3-(trifluoromethyl)phenyl]aminoquinoline-3-butenone (HTL-3-11)
The synthesis was carried out according to the synthetic method of HTL-2-34 to obtain a yellow solid, with a total yield of 22%. $^1$H-NMR (400 MHz, DMSO-$D_6$) δ (ppm): 9.68 (s, 1H), 9.47 (s, 1H), 9.07 (s, 1H), 8.38 (s, 1H), 8.12 - 8.00 (m, 2H), 7.62 (d, *J* = 8.6 Hz, 2H), 7.45 (dd, *J* = 16.1, 8.9 Hz, 2H), 7.23 (d, *J* = 7.7 Hz, 1H), 7.15 (d, *J* = 8.8 Hz, 2H), 6.98 - 6.81 (m, 3H), 2.00 (d, *J* = 7.8 Hz, 3H). MS (ESI): m/z 449.14 $[M + H]^+$. Mp 218-222°C.

Example 8

Synthesis of (*E*)-6-(4-fluoro)phenyl-4-[3-(tiifluoromethyl)phenyl]aminoquinoline-3-butenone (HTL-3-12)

The synthesis was carried out according to the synthetic method of HTL-2-34 to obtain a green solid, with a total yield of 24%. $^1$H-NMR (400 MHz, DMSO-$D_6$) δ (ppm): 9.50 (s, 1H), 9.09 (s, 1H), 8.47 (s, 1H), 8.14 - 8.04 (m, 2H), 7.83 (dd, *J* = 8.6, 5.5 Hz, 2H), 7.40 (ddd, *J* = 26.4, 17.1, 8.5 Hz, 4H), 7.22 (d, *J* = 7.7 Hz, 1H), 7.15 (d, *J* = 5.9 Hz, 2H), 6.88 (d, *J* = 16.5 Hz, 1H), 1.99 (s, 3H). MS (ESI): m/z 451.14 $[M + H]^+$. Mp 167-169°C.

Example 9

Synthesis of (*E*)-6-(4-cyano)phenyl-4-[3-(trifluoromethyl)phenyl]aminoquinoline-3-butenone (HTL-3-15)

The synthesis was carried out according to the synthetic method of HTL-2-34 to obtain a yellow solid, with a total yield of 25%. $^1$H-NMR (400 MHz, DMSO-$D_6$) δ (ppm): 9.54 (s, 1H), 9.07 (s, 1H), 8.58 (d, *J* = 1.5 Hz, 1H), 8.15 (dd, *J* = 8.8, 1.8 Hz, 1H), 8.09 (d, *J* = 8.7 Hz, 1H), 7.97 (q, *J* = 8.6 Hz, 4H), 7.45 (t, *J* = 7.8 Hz, 1H), 7.34 (d, *J* = 16.5 Hz, 1H), 7.23 (d, *J* = 7.6 Hz, 1H), 7.16 (d, *J* = 8.0 Hz, 2H), 6.83 (d, *J* = 16.5 Hz, 1H), 1.95 (s, 3H). MS (ESI): m/z 458.14 $[M + H]^+$. Mp 173 -174°C.

Example 10

Synthesis of (*E*)-6-[4-(trifluoromethyl)phenyl]-4-[3-(trifluoromethyl)phenyl] aminoquinoline-3-butenone (HTL-3-18)

The synthesis was carried out according to the synthetic method of HTL-2-34 to obtain a yellow solid, with a total yield of 23%. $^1$H-NMR (400 MHz, DMSO-$D_6$) δ (ppm): 9.53 (s, 1H), 9.11 (s, 1H), 8.59 (d, *J* = 1.6 Hz, 1H), 8.15 (dt, *J* = 18.4, 5.2 Hz, 2H), 8.01 (d, *J* = 8.2 Hz, 2H), 7.86 (d, *J* = 8.3 Hz, 2H), 7.51 - 7.34 (m, 2H), 7.23 (d, *J* = 7.8 Hz, 1H), 7.16 (d, *J* = 6.7 Hz, 2H), 6.88 (d, *J* = 16.5 Hz, 1H), 1.98 (s, 3H). MS (ESI): m/z 501.13 $[M + H]^+$. Mp 189 -191°C.

Example 11

Synthesis of (*E*)-6-(3-ethoxy)phenyl-4-[3-(trifluoromethyl)phenyl]aminoquinoline-3-butenone (HTL-3-16)

The synthesis was carried out according to the synthetic method of HTL-2-34 to obtain a yellow solid, with a total yield of 19%. $^1$H-NMR (400 MHz, DMSO-$D_6$) δ (ppm): 9.51 (s, 1H), 9.07 (s, 1H), 8.42 (d, *J* = 1.5 Hz, 1H), 8.08 (dt, *J* = 16.3, 5.2 Hz, 2H), 7.71 (d, *J* = 8.8 Hz, 2H), 7.44 (dd, *J* = 22.5, 12.1 Hz, 2H), 7.23 (d, *J* = 7.7 Hz, 1H), 7.15 (d, *J* = 7.3 Hz, 2H), 7.04 (d, *J* = 8.8 Hz, 2H), 6.87 (d, *J* = 16.5 Hz, 1H), 4.07 (q, *J* = 7.0 Hz, 2H), 1.99 (s, 3H), 1.34 (t, *J* = 7.0 Hz, 3H). MS (ESI): m/z 477.17 $[M + H]^+$. Mp 184-185°C.

Example 12

Synthesis of (*E*)-6-(3-furyl)-4-[3-(trifluoromethyl)phenyl]aminoquinoline-3-butenone (HTL-3-22)

The synthesis was carried out according to the synthetic method of HTL-2-34 to obtain a yellow solid, with a total yield of 23%. $^1$H-NMR (400 MHz, DMSO-$D_6$) δ (ppm): 9.37 (s, 1H), 9.04 (s, 1H), 8.37 (d, *J* = 1.3 Hz, 1H), 8.30 (s, 1H), 8.04 (dt, *J* = 24.3, 5.1 Hz, 2H), 7.79 (t, *J* = 1.5 Hz, 1H), 7.47 - 7.35 (m, 2H), 7.22 (d, *J* = 7.7 Hz, 1H), 7.14 (d, *J* = 7.4 Hz, 2H), 6.98 (d, *J* = 0.8 Hz, 1H), 6.86 (d, *J* = 16.5 Hz, 1H), 1.98 (s, 3H). MS (ESI): m/z 423.12 $(M + H]^+$. Mp 151-154°C.

Example 13

Synthesis of (*E*)-6-(2-thienyl)-4-[3-(trifluoromethyl)phenyl]aminoquinoline-3-butenone (HTL-3-24)

The synthesis was carried out according to the synthetic method of HTL-2-34 to obtain a yellow solid, with a total yield of 25%. $^1$H-NMR (400 MHz, DMSO-$D_6$) δ (ppm): 9.46 (s, 1H), 9.07 (s, 1H), 8.37 (d, *J* = 1.8 Hz, 1H), 8.10 (dd, *J* = 8.8, 2.0 Hz, 1H), 8.03 (d, *J* = 8.7 Hz, 1H), 7.62 (ddd, *J* = 6.1, 4.3, 1.0 Hz, 2H), 7.49 - 7.41 (m, 2H), 7.24 - 7.11 (m, 4H), 6.90 (d, *J* = 16.4 Hz, 1H), 2.03 (s, 3H). MS (ESI): m/z 439.10 $[M + H]^+$. Mp 181-185°C.

Example 14

Synthesis of (*E*)-6-(3-quinolyl)-4-[3-(trifluoromethyl)phenyl]aminoquinoline-3-butenone (HTL-3-25)

The synthesis was carried out according to the synthetic method of HTL-2-34 to obtain a yellow solid, with a total yield of 21%. $^1$H-NMR (400 MHz, DMSO-$D_6$) δ (ppm): 9.56 (s, 1H), 9.36 (d, *J* = 2.1 Hz, 1H), 9.12 (s, 1H), 8.74 (d, *J* = 7.3 Hz, 2H), 8.33 (d, *J* = 8.6 Hz, 1H), 8.17 (d, *J* = 8.7 Hz, 1H), 8.12 - 8.01 (m, 2H), 7.80 (t, *J* = 7.7 Hz, 1H), 7.67 (t, *J* = 7.5 Hz, 1H), 7.53 - 7.34 (m, 2H), 7.27 (d, *J* = 7.8 Hz, 1H), 7.21 (d, *J* = 1.9 Hz, 2H), 6.88 (d, *J* = 16.4 Hz, 1H), 1.99 (s, 3H). MS (ESI): m/z 484.16 $[M + H]^+$. Mp 173-176°C.

Example 15

Synthesis of (*E*)-6-(1H-4-pyrazolyl)-4-[3-(trifluoromethyl)phenyl]aminoquinoline-3-butenone (HTL-3-26)

The synthesis was carried out according to the synthetic method of HTL-2-34 to obtain a yellow solid, with a total yield of 18%. $^1$H-NMR (400 MHz, DMSO-$D_6$) $\delta$ (ppm): 13.05 (s, 1H), 9.37 (s, 1H), 9.02 (s, 1H), 8.39 (s, 1H), 8.03 (dd, *J* = 36.9, 8.7 Hz, 4H), 7.52 - 7.33 (m, 2H), 7.23 (d, *J* = 7.6 Hz, 1H), 7.15 (d, *J* = 8.0 Hz, 2H), 6.87 (d, *J* = 16.5 Hz, 1H), 1.99 (s, 3H). MS (ESI): m/z 423.14 $[M + H]^+$. Mp 177-179°C.

Example 16

Synthesis of (*E*)-6-(4-fluoro-3-methyl)phenyl-4-[3-(trifluoromethyl)phenyl] aminoquinoline-3-butenone (HTL-3-32)

The synthesis was carried out according to the synthetic method of HTL-2-34 to obtain a yellow solid, with a total yield of 19%. $^1$H-NMR (400 MHz, DMSO-$D_6$) $\delta$ (ppm): 10.54 (s, 1H), 9.07 (s, 1H), 8.74 (s, 1H), 8.24 (dd, *J* = 8.8, 1.6 Hz, 1H), 8.13 (d, *J* = 8.8 Hz, 1H), 7.73 (ddd, *J* = 10.4, 8.3, 4.8 Hz, 2H), 7.57 (t, *J* = 7.8 Hz, 1H), 7.43 (d, *J* = 7.4 Hz, 3H), 7.31 - 7.23 (m, 1H), 7.17 (d, *J* = 16.4 Hz, 1H), 6.74 (d, *J* = 16.4 Hz, 1H), 2.32 (d, *J* = 1.2 Hz, 3H), 1.89 (s, 3H). MS (ESI): m/z 465.15 $[M + H]^+$. Mp 209-210°C.

Example 17

Synthesis of (*E*)-6-(2,4-difluoro)phenyl-4-[3-(trifluoromethyl)phenyl]aminoquinoline-3-butenone (HTL-3-33)

The synthesis was carried out according to the synthetic method of HTL-2-34 to obtain a yellow solid, with a total yield of 20%. $^1$H-NMR (400 MHz, DMSO-$D_6$) $\delta$ (ppm): 10.99 (s, 1H), 9.12 (s, 1H), 8.84 (s, 1H), 8.25 (d, *J* = 8.8 Hz, 1H), 8.17 (d, *J* = 8.7 Hz, 1H), 7.81 (td, *J* = 8.9, 6.7 Hz, 1H), 7.65 - 7.42 (m, 5H), 7.29 (td, *J* = 8.5, 2.4 Hz, 1H), 7.06 (d, *J* = 16.4 Hz, 1H), 6.70 (d, *J* = 16.4 Hz, 1H), 1.85 (s, 3H). MS (ESI): m/z 469.13 $[M + H]^+$. Mp 162-164 ° C.

Example 18

Synthesis of (*E*)-6-(3,4-difluoro)phenyl-4-[3-(trifluoromethyl)phenyl]aminoquinoline-3-butenone (HTL-3-34)

The synthesis was carried out according to the synthetic method of HTL-2-34 to obtain a yellow solid, with a total yield of 23%. $^1$H-NMR (400 MHz, DMSO-$D_6$) $\delta$ (ppm): 11.42 (s, 1H), 9.13 (d, *J* = 1.3 Hz, 1H), 9.05 (s, 1H), 8.39 (dd, *J* = 8.9, 1.7 Hz, 1H), 8.23 (d, *J* = 8.9 Hz, 1H), 8.14 (ddd, *J* = 12.2, 7.7, 2.2 Hz, 1H), 7.85 (dd, *J* = 5.8, 2.8 Hz, 1H), 7.62 (ddd, *J* = 23.6, 9.2, 2.8 Hz, 5H), 6.92 (d, *J*= 16.4 Hz, 1H), 6.63 (d, *J* = 16.3 Hz, 1H), 1.79 (s, 3H). MS (ESI): m/z 469.13 $[M + H]^+$. Mp 210-213°C.

Example 19

Synthesis of (*E*)-6-(3-chloro)phenyl-4-[3-(trifluoromethyl)phenyl]aminoquinoline-3-butenone (HTL-3-36)

The synthesis was carried out according to the synthetic method of HTL-2-34 to obtain a yellow solid, with a total yield of 27%. $^1$H-NMR (400 MHz, DMSO-$D_6$) $\delta$ (ppm): 9.62 (s, 1H), 9.11 (d, *J* = 7.8 Hz, 1H), 8.54 (d, *J* = 1.6 Hz, 1H), 8.21 - 8.06 (m, 2H), 7.89 - 7.73 (m, 2H), 7.58 - 7.45 (m, 3H), 7.39 (d, *J* = 16.5 Hz, 1H), 7.27 (d, *J* = 7.7 Hz, 1H), 7.21 (d, *J* = 6.3 Hz, 2H), 6.91 - 6.82 (m, 1H), 1.99 (s, 3H). MS (ESI): m/z 467.11 $[M + H]^+$. Mp 182-183°C.

Example 20

Synthesis of (*E*)-6-(4-chloro)phenyl-4-[3-(trifluoromethyl)phenyl]aminoquinoline-3-butenone (HTL-3-37)

It was synthesized according to the synthetic method of HTL-2-34 to obtain a yellow solid, with a total yield of 28%. $^1$H-NMR (400 MHz, DMSO-$D_6$) $\delta$ (ppm): 9.95 (s, 1H), 9.07 (s, 1H), 8.64 (d, *J* = 1.6 Hz, 1H), 8.16 (dd, *J* = 8.8, 1.9 Hz, 1H), 8.09 (d, *J* = 8.7 Hz, 1H), 7.90 - 7.81 (m, 2H), 7.61 - 7.53 (m, 2H), 7.49 (t, *J* = 8.2 Hz, 1H), 7.35 - 7.18 (m, 4H), 6.81 (d, *J* = 16.4 Hz, 1H), 1.93 (s, 3H). MS (ESI): m/z 467.11 $[M + H]^+$. Mp 197-199°C.

Example 21

Synthesis of (*E*)-6-(4-isopropyl)phenyl-4-[3-(trifluoromethyl)phenyl]aminoquinoline-3-butenone (HTL-3-39)

The synthesis was carried out according to the synthetic method of HTL-2-34 to obtain a yellow solid, with a total yield of 26%. $^1$H-NMR (400 MHz, DMSO-$D_6$) δ (ppm): 10.04 (s, 1H), 9.09 (s, 1H), 8.61 (s, 1H), 8.19 (dd, *J* = 8.8, 1.8 Hz, 1H), 8.11 (d, *J* = 8.7 Hz, 1H), 7.74 (d, *J* = 8.3 Hz, 2H), 7.52 (t, *J* = 7.9 Hz, 1H), 7.33 (ddd, *J* = 16.4, 15.2, 6.4 Hz, 6H), 6.82 (d, *J* = 16.4 Hz, 1H), 2.96 (dt, *J* = 13.7, 6.9 Hz, 1H), 1.95 (s, 3H), 1.24 (d, *J* = 6.9 Hz, 6H). MS (ESI): m/z 475.19 $[M + H]^+$. Mp 143-144°C.

Example 22

Synthesis of (*E*)-6-(4-propyl)phenyl-4-[3-(trifluoromethyl)phenyl]aminoquinoline-3-butenone (HTL-3-40)

The synthesis was carried out according to the synthetic method of HTL-2-34 to obtain a yellow solid, with a total yield of 25%. $^1$H-NMR (400 MHz, DMSO-$D_6$) δ (ppm): 9.58 (s, 1H), 9.08 (s, 1H), 8.47 (d, *J* = 1.6 Hz, 1H), 8.10 (dt, *J* = 16.6, 5.3 Hz, 2H), 7.68 (d, *J* = 8.2 Hz, 2H), 7.50 - 7.35 (m, 2H), 7.29 (t, *J* = 11.5 Hz, 2H), 7.28 - 7.20 (m, 1H), 7.17 (d, *J* = 7.6 Hz, 2H), 6.87 (d, *J* = 16.5 Hz, 1H), 2.65 - 2.52 (m, 2H), 1.99 (s, 3H), 1.67 - 1.52 (m, 2H), 0.90 (t, *J* = 7.3 Hz, 3H). MS (ESI): m/z 475.19 $[M + H]^+$. Mp 166-169°C.

Example 23

Synthesis of (*E*)-6-(4-isobutyl)phenyl-4-[3-(trifluoromethyl)phenyl]aminoquinoline-3-butenone (HTL-3-41)

The synthesis was carried out according to the synthetic method of HTL-2-34 to obtain a yellow solid, with a total yield of 21%. $^1$H-NMR (400 MHz, DMSO-$D_6$) δ (ppm): 9.64 (s, 1H), 9.08 (s, 1H), 8.50 (d, *J* = 1.5 Hz, 1H), 8.11 (dt, *J* = 19.8, 5.3 Hz, 2H), 7.69 (d, *J* = 8.2 Hz, 2H), 7.47 (t, *J* = 8.0 Hz, 1H), 7.38 (d, *J* = 16.5 Hz, 1H), 7.26 (dd, *J* = 10.3, 8.3 Hz, 3H), 7.18 (d, *J* = 6.9 Hz, 2H), 6.86 (d, *J* = 16.4 Hz, 1H), 2.49 - 2.45 (m, 2H), 1.97 (s, 3H), 1.91 - 1.81 (m, 1H), 0.86 (t, *J* = 7.6 Hz, 6H). MS (ESI): m/z 489.21 $[M + H]^+$. Mp 187-191°C.

Example 24

Synthesis of (*E*)-6-(4-butyl)phenyl-4-[3-(trifluoromethyl)phenyl]aminoquinoline-3-butenone (HTL-3-42)

The synthesis was carried out according to the synthetic method of HTL-2-34 to obtain a yellow solid, with a total yield of 25%. $^1$H-NMR (400 MHz, DMSO-$D_6$) δ (ppm): 9.60 (s, 1H), 9.04 (s, 1H), 8.45 (d, *J* = 1.5 Hz, 1H), 8.05 (dt, *J* = 14.6, 5.2 Hz, 2H), 7.64 (d, *J* = 8.2 Hz, 2H), 7.43 (t, *J* = 7.9 Hz, 1H), 7.35 (d, *J* = 16.5 Hz, 1H), 7.26 (d, *J* = 8.2 Hz, 2H), 7.21 (d, *J* = 7.9 Hz, 1H), 7.15 (d, *J* = 11.7 Hz, 2H), 6.83 (d, *J* = 16.5 Hz, 1H), 2.57 (t, *J* = 7.6 Hz, 2H), 1.94 (s, 3H), 1.58 - 1.47 (m, 2H), 1.32 - 1.20 (m, 2H), 0.85 (t, *J* = 7.3 Hz, 3H). MS (ESI): m/z 489.21 $[M + H]^+$. Mp 149-150°C.

Example 25

Synthesis of (*E*)-6-[3-(6-fluoro)pyridyl]-4-[3-(trifluoromethyl)phenyl]aminoquinoline-3-butenone (HTL-3-43)

The synthesis was carried out according to the synthetic method of HTL-2-34 to obtain a yellow solid, with a total yield of 21%. $^1$H-NMR (400 MHz, DMSO-$D_6$) δ (ppm): 10.20 (s, 1H), 9.06 (s, 1H), 8.84 - 8.68 (m, 2H), 8.49 (td, *J* = 8.3, 2.7 Hz, 1H), 8.22 (dd, *J* = 8.8, 1.8 Hz, 1H), 8.11 (d, *J* = 8.7 Hz, 1H), 7.52 (t, *J* = 7.8 Hz, 1H), 7.43 - 7.24 (m, 4H), 7.18 (d, *J* = 16.4 Hz, 1H), 6.76 (d, *J* = 16.4 Hz, 1H), 1.90 (d, *J* = 3.9 Hz, 3H). MS (ESI): m/z 452.13 $[M + H]^+$. Mp 133-134°C.

Example 26

Synthesis of (*E*)-6-(4-carbamoyl)phenyl-4-[3-(trifluoromethyl)phenyl]aminoquinoline- 3-butenone (HTL-3-45)

The synthesis was carried out according to the synthetic method of HTL-2-34 to obtain a yellow solid, with a total yield of 26%. $^1$H-NMR (400 MHz, DMSO-$D_6$) δ (ppm): 11.32 (s, 1H), 9.10 (d, *J* = 29.5 Hz, 2H), 8.43 (dd, *J* = 8.8, 1.5 Hz, 1H), 8.25 (d, *J* = 8.8 Hz, 1H), 8.08 (d, *J* = 30.1 Hz, 5H), 7.73 - 7.49 (m, 4H), 7.46 (s, 1H), 6.98 (d, *J* = 16.3 Hz, 1H), 6.65 (d, *J* = 16.3 Hz, 1H), 1.81 (s, 3H). MS (ESI): m/z 476.15 $[M + H]^+$. Mp 211-214°C.

Example 27

Synthesis of (*E*)-6-[3-(5-cyano)pyridyl]-4-[3-(trifluoromethyl)phenyl]aminoquinoline-3-butenone (HTL-3-46)

The synthesis was carried out according to the synthetic method of HTL-2-34 to obtain a yellow solid, with a total yield of 22%. $^1$H-NMR (400 MHz, DMSO-$D_6$) δ (ppm): 11.17 (s, 1H), 9.42 (d, *J* = 2.3 Hz, 1H), 9.18 (t, *J* = 7.6 Hz, 1H), 9.13 - 8.97 (m, 2H), 8.88 (t, *J* = 2.1 Hz, 1H), 8.48 (dd, *J* = 8.9, 1.7 Hz, 1H), 8.22 (d, *J* = 8.8 Hz, 1H), 7.63 (ddd, *J* = 24.6, 18.2, 7.3 Hz, 4H), 7.01 - 6.86 (m, 1H), 6.62 (d, *J* = 16.3 Hz, 1H), 1.80 (s, 3H). MS (ESI): m/z 459.14 $[M + H]^+$. Mp 227-229°C.

Example 28

Synthesis of (*E*)-6-[3-(N-6-benzyloxyamido)pyridyl]-4-[3-(trifluoromethyl)phenyl] aminoquinoline-3-butenone (HTL-7-22)

The synthesis was carried out according to the synthetic method of HTL-2-34 to obtain a yellow solid, with a total yield of 24%. $^1$H-NMR (400 MHz, DMSO-$D_6$) δ (ppm): 10.48 (s, 1H), 9.56 (s, 1H), 9.07 (d, *J* = 11.6 Hz, 1H), 8.67 (s, 1H), 8.51 (s, 1H), 8.12 (ddd, *J* = 25.8, 18.6, 8.7 Hz, 3H), 7.94 (d, *J* = 8.8 Hz, 1H), 7.46 - 7.15 (m, 10H), 6.83 (d, *J* = 16.4 Hz, 1H), 5.18 (d, *J* = 11.0 Hz, 2H), 1.98 - 1.92 (m, 3H). MS (ESI): m/z 583.19 $[M + H]^+$. Mp 187-189°C.

Example 29

Synthesis of 8-[3-(6-amino)pyridyl]-1-[3-(trifluoromethyl)phenyl]oxazolo[5,4-*c*]quinolin-2(1*H*)-one (HTL-4-32)

80mL microwave tube was taken, to which 8-bromo-1-[3-(trifluoromethyl)phenyl]oxazolo[5,4-c]quinolin-2(1*H*)-one (4.08 g, 10 mmol), $Pd(Ph_3P)_4$ (1.16 g, 1 mmol), $K_2CO_3$ (2.76 g, 20 mmol) and 6-aminopyridineboronic acid (1.66 g, 12 mmol) were added and dissolved in a solution of 1,4-dioxane. The microwave tube was sealed with cover, placed in a microwave reactor, the temperature was set to 100°C, the reaction was carried out for 40 minutes, the microwave tube was taken out and cooled to room temperature. The reaction solution was transferred to a separatory funnel, diluted with water, and extracted with ethyl acetate. The organic layer was washed with saturated NaCl, dried with anhydrous $Na_2SO_4$, filtered, the filtrate was dried by a rotary evaporator, and purified with column chromatography (n-hexane/ethyl acetate 5:1) to obtain 3.59 g of a yellow solid, with a yield of 85%. $^1$H-NMR (400 MHz, DMSO-$D_6$) δ (ppm): 9.05 (s, 1H), 8.37 (s, 1H), 8.22 - 8.07 (m, 3H), 8.03 - 7.88 (m, 3H), 7.40 (d, *J* = 8.6 Hz, 1H), 6.95 (s, 1H), 6.45 (d, *J* = 8.6 Hz, 1H), 6.25 (s, 2H). MS (ESI): m/z 423.10 $[M + H]^+$. Mp 146-147°C.

Example 30

Synthesis of 8-(4-carbamoyl)phenyl-1-[3-(trifluoromethyl)phenyl]oxazolo[5,4-*c*]quinolin-2(1*H*)-one (HTL-5-21)

The synthesis was carried out according to the synthetic method of HTL-4-32 to obtain a yellow solid, with a total yield of 42%. $^1$H-NMR (400 MHz, DMSO-$D_6$) δ (ppm): 9.43 (s, 1H), 8.41 (d, *J* = 9.2 Hz, 2H), 8.28 - 8.21 (m, 2H), 8.19 (d, *J* = 7.9 Hz, 1H), 8.06 (dd, *J* = 18.3, 10.4 Hz, 2H), 7.92 (d, *J* = 8.3 Hz, 2H), 7.44 (d, *J* = 8.4 Hz, 3H), 7.19 (d, *J* = 1.7 Hz, 1H). MS (ESI): m/z 450.10 $[M + H]^+$. Mp 294-296°C.

Example 31

Synthesis of 8-[3-(6-methyl)pyridyl]-1-[3-(trifluoromethyl)phenyl]oxazolo[5,4-*c*] quinolin-2(1*H*)-one (HTL-5-23)

The synthesis was carried out according to the synthetic method of HTL-4-32 to obtain a yellow solid, with a total yield of 37%. $^1$H-NMR (400 MHz, DMSO-$D_6$) δ (ppm): 9.35 (s, 1H), 8.80 (d, *J* = 2.0 Hz, 1H), 8.40 - 8.33 (m, 2H), 8.26 (ddd, *J* = 15.9, 8.6, 4.5 Hz,3H), 8.11 (d, *J* = 8.0 Hz, 1H), 8.02 (t, *J* = 7.9 Hz, 1H), 7.91 (d, *J* = 8.4 Hz, 1H), 7.27 (d, *J* = 1.8 Hz, 1H), 2.75 (s, 3H). MS (ESI): m/z 422.10 $[M + H]^+$. Mp 299-300°C.

Example 32

Synthesis of 8-(3-furyl)-1-[3-(trifluoromethyl)phenyl]oxazolo[5,4-*c*]quinolin-2(1*H*)-one (HTL-5-25)

The synthesis was carried out according to the synthetic method of HTL-4-32 to obtain a yellow solid, with a total yield of 44%. $^1$H-NMR (400 MHz, DMSO-$D_6$) δ (ppm): 9.06 (s, 1H), 8.35 (s, 1H), 8.18 (t, *J* = 8.5 Hz, 2H), 8.11 - 8.07

(m, 2H), 8.03 (t, *J* = 7.9 Hz, 1H), 7.97 (dd, *J* = 8.9, 1.9 Hz, 1H), 7.75 (t, *J* = 1.7 Hz, 1H), 6.91 (d, *J* = 1.8 Hz, 1H), 6.16 (dd, *J* = 1.8, 0.7 Hz, 1H). MS (ESI): m/z 397.07 $[M + H]^+$. Mp 276-277°C.

Example 33

Synthesis of 8-(2-thienyl)-1-[3-(trifluoromethyl)phenyl]oxazolo[5,4-*c*]quinolin-2(1*H*)-one (HTL-5-26)

The synthesis was carried out according to the synthetic method of HTL-4-32 to obtain a yellow solid, with a total yield of 41%. $^1$H-NMR (400 MHz, DMSO-$D_6$) δ (ppm): 9.08 (s, 1H), 8.37 (s, 1H), 8.20 (t, *J* = 8.4 Hz, 2H), 8.11 (dd, *J* = 10.2, 5.4 Hz, 2H), 8.05 (dd, *J* = 13.3, 5.3 Hz, 1H), 7.58 (dd, *J* = 5.1, 0.8 Hz, 1H), 7.45 - 7.42 (m, 1H), 7.12 (dd, *J* = 5.0, 3.7 Hz, 1H), 7.07 (d, *J* = 1.6 Hz, 1H). MS (ESI): m/z 413.05 $[M + H]^+$. Mp 272-274°C.

Example 34

Synthesis of 8-(4-ethoxy)phenyl-1-[3-(trifluoromethyl)phenyl]oxazolo[5,4-*c*]quinolin-2(1*H*)-one (HTL-5-27)

The synthesis was carried out according to the synthetic method of HTL-4-32 to obtain a yellow solid, with a total yield of 39%. $^1$H-NMR (400 MHz, DMSO-$D_6$) δ (ppm): 9.08 (s, 1H), 8.38 (s, 1H), 8.16 (dd, *J* = 25.3, 8.3 Hz, 2H), 8.00 (t, *J* = 8.1 Hz, 2H), 7.29 (d, *J* = 8.6 Hz, 2H), 7.04 (s, 1H), 6.92 (d, *J* = 8.6 Hz, 2H), 4.05 (q, *J* = 6.9 Hz, 2H), 1.33 (t, *J* = 6.9 Hz, 3H). MS (ESI): m/z 451.12 $[M + H]^+$. Mp 254-254°C.

Example 35

Synthesis of 8-[3-(6-fluoro)pyridyl]-1-[3-(trifluoromethyl)phenyl]oxazolo[5,4-*c*]quinolin-2(1*H*)-one (HTL-5-28)

The synthesis was carried out according to the synthetic method of HTL-4-32 to obtain a yellow solid, with a total yield of 36%. $^1$H-NMR (400 MHz, DMSO-$D_6$) δ (ppm): 9.15 (s, 1H), 8.35 (s, 1H), 8.23- 8.17 (m, 3H), 8.11 (d, *J* = 7.9 Hz, 1H), 8.06 - 7.96 (m, 3H), 7.26 (dd, *J* = 8.4, 2.7 Hz, 1H), 7.11 (d, *J* = 1.8 Hz, 1H). MS (ESI): m/z 426.08 $[M + H]^+$. Mp 262-263°C.

Example 36

Synthesis of 8-(4-trifluoromethyl)phenyl-1-[3-(trifluoromethyl)phenyl]oxazolo[5,4-*c*] quinolin-2(1*H*)-one (HTL-5-29)

The synthesis was carried out according to the synthetic method of HTL-4-32 to obtain a yellow solid, with a total yield of 41%. $^1$H-NMR (400 MHz, DMSO-$D_6$) δ (ppm): 9.16 (s, 1H), 8.38 (s, 1H), 8.24 - 8.18 (m, 2H), 8.13 (d, *J* = 8.0 Hz, 1H), 8.07 (dd, *J* = 8.9, 2.0 Hz, 1H), 8.00 (t, *J* = 7.9 Hz, 1H), 7.76 (d, *J* = 8.3 Hz, 2H), 7.57 (d, *J* = 8.2 Hz, 2H), 7.17 (d, *J* = 1.9 Hz, 1H). MS (ESI): m/z 475.08 $[M + H]^+$. Mp 265-266°C.

Example 37

Synthesis of 8-[3-(5-methoxy)pyridyl]-1-[3-(trifluoromethyl)phenyl]oxazolo[5,4-*c*] quinolin-2(1*H*)-one (HTL-5-30)

The synthesis was carried out according to the synthetic method of HTL-4-32 to obtain a yellow solid, with a total yield of 42%. $^1$H-NMR (400 MHz, DMSO-$D_6$) δ (ppm): 9.15 (s, 1H), 8.37 (s, 1H), 8.27 (d, *J* = 2.7 Hz, 1H), 8.21 (d, *J* = 8.9 Hz, 2H), 8.16 - 8.09 (m, 3H), 8.00 (t, *J* = 7.9 Hz, 1H), 7.31 - 7.28 (m, 1H), 7.14 (d, *J* = 1.8 Hz, 1H), 3.83 (s, 3H). MS (ESI): m/z 438.10 $[M + H]^+$. Mp 257-258°C.

Example 38

Synthesis of 8-(3-quinolyl)-1-[3-(trifluoromethyl)phenyl]oxazolo[5,4-*c*]quinolin- 2(1*H*)-one (HTL-5-32)

The synthesis was carried out according to the synthetic method of HTL-4-32 to obtain a yellow solid, with a total yield of 41%. $^1$H-NMR (400 MHz, DMSO-$D_6$) δ (ppm): 9.17 (s, 1H), 8.84 (d, *J* = 2.4 Hz, 1H), 8.43 - 8.39 (m, 2H), 8.28 - 8.22 (m, 3H), 8.18 (d, *J* = 8.0 Hz, 1H), 8.07 - 8.00 (m, 2H), 7.95 (d, *J* = 7.3 Hz, 1H), 7.82 - 7.77 (m, 1H), 7.70 - 7.66 (m, 1H), 7.28 (s, 1H). MS (ESI): m/z 458.10 $[M + H]^+$. Mp 242-244°C.

Example 39

Synthesis of 8-(4-chloro)phenyl-1-[3-(trifluoromethyl)phenyl]oxazolo[5,4-*c*]quinolin-2(1*H*)-one (HTL-5-33)

The synthesis was carried out according to the synthetic method of HTL-4-32 to obtain a yellow solid, with a total yield of 45%. $^1$H-NMR (400 MHz, DMSO-$D_6$) δ (ppm): 9.14 (d, *J* = 4.8 Hz, 1H), 8.39 (s, 1H), 8.22 - 8.16 (m, 2H), 8.13 (d, *J* = 7.9 Hz, 1H), 8.04 - 7.98 (m, 2H), 7.47 (d, *J* = 8.6 Hz, 2H), 7.38 (d, *J* = 8.6 Hz, 2H), 7.09 (d, *J* = 1.8 Hz, 1H). MS (ESI): m/z 441.05 $[M + H]^+$. Mp 283-286°C.

Example 40

Synthesis of 8-(4-propyl)phenyl-1-[3-(trifluoromethyl)phenyl]oxazolo[5,4-*c*]quinolin-2(1*H*)-one (HTL-5-34)

The synthesis was carried out according to the synthetic method of HTL-4-32 to obtain a yellow solid, with a total yield of 44%. $^1$H-NMR (400 MHz, DMSO-$D_6$) δ 9.11 (s, 1H), 8.40 (s, 1H), 8.21 - 8.12 (m, 3H), 8.01 (dt, *J* = 16.1, 5.4 Hz, 2H), 7.28 (d, *J* = 8.2 Hz, 2H), 7.21 (d, *J* = 8.2 Hz, 2H), 7.10 (d, *J* = 1.7 Hz, 1H), 2.59 - 2.53 (m, 2H), 1.58 (dd, *J* = 15.0, 7.4 Hz, 2H), 0.89 (t, *J* = 7.3 Hz, 3H). MS (ESI): m/z 449.14 $[M + H]^+$. Mp 220-221°C.

Example 41

Synthesis of 8-(4-isopropyl)phenyl-1-[3-(trifluoromethyl)phenyl]oxazolo[5,4-c]quinolin-2(1*H*)-one (HTL-5-35)

The synthesis was carried out according to the synthetic method of HTL-4-32 to obtain a yellow solid, with a total yield of 46%. $^1$H-NMR (400 MHz, DMSO-$D_6$) δ 9.10 (s, 1H), 8.39 (s, 1H), 8.21 - 8.12 (m, 3H), 8.01 (dd, *J* = 13.6, 4.8 Hz, 2H), 7.27 (q, *J* = 8.4 Hz, 4H), 7.11 (d, *J* = 1.8 Hz, 1H), 2.90 (dt, *J* = 13.7, 6.9 Hz, 1H), 1.19 (d, *J* = 6.9 Hz, 6H). MS (ESI): m/z 449.14 $[M + H]^+$. Mp 219-220°C.

Example 42

Synthesis of 8-[3-(5-cyano)pyridyl]-1-[3-(trifluoromethyl)phenyl]oxazolo[5,4-c]quinolin-2(1*H*)-one (HTL-5-36)

The synthesis was carried out according to the synthetic method of HTL-4-32 to obtain a yellow solid, with a total yield of 46%. $^1$H-NMR (400 MHz, DMSO-$D_6$) δ 9.18 (s, 1H), 9.01 (d, *J* = 1.7 Hz, 1H), 8.76 (d, *J* = 2.1 Hz, 1H), 8.39 - 8.33 (m, 2H), 8.22 (dd, *J* = 14.9, 8.4 Hz, 2H), 8.15 - 8.10 (m, 2H), 8.00 (t, *J* = 7.9 Hz, 1H), 7.18 (d, *J* = 1.5 Hz, 1H). MS (ESI): m/z 433.36 $[M + H]^+$. Mp 262-263°C.

Example 43

Synthesis of 8-[3-(6-valerylamino)pyridyl]-1-[3-(trifluoromethyl)phenyl]oxazolo[5,4-*c*] quinolin-2(1*H*)-one (WSX-1-24)

The synthesis was carried out according to the synthetic method of HTL-4-32 to obtain a yellow solid, with a total yield of 41%. $^1$H-NMR (400 MHz, DMSO-$D_6$) δ 10.61 (s, 1H), 9.09 (s, 1H), 8.35 (s, 1H), 8.24 - 8.04 (m, 5H), 8.05 - 7.93 (m, 2H), 7.77 (dd, *J* = 8.7, 2.3 Hz, 1H), 7.04 (d, *J* = 1.6 Hz, 1H), 2.36 (t, *J* = 7.4 Hz, 2H), 1.58 - 1.46 (m, 2H), 1.27 (dq, *J* = 14.5, 7.3 Hz, 2H), 0.85 (t, *J* = 7.3 Hz, 3H). MS (ESI): m/z 507.16 $[M + H]^+$. Mp 231-232°C.

Example 44

Synthesis of 8-[3-(6-benzyloxyamido)pyridyl]-1-[3-(trifluoromethyl)phenyl]oxazolo[5,4-*c*] quinolin-2(1*H*)-one (HTL-7-23)

The synthesis was carried out according to the synthetic method of HTL-4-32 to obtain a yellow solid, with a total yield of 36%. $^1$H-NMR (400 MHz, DMSO-$D_6$) δ 10.49 (s, 1H), 9.09 (s, 1H), 8.35 (s, 1H), 8.26 - 8.05 (m, 4H), 8.07 - 7.94 (m, 2H), 7.85(d, *J* = 8.7 Hz, 1H), 7.75 (dd, *J* = 8.8, 2.4 Hz, 1H), 7.47 - 7.20 (m, 5H), 7.05 (d, *J* = 1.7 Hz, 1H), 5.16 (s, 2H). MS (ESI): m/z 557.14 $[M + H]^+$. Mp 284-286°C.

Example 45

Synthesis of (*E*)-8-(2-carbamoyl-vinyl)-1-[3-(trifluoromethyl)phenyl]oxazolo[5,4-c] quinolin-2(1*H*)-one (HTL-6-30)

8-Bromo-1-[3-(trifluoromethyl)phenyl]oxazolo[5,4-*c*]quinolin-2(1*H*)-one (4.08 g, 10 mmol) was dissolved in DMF solution, and $Pd(OAc)_2$ (0.45 g, 2 mmol), acrylamide (1.42 g, 20 mmol), tri(o-tolyl)phosphine (1.22 g, 4 mmol) and $Et_3N$ (10.12 g, 100 mmol) were added. Under the protection of $N_2$, the mixed solution was heated to 100°C and reacted for 2 hours. After the completion of the reaction monitored by TLC, the reaction solution was diluted with water and extracted with ethyl acetate. The organic layer was washed with saturated NaCl and dried with anhydrous $MgSO_4$, and then filtrated, the filtrate was dried by a rotary evaporator, and purified by column chromatography (n-hexane/ethyl acetate 5:1) to obtain 3.16 g of a yellow solid, with a yield of 79%. $^1$H-NMR (400 MHz, DMSO-$D_6$) δ 9.12 (s, 1H), 8.29 (s, 1H), 8.14 (dd, *J* = 16.6, 8.3 Hz, 3H), 8.01 (t, *J* = 7.9 Hz, 1H), 7.90 (dd, *J* = 9.0, 1.8 Hz, 1H), 7.58 (s, 1H), 7.23 (s, 1H), 7.06 (d, *J* = 15.8 Hz, 1H), 6.94 (d, *J* = 1.6 Hz, 1H), 6.55 (d, *J* = 15.8 Hz, 1H). MS (ESI): m/z 400.08 $[M + H]^+$. Mp 291-292°C.

Example 46

Synthesis of (*E*)-8-(3-cyano-propenyl)-1-[3-(trifluoromethyl)phenyl]oxazolo[5,4-*c*] quinolin-2(1*H*)-one (HTL-6-31)

The synthesis was carried out according to the synthetic method of HTL-6-30 to obtain a yellow solid, with a yield of 77%. $^1$H-NMR (400 MHz, DMSO-$D_6$) δ 9.09 (d, *J* = 4.3 Hz, 1H), 8.30 (s, 1H), 8.19 - 8.10 (m, 2H), 8.06 (d, *J* = 8.9 Hz, 1H), 7.99 (t, *J* = 7.9 Hz, 1H), 7.92 (dd, *J* = 9.0, 1.9 Hz, 1H), 6.80 (s, 1H), 6.42 (d, *J* = 15.9 Hz, 1H), 6.18 (dt, *J* = 15.8, 5.9 Hz, 1H), 3.54 (dd, *J* = 5.8, 1.4 Hz, 2H). MS (ESI): m/z 396.09 $[M + H]^+$. Mp 177-179°C.

Example 47

Synthesis of (*E*)-8-(2-cyano-vinyl)-1-[3-(trifluoromethyl)phenyl]oxazolo[5,4-c]quinolin-2(1*H*)-one (HTL-6-32)

The synthesis was carried out according to the synthetic method of HTL-6-30 to obtain a yellow solid, with a yield of 77%. $^1$H-NMR (400 MHz, DMSO-$D_6$) δ 9.15 (d, *J* = 3.0 Hz, 1H), 8.24 (s, 1H), 8.12 (t, *J* = 8.1 Hz, 3H), 8.04 (dd, *J* = 9.1, 1.7 Hz, 1H), 8.00 - 7.94 (m, 1H), 7.51 (d, *J* = 16.6 Hz, 1H), 7.09 (s, 1H), 6.42 (d, *J* = 16.6 Hz, 1H). MS (ESI) : m/z 382.07 $[M + H]^+$. Mp 235-237°C.

Example 48

Synthesis of (*E*)-8-(2-methoxycarbonyl-vinyl)-1-[3-(trifluoromethyl)phenyl]oxazolo[5,4-*c*] quinolin-2(1*H*)-one (HTL-6-33)

The synthesis was carried out according to the synthetic method of HTL-6-30 to obtain a yellow solid, with a yield of 72%. $^1$H-NMR (400 MHz, DMSO-$D_6$) δ 9.17 (d, *J* = 11.8 Hz, 1H), 8.31 (s, 1H), 8.15 (d, *J* = 8.0 Hz, 2H), 8.11 (s, 2H), 8.01 (t, *J* = 7.9 Hz, 1H), 7.37 (d, *J* = 16.0 Hz, 1H), 7.08 (s, 1H), 6.50 (d, *J* = 16.0 Hz, 1H), 3.71 (s, 3H). MS (ESI): m/z 415.08 $[M + H]^+$. Mp 243-245°C.

Example 49

Synthesis of (*E*)-8-(3-ureido-propenyl)-1-[3-(trifluoromethyl)phenyl]oxazolo[5,4-*c*] quinolin-2(1*H*)-one (HTL-6-34)

The synthesis was carried out according to the synthetic method of HTL-6-30 to obtain a yellow solid, with a yield of 75%. $^1$H-NMR (400 MHz, DMSO-$D_6$) δ 9.05 (s, 1H), 8.29 (s, 1H), 8.17 - 8.09 (m, 2H), 8.04 - 7.95 (m, 2H), 7.85 (d, *J* = 9.1 Hz, 1H), 6.77 (s, 1H), 6.24 - 6.10 (m, 3H), 5.50 (s, 2H), 3.70 (t, *J* = 5.4 Hz, 2H). MS (ESI): m/z 429.11 $[M + H]^+$. Mp 157-160°C.

Example 50

Synthesis of (*E*)-8-(2-tert-butoxycarbonyl-vinyl)-1-[3-(trifluoromethyl)phenyl]oxazolo[5,4-c]quinolin-2(1*H*)-one (HTL-6-35)

The synthesis was carried out according to the synthetic method of HTL-6-30 to obtain a yellow solid, with a yield of 71%. $^1$H-NMR (400 MHz, DMSO-$D_6$) δ 9.14 (s, 1H), 8.32 (s, 1H), 8.18 - 8.11 (m, 2H), 8.08 (d, *J* = 1.0 Hz, 2H), 8.01 (t, *J* = 7.9 Hz, 1H), 7.24 (d, *J* = 15.9 Hz, 1H), 7.01 (s, 1H), 6.37 (d, *J* = 15.9 Hz, 1H), 1.45 (s, 9H). MS (ESI) : m/z

457.13 [M + H]$^+$. Mp 186-187°C.

Example 51

Synthesis of (*E*)-8-(4-ethoxycarbonyl-but-1-enyl)-1-[3-(trifluoromethyl)phenyl]oxazolo [5,4-*c*]quinolin-2(1*H*)-one (HTL-6-38)

The synthesis was carried out according to the synthetic method of HTL-6-30 to obtain a yellow solid, with a yield of 73%. $^1$H-NMR (400 MHz, DMSO-$D_6$) δ 9.06 (s, 1H), 8.29 (s, 1H), 8.13 (t, *J* = 8.2 Hz, 2H), 8.04 - 7.95 (m, 2H), 7.80 (dd, *J* = 9.0, 1.8 Hz, 1H), 6.75 (s, 1H), 6.21 (d, *J* = 15.9 Hz, 1H), 6.11 (d, *J* = 15.8 Hz, 1H), 4.03 (dd, *J* = 14.2, 7.1 Hz, 2H), 2.38 (s, 4H), 1.13 (t, *J* = 7.1 Hz, 3H). MS (ESI): m/z 457.13 [M + H]$^+$. Mp 162-164°C.

Example 52

Synthesis of (*E*)-8-(2-ethoxycarbonyl-vinyl)-1-[3-(trifluoromethyl)phenyl]oxazolo[5,4-*c*]quinolin-2(1*H*)-one (WSX-1-13)

The synthesis was carried out according to the synthetic method of HTL-6-30 to obtain a yellow solid, with a yield of 77%. $^1$H-NMR (400 MHz, DMSO-$D_6$) δ 9.15 (s, 1H), 8.30 (s, 1H), 8.18 - 8.06 (m, 4H), 8.00 (t, *J* = 7.8 Hz, 1H), 7.34 (d, *J* = 16.0 Hz, 1H), 7.07 (s, 1H), 6.49 (d, *J* = 16.0 Hz, 1H), 4.16 (dd, *J* = 13.7, 6.6 Hz, 2H), 1.23 (t, *J* = 7.1 Hz, 6H). MS (ESI): m/z 429.10 [M + H]$^+$. Mp 221-224°C.

Example 53

Synthesis of 9-[3-(6-propionylamino)pyridyl]-2-oxo-1-[3-(trifluoromethyl)phenyl]-1,2-dihydrobenzo[*h*][1,6]naphthyridine (HTL-6-45)

80mL microwave tube was taken, to which 9-bromo-2-oxo-1-[3-(trifluoromethyl)phenyl]-1,2-dihydrobenzo[*h*][1,6]naphthyridine (4.18 g, 10 mmol), $Pd(Ph_3P)_4$ (1.16 g, 1 mmol), $K_2CO_3$ (2.76 g, 20 mmol) and 6-aminopyridineboronic acid (1.66 g, 12 mmol) were added and dissolved in a solution of 1,4-dioxane. The microwave tube was sealed with cover, and placed in a microwave reactor, the temperature was set to 100°C, the reaction was carried out for 40 minutes, the microwave tube was taken out and cooled to room temperature. The reaction solution was transferred to a separatory funnel, diluted with water, and extracted with ethyl acetate. The organic layer was washed with saturated NaCl, dried with anhydrous $Na_2SO_4$, and filtered, the filtrate was dried by a rotary evaporator and purified with column chromatography (n-hexane/ethyl acetate 5:1) to obtain 3.59 g of a yellow solid, with a yield of 84%. $^1$H-NMR (400 MHz, DMSO-$D_6$) δ 10.63 (s, 1H), 9.17 (s, 1H), 8.34 (d, *J* = 9.5 Hz, 1H), 8.06 (ddd, *J* = 16.3, 11.5, 5.5 Hz, 6H), 7.95 - 7.82 (m, 2H), 7.48 (dd, *J* = 8.7, 2.5 Hz, 1H), 6.98 (dd, *J* = 22.2, 5.5 Hz, 2H), 2.42 (q, *J* = 7.5 Hz, 2H), 1.07 (t, *J* = 7.5 Hz, 3H). MS (ESI): m/z 489.15 [M + H]$^+$. Mp 249-250°C.

Example 54

Synthesis of 9-(6-quinolyl)-2-oxo-1-[3-(trifluoromethyl)phenyl]-1,2-dihydrobenzo[*h*][1,6]naphthyridine (HTL-6-47)

The synthesis was carried out according to the synthetic method of HTL-6-45 to obtain a yellow solid, with a yield of 86%. $^1$H-NMR (400 MHz, DMSO-$D_6$) δ 9.19 (s, 1H), 8.92 (dd, *J* = 4.1, 1.5 Hz, 1H), 8.34 (dd, *J* = 12.5, 9.1 Hz, 2H), 8.17 (s, 4H), 7.96 (dd, *J* = 20.0, 8.3 Hz, 2H), 7.84 (d, *J* = 7.9 Hz, 1H), 7.70 (d, *J* = 1.6 Hz, 1H), 7.60 (dd, *J* = 8.3, 4.2 Hz, 1H), 7.43 (dd, *J* = 8.8, 2.0 Hz, 1H), 7.20 (s, 1H), 6.97 (d, *J* = 9.4 Hz, 1H). MS (ESI): m/z 468.12 (M + H]$^+$. Mp 196-197°C.

Example 55

Synthesis of 9-[3-(2-fluoro)pyridyl]-2-oxo-1-[3-(trifluoromethyl)phenyl]-1,2-dihydrobenzo [*h*][1,6]naphthyridine (HTL-6-48)

The synthesis was carried out according to the synthetic method of HTL-6-45 to obtain a yellow solid, with a yield of 82%. $^1$H-NMR (400 MHz, DMSO-$D_6$) δ 9.21 (s, 1H), 8.35 (d, *J*= 9.5 Hz, 1H), 8.28 - 8.20 (m, 1H), 8.15 (d, *J*= 8.7 Hz, 1H), 8.07 (s, 1H), 7.97 - 7.87 (m, 2H), 7.87 - 7.76 (m, 2H), 7.52 - 7.44 (m, 1H), 7.42 - 7.34 (m, 1H), 6.96 (dd, *J* = 9.5, 3.8 Hz, 1H), 6.87 (d, *J* = 1.7 Hz, 1H). MS (ESI): m/z 436.10 [M + H]$^+$. Mp 158-161°C.

Example 56

Synthesis of 9-[3-(2-methyl)pyridyl]-2-oxo-1-[3-(trifluoromethyl)phenyl]-1,2-dihydrobenzo[*h*][1,6]naphthyridine (HTL-6-49)

The synthesis was carried out according to the synthetic method of HTL-6-45 to obtain a yellow solid, with a yield of 83%. $^1$H-NMR (400 MHz, DMSO-$D_6$) δ 9.16 (s, 1H), 8.32 (d, *J*= 9.5 Hz, 1H), 8.25 (d, *J*= 2.0 Hz, 1H), 8.11 (t, *J*= 6.6 Hz, 2H), 8.03 (dd, *J* = 8.7, 1.8 Hz, 2H), 7.90 - 7.78 (m, 2H), 7.42 - 7.29 (m, 2H), 6.95 (dd, *J* = 16.0, 5.6 Hz, 2H), 2.50 (s, 3H). MS (ESI): m/z 432.12 $[M + H]^+$. Mp 2046-207°C.

Example 57

Synthesis of 9-[(1*H*)-3-pyrazolyl]-2-oxo-1-[3-(trifluoromethyl)phenyl]-1,2-dihydrobenzo[*h*][1,6]naphthyridine (HTL-6-50)

The synthesis was carried out according to the synthetic method of HTL-6-45 to obtain a yellow solid, with a yield of 85%. $^1$H-NMR (400 MHz, DMSO-$D_6$) δ 12.93 (s, 1H), 9.08 (s, 1H), 8.29 (d, *J*= 9.5 Hz, 1H), 8.16 (d, *J*= 7.5 Hz, 1H), 8.08 (s, 1H), 8.02 (d, *J*= 8.3 Hz, 2H), 7.88 (t, *J*= 7.9 Hz, 1H), 7.78 (d, *J*= 8.0 Hz, 1H), 7.69 (s, 1H), 7.08 (s, 1H), 6.90 (d, *J*= 9.4 Hz, 1H), 5.72 (s, 1H). MS (ESI): m/z 407.10 $[M + H]^+$. Mp 283-286°C.

Example 58

Synthesis of 9-[3-(6-fluoro)pyridyl]-2-oxo-1-[3-(trifluoromethyl)phenyl]-1,2-dihydrobenzo[*h*][1,6]naphthyridine (HTL-7-01)

The synthesis was carried out according to the synthetic method of HTL-6-45 to obtain a yellow solid, with a yield of 80%. $^1$H-NMR (400 MHz, DMSO-$D_6$) δ 9.15 (s, 1H), 8.31 (d, *J* = 9.5 Hz, 1H), 8.10 (d, *J* = 8.7 Hz, 2H), 8.05 - 7.96 (m, 2H), 7.92 - 7.82 (m, 2H), 7.80 (d, *J* = 8.0 Hz, 1H), 7.65 (td, *J* = 8.2, 2.7 Hz, 1H), 7.19 (dd, *J*= 8.5, 2.8 Hz, 1H), 6.91 (dd, *J* = 14.4, 5.6 Hz, 2H). MS (ESI): m/z 436.10 $[M + H]^+$. Mp 264-266°C.

Example 59

Synthesis of 9-[3-(2-methoxy)pyridyl]-2-oxo-1-[3-(trifluoromethyl)phenyl]-1,2-dihydrobenzo[*h*][1,6]naphthyridine (HTL-7-02)

The synthesis was carried out according to the synthetic method of HTL-6-45 to obtain a yellow solid, with a yield of 81%. $^1$H-NMR (400 MHz, DMSO-$D_6$) δ 9.14 (s, 1H), 8.31 (d, *J*= 9.5 Hz, 1H), 8.13 (dd, *J* = 4.8, 2.1 Hz, 1H), 8.07-8.01 (m, 2H), 7.91 - 7.84 (m, 2H), 7.83 - 7.71 (m, 2H), 7.01 - 6.88 (m, 3H), 6.75 (d, *J*= 1.7 Hz, 1H), 3.76 (s, 3H). MS (ESI): m/z 448.12 $[M + H]^+$. Mp 272-275°C.

Example 60

Synthesis of 9-[3-(6-methoxy)pyridyl]-2-oxo-1-[3-(trifluoromethyl)phenyl]-1,2-dihydrobenzo[*h*][1,6]naphthyridine (HTL-7-03)

The synthesis was carried out according to the synthetic method of HTL-6-45 to obtain a yellow solid, with a yield of 86%. $^1$H-NMR (400 MHz, DMSO-$D_6$) δ 9.12 (s, 1H), 8.30 (d, *J* = 9.5 Hz, 1H), 8.12 - 8.04 (m, 2H), 8.03 - 7.92 (m, 3H), 7.85 (t, *J*= 7.9 Hz, 1H), 7.76 (d, *J*= 8.0 Hz, 1H), 7.24 (dd, *J* = 8.7, 2.6 Hz, 1H), 6.90 (dd, *J*= 10.5, 5.6 Hz, 2H), 6.76 (dd, *J*= 8.6, 0.4 Hz, 1H), 3.84 (s, 3H). MS (ESI): m/z 448.12 $[M+H]^+$. Mp 211-216°C.

Example 61

Synthesis of 9-[5-(2-oxo)indolyl]-2-oxo-1-[3-(trifluoromethyl)phenyl]-1,2-dihydrobenzo[*h*][1,6]naphthyridine (HTL-7-04)

The synthesis was carried out according to the synthetic method of HTL-6-45 to obtain a yellow solid, with a yield of 79%. $^1$H-NMR (400 MHz, DMSO-$D_6$) δ 10.49 (s, 1H), 9.11 (s, 1H), 8.29 (d, *J*= 9.5 Hz, 1H), 8.09 (dd, *J*= 25.1, 8.2 Hz, 3H), 8.00 - 7.82 (m, 2H), 7.79 (d, *J*= 7.9 Hz, 1H), 7.11 (d, *J* = 7.7 Hz, 1H), 6.94 (dd, *J* = 22.7, 5.5 Hz, 2H), 6.60 - 6.43 (m, 2H), 3.47 (s, 2H). MS (ESI): m/z 472.12 $[M + H]^+$. Mp 281-282°C.

Example 62

Synthesis of 9-[3-(6-butyrylamino)pyridyl]-2-oxo-1-[3-(trifluoromethyl)phenyl]-1,2-dihydrobenzo[*h*][1,6]naphthyridine (HTL-7-05)

The synthesis was carried out according to the synthetic method of HTL-6-45 to obtain a yellow solid, with a yield of 83%. $^1$H-NMR (400 MHz, DMSO-$D_6$) δ 10.60 (s, 1H), 9.12 (s, 1H), 8.30 (d, *J*= 9.5 Hz, 1H), 8.08 (d, *J*= 8.8 Hz, 3H), 8.03 - 7.92 (m, 3H), 7.91 - 7.78 (m, 2H), 7.44 (dd, *J* = 8.7, 2.5 Hz, 1H), 6.94 (dd, *J*= 24.4, 5.5 Hz, 2H), 2.35 (t, *J*= 7.3 Hz, 2H), 1.65 - 1.50 (m, 2H), 0.87 (t, *J*= 7.4 Hz, 3H). MS (ESI): m/z 503.16 $[M + H]^+$. Mp 279-280°C.

Example 63

Synthesis of 9-[5-(2-methoxy)pyrimidyl]-2-oxo-1-[3-(trifluoromethyl)phenyl]-1,2-dihydrobenzo[*h*][1,6]naphthyridine (HTL-7-06)

The synthesis was carried out according to the synthetic method of HTL-6-45 to obtain a yellow solid, with a yield of 77%. $^1$H-NMR (400 MHz, DMSO-$D_6$) δ 9.15 (s, 1H), 8.36 - 8.25 (m, 3H), 8.10 (d, *J* = 8.8 Hz, 2H), 8.05 - 7.95 (m, 2H), 7.87 (t, *J* = 7.9 Hz, 1H), 7.79 (d, *J* = 8.0 Hz, 1H), 6.93 (d, *J*= 9.4 Hz, 1H), 6.87 (d, *J*= 1.6 Hz, 1H), 3.91 (s, 3H). MS (ESI): m/z 449.11 $[M + H]^+$. Mp 262-264°C.

Example 64

Synthesis of 9-[3-(2-isobutyrylamino)pyridyl]-2-oxo-1-[3-(trifluoromethyl)phenyl]-1,2-dihydrobenzo[*h*][1,6]naphthyridine (HTL-7-07)

The synthesis was carried out according to the synthetic method of HTL-6-45 to obtain a yellow solid, with a yield of 76%. $^1$H-NMR (400 MHz, DMSO-$D_6$) δ 10.60 (s, 1H), 9.13 (s, 1H), 8.30 (d, *J*= 9.4 Hz, 1H), 8.13 - 8.04 (m, 3H), 8.04 - 7.96 (m, 3H), 7.91 - 7.79 (m, 2H), 7.44 (dd, *J*= 8.7, 2.5 Hz, 1H), 6.99 (d, *J* = 1.5 Hz, 1H), 6.96 - 6.88 (m, 1H), 2.75 (dt, *J* = 13.6, 6.8 Hz, 1H), 1.06 (d, *J*= 6.8 Hz, 6H). MS (ESI): m/z 503.16 $[M + H]^+$. Mp 213-215°C.

Example 65

Synthesis of 9-[3-(6-valerylamino)pyridyl]-2-oxo-1-[3-(trifluoromethyl)phenyl]- 1,2-dihydrobenzo[*h*][1,6]naphthyridine (HTL-7-08)

The synthesis was carried out according to the synthetic method of HTL-6-45 to obtain a yellow solid, with a yield of 78%. $^1$H-NMR (400 MHz, DMSO-$D_6$) δ 10.60 (s, 1H), 9.13 (s, 1H), 8.30 (d, *J*= 9.5 Hz, 1H), 8.08 (dd, *J*= 8.5, 6.3 Hz, 3H), 8.03 - 7.93 (m, 3H), 7.92 - 7.79 (m, 2H), 7.45 (dd, *J* = 8.7, 2.5 Hz, 1H), 6.99 (d, *J*= 1.6 Hz, 1H), 6.92 (d, *J*= 9.4 Hz, 1H), 2.38 (t, *J*= 7.4 Hz, 2H), 1.59 - 1.47 (m, 2H), 1.28 (dq, *J* = 14.6, 7.3 Hz, 2H), 0.93 - 0.81 (m, 3H). MS (ESI): m/z 517.18 $[M + H]^+$. Mp 274-277°C.

Example 66

Synthesis of 9-[3-(6-phenylacetylamino)pyridyl]-2-oxo-1-[3-(trifluoromethyl)phenyl]-1,2-dihydrobenzo[*h*][1,6]naphthyridine (HTL-7-10)

The synthesis was carried out according to the synthetic method of HTL-6-45 to obtain a yellow solid, with a yield of 72%. $^1$H-NMR (400 MHz, DMSO-$D_6$) δ 10.90 (s, 1H), 9.13 (s, 1H), 8.30 (d, *J* = 9.5 Hz, 1H), 8.14 - 7.95 (m, 6H), 7.90 - 7.78 (m, 2H), 7.45 (dd, *J* = 8.7, 2.5 Hz, 1H), 7.38 - 7.25 (m, 4H), 7.26 - 7.17 (m, 1H), 6.98 (d, *J*= 1.7 Hz, 1H), 6.92 (d, *J*= 9.4 Hz, 1H), 3.72 (s, 2H). MS (ESI): m/z 551.16 $[M+H]^+$. Mp 143-145°C.

Example 67

Synthesis of 9-{3-[6-(4-methoxy)phenylacetylamino]pyridyl}-2-oxo-1-[3-(trifluoromethyl)phenyl]-1,2-dihydrobenzo[*h*][1,6]naphthyridine (HTL-7-11)

The synthesis was carried out according to the synthetic method of HTL-6-45 to obtain a yellow solid, with a yield of 76%. $^1$H-NMR (400 MHz, DMSO-$D_6$) δ 10.83 (s, 1H), 9.13 (s, 1H), 8.30 (d, *J*= 9.5 Hz, 1H), 8.14 - 7.94 (m, 6H),

7.90 - 7.77 (m, 2H), 7.45 (dd, *J*= 8.7, 2.5 Hz, 1H), 7.24 (d, *J*= 8.7 Hz, 2H), 6.98 (d, *J*= 1.7 Hz, 1H), 6.92 (d, J= 9.4 Hz, 1H), 6.90 - 6.83 (m, 2H), 3.71 (d, *J*= 10.3 Hz, 3H), 3.63 (s, 2H). MS (ESI): m/z 581.17 $[M + H]^+$. Mp 241-243°C.

Example 68

Synthesis of 9-[4-(3,5-dimethyl)isoxazolyl]-2-oxo-1-[3-(trifluoromethyl)phenyl]-1,2-dihydrobenzo[*h*][1,6]naphthyridine (HTL-7-12)

The synthesis was carried out according to the synthetic method of HTL-6-45 to obtain a yellow solid, with a yield of 70%. $^1$H-NMR (400 MHz, DMSO-$D_6$) δ 9.16 (s, 1H), 8.31 (d, *J* = 9.5 Hz, 1H), 8.10 (d, *J* = 8.6 Hz, 1H), 7.96 (s, 1H), 7.87 (ddd, *J* = 8.1, 4.4, 1.1 Hz, 1H), 7.84 - 7.77 (m, 2H), 7.66 (dd, *J* = 8.6, 1.8 Hz, 1H), 6.91 (d, *J* = 9.4 Hz, 1H), 6.61 (d, *J* = 1.6 Hz, 1H), 2.03 (s, 3H), 1.89 (s, 3H). MS (ESI): m/z 436.12 $[M + H]^+$. Mp 121-122°C.

Example 69

Synthesis of 9-(1,4-dioxa-spiro[4.5]dec-7-en-8-yl)-2-oxo-1-[3-(trifluoromethyl)phenyl]-1,2-dihydrobenzo[*h*][1,6]naphthyridine (HTL-7-13)

The synthesis was carried out according to the synthetic method of HTL-6-45 to obtain a yellow solid, with a yield of 71%. $^1$H-NMR (400 MHz, DMSO-$D_6$) δ 9.05 (s, 1H), 8.27 (d, *J* = 9.5 Hz, 1H), 8.08 (s, 1H), 7.97 (d, *J*= 7.9 Hz, 1H), 7.91 (d, *J*= 8.8 Hz, 1H), 7.86 - 7.75 (m, 2H), 7.68 (d, *J* = 8.1 Hz, 1H), 6.85 (dd, *J* = 17.0, 5.6 Hz, 2H), 5.74 (t, *J* = 3.9 Hz, 1H), 3.95 - 3.78 (m, 4H), 2.24 (s, 2H), 1.94 - 1.77 (m, 2H), 1.62 (t, *J*= 6.4 Hz, 2H). MS (ESI): m/z 479.15 $[M + H]^+$. Mp 210-211°C.

Example 70

Synthesis of 9-[3-(6-benzyloxyamido)pyridyl]-2-oxo-1-[3-(trifluoromethyl)phenyl]-1,2-dihydrobenzo[*h*][1,6]naphthyridine (HTL-7-14)

The synthesis was carried out according to the synthetic method of HTL-6-45 to obtain a yellow solid, with a yield of 68%. $^1$H-NMR (400 MHz, DMSO-$D_6$) δ 10.45 (s, 1H), 9.16 (d, *J* = 14.3 Hz, 1H), 8.31 (d, *J* = 9.5 Hz, 1H), 8.20 - 7.91 (m, 5H), 7.93 - 7.71 (m, 3H), 7.53 - 7.14 (m, 6H), 7.05 - 6.85 (m, 2H), 5.17 (s, 2H). MS (ESI): m/z 567.16 $[M + H]^+$. Mp 150-153°C.

Example 71

Synthesis of 9-[3-(6-phenoxyamido)pyridyl]-2-oxo-1-[3-(trifluoromethyl)phenyl]-1,2-dihydrobenzo[*h*][1,6]naphthyridine (HTL-7-15)

The synthesis was carried out according to the synthetic method of HTL-6-45 to obtain a yellow solid, with a yield of 73%. $^1$H-NMR (400 MHz, DMSO-$D_6$) δ 10.94 (s, 1H), 9.15 (d, *J* = 10.9 Hz, 1H), 8.31 (dt, *J* = 11.1, 5.6 Hz, 1H), 8.18 - 8.06 (m, 3H), 8.04 - 7.98 (m, 1H), 7.85 (ddd, *J* = 14.7, 13.6, 7.7 Hz, 3H), 7.43 (ddd, *J* = 11.5, 9.8, 5.8 Hz, 3H), 7.34 - 7.16 (m, 4H), 6.99 (d, *J* = 1.9 Hz, 1H), 6.92 (dd, *J* = 9.4, 1.3 Hz, 1H). MS (ESI): m/z 553.14 $[M + H]^+$. Mp 150-152 °C.

Example 72

Synthesis of 9-[3-(6-N,N-dimethyl)pyridyl]-2-oxo-1-[3-(trifluoromethyl)phenyl]-1,2-dihydrobenzo[*h*][1,6]naphthyridine (HTL-7-16)

The synthesis was carried out according to the synthetic method of HTL-6-45 to obtain a yellow solid, with a yield of 78%. $^1$H-NMR (400 MHz, DMSO-$D_6$) δ 9.06 (s, 1H), 8.28 (d, *J* = 9.5 Hz, 1H), 8.09 (s, 1H), 8.02 (dd, *J* = 8.1, 3.5 Hz, 2H), 7.96 (d, *J* = 2.4 Hz, 1H), 7.92 (dd, *J*= 8.7, 1.8 Hz, 1H), 7.86 (t, *J*= 7.9 Hz, 1H), 7.76 (d, *J*= 8.0 Hz, 1H), 7.02 (dd, *J*= 8.9, 2.6 Hz, 1H), 6.89 (t, *J*= 5.6 Hz, 2H), 6.53 (d, *J*= 8.9 Hz, 1H), 3.01 (s, 6H). MS (ESI) : m/z 461.15 $[M + H]^+$. Mp 220-222°C.

Example 73

Synthesis of 9-{3-[6-(4-methylpiperazin-1-yl)pyridyl]}-2-oxo-1-[3-(trifluoromethyl) phenyl]-1,2-dihydrobenzo[*h*][1,6]naphthyridine (HTL-7-17)

The synthesis was carried out according to the synthetic method of HTL-6-45 to obtain a yellow solid, with a yield of 75%. $^1$H-NMR (400 MHz, DMSO-$D_6$) $\delta$ 9.08 (s, 1H), 8.29 (d, *J* = 9.5 Hz, 1H), 8.08 (s, 1H), 8.05 - 7.99 (m, 2H), 7.98 - 7.90 (m, 2H), 7.86 (t, *J*= 7.9 Hz, 1H), 7.78 (d, *J*= 8.0 Hz, 1H), 7.07 (dd, *J*= 8.9, 2.6 Hz, 1H), 6.90 (dd, *J* = 5.6, 3.8 Hz, 2H), 6.74 (d, *J*= 8.9 Hz, 1H), 3.50 (dd, *J*= 16.8, 12.0 Hz, 4H), 2.41 - 2.28 (m, 4H), 2.19 (s, 3H). MS (ESI): m/z 516.19 $[M + H]^+$. Mp 161-162°C.

Example 74

Synthesis of 9-[3-(6-morpholin-4-yl)pyridyl]-2-oxo-1-[3-(trifluoromethyl)phenyl]-1,2-dihydrobenzo[*h*][1,6]naphthyridine (HTL-7-18)

The synthesis was carried out according to the synthetic method of HTL-6-45 to obtain a yellow solid, with a yield of 72%. $^1$H-NMR (400 MHz, DMSO-$D_6$) $\delta$ 9.08 (s, 1H), 8.28 (d, *J* = 9.5 Hz, 1H), 8.08 (s, 1H), 8.02 (dd, *J* = 8.2, 5.0 Hz, 2H), 7.99 - 7.90 (m, 2H), 7.85 (t, *J* = 7.9 Hz, 1H), 7.76 (d, *J* = 8.0 Hz, 1H), 7.10 (dd, *J* = 8.9, 2.6 Hz, 1H), 6.90 (dd, *J* = 5.6, 3.9 Hz, 2H), 6.75 (d, *J* = 8.9 Hz, 1H), 3.77 - 3.60 (m, 4H), 3.52 - 3.38 (m, 4H). MS (ESI): m/z 503.16 $[M + H]^+$. Mp 286-287°C.

Example 75

Synthesis of 9-[3-(6-amino-5-methoxy)pyridyl]-2-oxo-1-[3-(trifluoromethyl)phenyl]-1,2-dihydrobenzo[*h*][1,6]naphthyridine (HTL-7-19)

The synthesis was carried out according to the synthetic method of HTL-6-45 to obtain a yellow solid, with a yield of 81%. $^1$H-NMR (400 MHz, DMSO-$D_6$) $\delta$ 9.07 (s, 1H), 8.28 (d, *J*= 9.5 Hz, 1H), 8.06 - 7.81 (m, 6H), 7.05 (d, *J*= 2.0 Hz, 1H), 6.97 (d, *J*= 1.8 Hz, 1H), 6.88 (dd, *J* = 8.5, 5.7 Hz, 2H), 6.01 (s, 2H), 3.78 (s, 3H). MS (ESI): m/z 463.13 $[M + H]^+$. Mp 233-234°C.

Example 76

Synthesis of 9-[3-(6-pyrrolidinyl)pyridyl]-2-oxo-1-[3-(trifluoromethyl)phenyl]-1,2-dihydrobenzo[*h*][1,6]naphthyridine (HTL-7-20)

The synthesis was carried out according to the synthetic method of HTL-6-45 to obtain a yellow solid, with a yield of 77%. $^1$H-NMR (400 MHz, DMSO-$D_6$) $\delta$ 9.05 (s, 1H), 8.27 (d, *J* = 9.5 Hz, 1H), 8.09 (s, 1H), 8.05 - 7.94 (m, 3H), 7.93 - 7.81 (m, 2H), 7.75 (d, *J*= 8.0 Hz, 1H), 6.98 (dd, *J* = 8.8, 2.6 Hz, 1H), 6.87 (dd, *J* = 10.8, 5.6 Hz, 2H), 6.32 (d, *J* = 8.8 Hz, 1H), 3.35 (t, *J* = 6.5 Hz, 4H), 1.91 (t, *J* = 6.6 Hz, 4H). MS (ESI): m/z 487.17 $[M + H]^+$. Mp 252- 254°C.

Example 77

Synthesis of 9-[3-(6-tert-butoxycarbonylamino)pyridyl]-2-oxo-1-[3-(trifluoromethyl) phenyl]-1,2-dihydrobenzo[*h*][1,6]naphthyridine (HTL-7-21)

The synthesis was carried out according to the synthetic method of HTL-6-45 to obtain a yellow solid, with a yield of 73%. $^1$H-NMR (400 MHz, DMSO-$D_6$) $\delta$ 9.97 (s, 1H), 9.12 (s, 1H), 8.30 (d, *J* = 9.5 Hz, 1H), 8.08 (dd, *J* = 11.2, 2.3 Hz, 3H), 8.00 (dt, *J* = 8.8, 4.4 Hz, 2H), 7.87 (t, *J* = 7.8 Hz, 1H), 7.78 (dd, *J*= 17.8, 8.4 Hz, 2H), 7.28 (dd, *J*= 8.8, 2.5 Hz, 1H), 6.93 (dd, *J*= 14.7, 5.6 Hz, 2H), 1.45 (s, 9H). MS (ESI): m/z 533.17 $[M + H]^+$. Mp 234-236°C.

Example 78

Synthesis of 9-[3-(6-allylamino)pyridyl]-2-oxo-1-[3-(trifluoromethyl)phenyl]-1,2-dihydrobenzo[*h*][1,6]naphthyridine (HTL-7-28)

The synthesis was carried out according to the synthetic method of HTL-6-45 to obtain a yellow solid, with a

yield of 70%. $^1$H-NMR (400 MHz, DMSO-$D_6$) δ 9.07 (s, 1H), 8.29 (d, *J* = 9.7 Hz, 1H), 8.19 - 7.45 (m, 7H), 7.13 - 6.77 (m, 3H), 6.44 (d, *J*= 8.2 Hz, 1H), 5.88 (s, 1H), 5.32 - 4.85 (m, 2H), 4.14 (d, *J* = 35.1 Hz, 1H), 3.89 (s, 2H). MS (ESI): m/z 473.15 $[M + H]^+$. Mp 142-146°C.

Example 79

Synthesis of 9-[3-(6-propargylamino)pyridyl]-2-oxo-1-[3-(trifluoromethyl)phenyl]-1,2-dihydrobenzo[*h*][1,6]naphthyridine (HTL-7-29)

The synthesis was carried out according to the synthetic method of HTL-6-45 to obtain a yellow solid, with a yield of 68%. $^1$H-NMR (400 MHz, DMSO-$D_6$) δ 9.14 (s, 1H), 8.31 (t, *J* = 7.5 Hz, 1H), 8.16 - 8.07 (m, 2H), 8.00 (dd, *J*= 14.2, 5.4 Hz, 1H), 7.94 - 7.86 (m, 1H), 7.80 (d, *J* = 9.3 Hz, 1H), 7.71 - 7.59 (m, 4H), 7.42 - 7.32 (m, 1H), 6.98 - 6.87 (m, 2H), 4.19 (t, *J*= 6.5 Hz, 2H), 4.10 (dd, *J* = 5.5, 3.6 Hz, 1H). MS (ESI): m/z 471.14 $[M + H]^+$. Mp 139-141°C.

Example 80

Synthesis of 9-(3-aminophenyl)imino-2-oxo-1-[3-(trifluoromethyl)phenyl]-1,2-dihydrobenzo[*h*]1,6]naphthyridine (HTL-6-11)

9-Bromo-2-oxo-1-[3-(trifluoromethyl)phenyl]-1,2-dihydrobenzo[*h*][1,6]naphthyridine (4.18 g, 10 mmol) was dissolved in a solution of 1,4-dioxane, then $Pd(dba)_3$ (0.23 g, 0.25 mmol), $CsCO_3$ (4.89 g, 15 mmol), 1,3-phenylenediamine (1.62 g, 15 mmol) and 4,5-bis(diphenylphosphine)-9,9-dimethylxanthene (0.14 g, 0.25 mmol) were added. Under the protection of $N_2$, the resulting mixture was heated to 100°C and reacted for 2 hours. When the reaction was completed, the reaction solution was diluted with water and extracted with ethyl acetate. The organic layer was washed with saturated NaCl solution, dried with anhydrous $MgSO_4$, and filtered, the filtrate was dried by a rotary evaporator to remove solvent, and purified by column chromatography (n-hexane/ethyl acetate 5:1) to obtain 3.66 g of a yellow solid, with a yield of 82%. $^1$H-NMR (400 MHz, DMSO-$D_6$) δ 8.86 (s, 1H), 8.24 (d, *J*= 9.5 Hz, 1H), 7.96 (s, 1H), 7.88 (d, *J* = 9.0 Hz, 1H), 7.80 (d, *J* = 8.1 Hz, 1H), 7.64 (dd, *J*= 14.4, 6.4 Hz, 2H), 7.55 (d, *J* = 7.9 Hz, 1H), 7.31 (dd, *J* = 9.0, 2.4 Hz, 1H), 6.91 - 6.82 (m, 2H), 6.44 (d, *J* = 2.3 Hz, 1H), 6.22 (dd, *J*= 7.9, 1.3 Hz, 1H), 6.08 (t, *J*= 2.0 Hz, 1H), 5.89 - 5.77 (m, 1H), 5.16 (s, 2H). MS (ESI): m/z 447.14 $[M + H]^+$. Mp 217-218°C.

Example 81

Synthesis of 9-[2-(6-aminopyridyl)]imino-2-oxo-1-[3-(trifluoromethyl)phenyl]-1,2-dihydrobenzo[*h*][1,6]naphthyridine (HTL-6-12)

The synthesis was carried out according to the synthetic method of HTL-6-11 to obtain a yellow solid, with a yield of 81%. $^1$H-NMR (400 MHz, DMSO-$D_6$) δ 8.90 (s, 1H), 8.49 (s, 1H), 8.24 (d, *J* = 9.5 Hz, 1H), 7.94 (d, *J* = 8.0 Hz, 1H), 7.87 (d, *J* = 9.0 Hz, 1H), 7.66 (dt, *J* = 13.4, 7.8 Hz, 3H), 7.51 (dd, *J* = 9.0, 2.3 Hz, 1H), 7.21 - 7.10 (m, 2H), 6.85 (d, *J* = 9.4 Hz, 1H), 5.91 (d, *J* = 7.9 Hz, 1H), 5.70 (t, *J*= 14.5 Hz, 3H). MS (ESI): m/z 448.13 $[M + H]^+$. Mp 259-261°C.

Example 82

Synthesis of 9-[4-(trifluoromethyl)phenyl]imino-2-oxo-1-[3-(trifluoromethyl)phenyl]-1,2-dihydrobenzo[*h*][1,6]naphthyridine (HTL-6-16)

The synthesis was carried out according to the synthetic method of HTL-6-11 to obtain a yellow solid, with a yield of 83%. $^1$H-NMR (400 MHz, DMSO-$D_6$) δ 8.98 (s, 1H), 8.71 (s, 1H), 8.26 (d, *J*= 9.5 Hz, 1H), 7.99 (d, *J*= 8.9 Hz, 1H), 7.86 (s, 1H), 7.69 - 7.59 (m, 2H), 7.52 (d, *J* = 7.4 Hz, 1H), 7.47 (d, *J* = 8.6 Hz, 2H), 7.39 (dd, *J* = 9.0, 2.4 Hz, 1H), 6.87 (d, *J* = 9.4 Hz, 1H), 6.78 (d, *J* = 8.4 Hz, 2H), 6.71 (d, *J* = 2.3 Hz, 1H). MS (ESI): m/z 450.11 $[M + H]^+$. Mp 151-153°C.

Example 83

Synthesis of 9-(3,4-dimethylphenyl)imino-2-oxo-1-[3-(trifluoromethyl)phenyl]-1,2-dihydrobenzo[*h*][1,6]naphthyridine (HTL-6-17)

The synthesis was carried out according to the synthetic method of HTL-6-11 to obtain a yellow solid, with a yield of 85%. $^1$H-NMR (400 MHz, DMSO-$D_6$) δ 8.86 (s, 1H), 8.21 (d, *J*= 9.5 Hz, 1H), 7.98 (s, 1H), 7.87 (d, *J*= 9.0 Hz,

1H), 7.64 (d, *J*= 7.7 Hz, 1H), 7.57 (s, 1H), 7.49 - 7.37 (m, 2H), 7.29 (dd, *J* = 9.0, 2.4 Hz, 1H), 6.97 (d, *J* = 8.0 Hz, 1H), 6.82 (d, *J* = 9.4 Hz, 1H), 6.58 (d, *J* = 2.0 Hz, 1H), 6.37 (dd, *J* = 8.0, 2.2 Hz, 1H), 6.27 (d, *J* = 2.3 Hz, 1H), 2.22 (d, *J* = 20.6 Hz, 6H). MS (ESI): m/z 460.16 $[M + H]^+$. Mp 140-141°C.

Example 84

Synthesis of 9-(4-tert-butylphenyl)imino-2-oxo-1-[3-(trifluoromethyl)phenyl]-1,2-dihydrobenzo[*h*][1,6]naphthyridine (HTL-6-18)

The synthesis was carried out according to the synthetic method of HTL-6-11 to obtain a yellow solid, with a yield of 80%. $^1$H-NMR (400 MHz, DMSO-$D_6$) $\delta$ 8.87 (s, 1H), 8.22 (d, *J* = 9.5 Hz, 1H), 8.14 (s, 1H), 7.88 (d, *J* = 9.0 Hz, 1H), 7.74 - 7.65 (m, 2H), 7.58 (t, *J* = 7.8 Hz, 1H), 7.49 (d, *J* = 7.9 Hz, 1H), 7.31 (dd, *J* = 9.0, 2.4 Hz, 1H), 7.24 - 7.17 (m, 2H), 6.83 (d, *J* = 9.4 Hz, 1H), 6.59 (d, *J*= 8.6 Hz, 2H), 6.49 (d, *J*= 2.3 Hz, 1H), 1.32 (d, *J*= 7.5 Hz, 9H). MS (ESI): m/z 488.19 $[M + H]^+$. Mp 141-142°C.

Example 85

Synthesis of 9-[3-(6-methylpyridyl)]imino-2-oxo-1-[3-(trifluoromethyl)phenyl]-1,2-dihydrobenzo[*h*][1,6]naphthyridine (HTL-6-19)

The synthesis was carried out according to the synthetic method of HTL-6-11 to obtain a yellow solid, with a yield of 79%. $^1$H-NMR (400 MHz, DMSO-$D_6$) $\delta$ 8.92 (s, 1H), 8.50 (s, 1H), 8.25 (d, *J*= 9.5 Hz, 1H), 7.99 - 7.91 (m, 2H), 7.75 - 7.65 (m, 2H), 7.59 (t, *J*= 7.8 Hz, 1H), 7.52 (d, *J* = 7.8 Hz, 1H), 7.35 (dd, *J* = 9.0, 2.4 Hz, 1H), 7.24 (d, *J* = 8.4 Hz, 1H), 7.13 (dd, *J* = 8.4, 2.5 Hz, 1H), 6.85 (d, *J*= 9.4 Hz, 1H), 6.37 (d, *J*= 2.3 Hz, 1H), 2.53 (s, 3H). MS (ESI): m/z 447.14 $[M + H]^+$. Mp 234-236°C.

Example 86

Synthesis of 9-[3-(6-fluoropyridyl)]imino-2-oxo-1-[3-(trifluoromethyl)phenyl]-1,2-dihydrobenzo[*h*][1,6]naphthyridine (HTL-6-20)

The synthesis was carried out according to the synthetic method of HTL-6-11 to obtain a yellow solid, with a yield of 83%. $^1$H-NMR (400 MHz, DMSO-$D_6$) $\delta$ 8.90 (s, 1H), 8.34 (s, 1H), 8.23 (d, *J* = 9.5 Hz, 1H), 7.93 (d, *J* = 9.0 Hz, 1H), 7.71 (s, 1H), 7.59 (ddd, *J* = 36.5, 19.9, 7.9 Hz, 4H), 7.35 - 7.24 (m, 2H), 7.04 (dd, *J* = 8.7, 3.2 Hz, 1H), 6.84 (d, *J*= 9.4 Hz, 1H), 6.32 (d, *J* = 2.3 Hz, 1H). MS (ESI): m/z 451.11 $[M + H]^+$. Mp 232-233°C.

Example 87

Synthesis of 9-[3-(6-chloropyridyl)]imino-2-oxo-1-[3-(trifluoromethyl)phenyl]-1,2-dihydrobenzo[*h*][1,6]naphthyridine (HTL-6-21)

The synthesis was carried out according to the synthetic method of HTL-6-11 to obtain a yellow solid, with a yield of 82%. $^1$H-NMR (400 MHz, DMSO-$D_6$) $\delta$ 8.95 (s, 1H), 8.51 (s, 1H), 8.25 (d, *J*= 9.5 Hz, 1H), 7.96 (d, *J*= 8.9 Hz, 1H), 7.81 (d, *J*= 2.7 Hz, 1H), 7.77 (s, 1H), 7.69 (d, *J* = 8.0 Hz, 1H), 7.62 (t, *J* = 7.8 Hz, 1H), 7.55 (d, *J* = 7.7 Hz, 1H), 7.35 (dd, *J* = 9.0, 2.4 Hz, 1H), 7.30 (d, *J* = 8.5 Hz, 1H), 7.11 (dd, *J* = 8.6, 3.0 Hz, 1H), 6.86 (d, *J* = 9.4 Hz, 1H), 6.46 (d, *J* = 2.3 Hz, 1H). MS (ESI): m/z 467.08 $[M + H]^+$. Mp 137-138°C.

Example 88

Synthesis of 9-(4-cyanophenyl)imino-2-oxo-1-[3-(trifluoromethyl)phenyl]-1,2-dihydrobenzo[*h*][1,6]naphthyridine (HTL-6-22)

The synthesis was carried out according to the synthetic method of HTL-6-11 to obtain a yellow solid, with a yield of 84%. $^1$H-NMR (400 MHz, DMSO-$D_6$) $\delta$ 9.00 (s, 1H), 8.88 (s, 1H), 8.27 (d, *J* = 9.5 Hz, 1H), 8.00 (d, *J* = 8.9 Hz, 1H), 7.86 (s, 1H), 7.68 (dd, *J* = 15.2, 4.4 Hz, 3H), 7.54 (d, *J* = 8.6 Hz, 2H), 7.40 (dd, *J* = 8.9, 2.2 Hz, 1H), 6.88 (d, *J* = 9.4 Hz, 1H), 6.71 (dd, *J*= 5.4, 3.1 Hz, 3H). MS (ESI): m/z 457.12 $[M + H]^+$. Mp 259-260°C.

Example 89

Synthesis of 9-(4-fluorophenyl)imino-2-oxo-1-[3-(trifluoromethyl)phenyl]-1,2-dihydrobenzo[*h*][1,6]naphthyridine (HTL-6-23)

The synthesis was carried out according to the synthetic method of HTL-6-11 to obtain a yellow solid, with a yield of 80%. $^1$H-NMR (400 MHz, DMSO-$D_6$) δ 8.90 (s, 1H), 8.26 - 8.15 (m, 2H), 7.91 (d, *J* = 9.0 Hz, 1H), 7.70 - 7.62 (m, 2H), 7.57 (dd, *J* = 18.8, 7.7 Hz, 2H), 7.31 (dd, *J* = 9.0, 2.3 Hz, 1H), 7.05 (t, *J* = 8.8 Hz, 2H), 6.84 (d, *J* = 9.4 Hz, 1H), 6.75 - 6.65 (m, 2H), 6.37 (d, *J*= 2.3 Hz, 1H). MS (ESI): m/z 450.12 $[M + H]^+$. Mp 176-179°C.

Example 90

Synthesis of 9-[(4-fluoro-3-methyl)phenyl]imino-2-oxo-1-[3-(trifluoromethyl)phenyl]-1,2-dihydrobenzo[*h*][1,6]naphthyridine (HTL-6-24)

The synthesis was carried out according to the synthetic method of HTL-6-11 to obtain a yellow solid, with a yield of 78%. $^1$H-NMR (400 MHz, DMSO-$D_6$) δ 8.84 (s, 1H), 8.21 (d, *J*= 9.4 Hz, 1H), 8.00 (s, 1H), 7.87 (d, *J*= 9.0 Hz, 1H), 7.67 - 7.57 (m, 2H), 7.54 - 7.42 (m, 2H), 7.26 (dd, *J*= 9.0, 2.4 Hz, 1H), 7.02 - 6.93 (m, 1H), 6.81 (d, *J*= 9.4 Hz, 1H), 6.69 (dd, *J*= 6.8, 2.5 Hz, 1H), 6.52 - 6.44 (m, 1H), 6.25 (d, *J*= 2.3 Hz, 1H), 2.21 (d, *J*= 1.6 Hz, 3H). MS (ESI): m/z 464.13 $[M + H]^+$. Mp 210-211°C.

Example 91

Synthesis of 9-(4-chlorophenyl)imino-2-oxo-1-[3-(trifluoromethyl)phenyl]-1,2-dihydrobenzo[*h*][1,6]naphthyridine (HTL-6-25)

The synthesis was carried out according to the synthetic method of HTL-6-11 to obtain a yellow solid, with a yield of 83%. $^1$H-NMR (400 MHz, DMSO-$D_6$) δ 8.89 (s, 1H), 8.31 (s, 1H), 8.23 (d, *J* = 9.4 Hz, 1H), 7.92 (d, *J* = 8.9 Hz, 1H), 7.73 (s, 1H), 7.67 (d, *J* = 7.9 Hz, 1H), 7.61 (t, *J* = 7.8 Hz, 1H), 7.55 (d, *J* = 7.7 Hz, 1H), 7.31 (dd, *J* = 9.0, 2.4 Hz, 1H), 7.26 - 7.14 (m, 2H), 6.83 (d, *J*= 9.4 Hz, 1H), 6.72 - 6.62 (m, 2H), 6.50 (d, *J*= 2.3 Hz, 1H). MS (ESI): m/z 466.09 $[M + H]^+$. Mp 212-213°C.

Example 92

Synthesis of 9-(4-methoxyphenyl)imino-2-oxo-1-[3-(trifluoromethyl)phenyl]-1,2-dihydrobenzo[*h*][1,6]naphthyridine (HTL-6-26)

The synthesis was carried out according to the synthetic method of HTL-6-11 to obtain a yellow solid, with a yield of 84%. $^1$H-NMR (400 MHz, DMSO-$D_6$) δ 8.83 (s, 1H), 8.19 (d, *J* = 9.4 Hz, 1H), 7.93 (s, 1H), 7.85 (d, *J*= 9.0 Hz, 1H), 7.68 - 7.55 (m, 2H), 7.55 - 7.46 (m, 2H), 7.26 (dd, *J* = 9.0, 2.4 Hz, 1H), 6.82 (ddd, *J* = 9.3, 6.1, 3.3 Hz, 3H), 6.68 - 6.57 (m, 2H), 6.21 (d, *J*= 2.3 Hz, 1H), 3.81 (s, 3H). MS (ESI): m/z 462.14 $[M + H]^+$. Mp 138-140°C.

Example 93

Synthesis of 9-(4-acetylaminophenyl)imino-2-oxo-1-[3-(trifluoromethyl)phenyl]-1,2-dihydrobenzo[*h*][1,6]naphthyridine (HTL-6-27)

The synthesis was carried out according to the synthetic method of HTL-6-11 to obtain a yellow solid with a yield of 79%. $^1$H-NMR (400 MHz, DMSO-$D_6$) δ 9.85 (s, 1H), 8.83 (s, 1H), 8.20 (d, *J*= 9.4 Hz, 1H), 8.02 (s, 1H), 7.86 (d, *J*= 9.0 Hz, 1H), 7.70 (d, *J*= 7.8 Hz, 1H), 7.62 - 7.45 (m, 3H), 7.42 (d, *J*= 8.8 Hz, 2H), 7.27 (dd, *J*= 9.0, 2.4 Hz, 1H), 6.81 (d, *J*= 9.4 Hz, 1H), 6.61 (d, *J* = 8.8 Hz, 2H), 6.30 (d, *J* = 2.3 Hz, 1H), 2.09 (s, 3H). MS (ESI) : m/z 489.15 $[M + H]^+$. Mp 175-177°C.

Example 94

Synthesis of 9-[2-(6-aminopyrazinyl)]imino-2-oxo-1-[3-(trifluoromethyl)phenyl]-1,2-dihydrobenzo[*h*][1,6]naphthyridine (HTL-6-28)

The synthesis was carried out according to the synthetic method of HTL-6-11 to obtain a yellow solid, with a yield of 72%. $^1$H-NMR (400 MHz, DMSO-$D_6$) δ 9.55 (s, 1H), 9.30 (s, 1H), 8.36 (d, *J* = 9.6 Hz, 1H), 8.11 (d, *J* = 9.0 Hz, 1H), 8.03 (d, *J* = 7.7 Hz, 1H), 7.78 (s, 1H), 7.69 (dt, *J* = 16.2, 7.7 Hz, 2H), 7.40 (d, *J* = 2.1 Hz, 1H), 7.32 (s, 1H), 7.19 (s, 1H), 7.02 (d, *J* = 9.5 Hz, 1H), 6.69 (s, 2H). MS (ESI): m/z 449.13 $[M + H]^+$. Mp 192-194°C.

Example 95

Synthesis of 9-[3-(5-aminopyridyl)]imino-2-oxo-1-[3-(trifluoromethyl)phenyl]-1,2-dihydrobenzo[*h*][1,6]naphthyridine (HTL-6-29)

The synthesis was carried out according to the synthetic method of HTL-6-11 to obtain a yellow solid, with a yield of 82%. $^1$H-NMR (400 MHz, DMSO-$D_6$) δ 8.89 (s, 1H), 8.25 (d, *J* = 9.5 Hz, 1H), 8.14 (s, 1H), 7.90 (d, *J* = 8.9 Hz, 1H), 7.79 (d, *J* = 7.7 Hz, 1H), 7.62 (dd, *J* = 8.8, 6.9 Hz, 2H), 7.56 (d, *J* = 2.3 Hz, 1H), 7.48 (d, *J* = 7.9 Hz, 1H), 7.30 (dd, *J* = 9.0, 2.4 Hz, 1H), 7.22 (d, *J* = 2.2 Hz, 1H), 6.85 (d, *J* = 9.4 Hz, 1H), 6.41 (t, *J* = 2.3 Hz, 1H), 6.33 (d, *J* = 2.3 Hz, 1H), 5.43 (s, 2H). MS (ESI): m/z 448.13 $[M + H]^+$. Mp 156-158°C.

Example 96

Synthesis of (*E*)-9-(2-carbamoyl-vinyl)-2-oxo-1-[3-(trifluoromethyl)phenyl]-1,2-dihydrobenzo[*h*][1,6]naphthyridine (HTL-7-24)

9-Bromo-2-oxo-1-[3-(trifluoromethyl)phenyl]-1,2-dihydrobenzo[*h*][1,6]naphthyridine (4.18 g, 10 mmol) was dissolved in a solution of DMF, then $Pd(OAc)_2$ (0.45 g, 2 mmol), acrylamide (1.42 g, 20 mmol), tri(o-tolyl)phosphine (1.22 g, 4 mmol) and $Et_3N$ (10.12 g, 100 mmol) were added. Under the protection of $N_2$, the mixed solution was heated to 100°C and reacted for 2 hours. After the completion of the reaction monitored by TLC, the reaction solution was diluted with water and extracted with ethyl acetate. The organic layer was washed with saturated brine, dried with $MgSO_4$, and filtered, and the filtrate was dried by a rotary evaporator, and purified by column chromatography (n-hexane/ethyl acetate 5:1) to obtain 3.40 g of a yellow solid, with a yield of 83%. $^1$H-NMR (400 MHz, DMSO-$D_6$) δ 9.12 (s, 1H), 8.29 (d, *J* = 9.5 Hz, 1H), 7.99 (ddd, *J* = 25.9, 19.1, 7.9 Hz, 4H), 7.87 - 7.80 (m, 2H), 7.40 (s, 1H), 7.16 (s, 1H), 6.92 (d, *J* = 9.4 Hz, 1H), 6.79 (d, *J* = 15.7 Hz, 1H), 6.55 (s, 1H), 6.25 (d, *J* = 15.7 Hz, 1H). MS (ESI): m/z 410.10 $[M + H]^+$. Mp 307-308°C.

Example 97

Synthesis of (*E*)-9-[3-ureido-propenyl]-2-oxo-1-[3-(trifluoromethyl)phenyl]-1,2-dihydrobenzo[*h*][1,6]naphthyridine (HTL-7-25)

The synthesis was carried out according to the synthetic method of HTL-7-24 to obtain a yellow solid, with a yield of 80%. $^1$H-NMR (400 MHz, DMSO-$D_6$) δ 9.06 (s, 1H), 8.26 (dd, *J* = 10.2, 6.3 Hz, 2H), 8.07 (s, 1H), 7.96 - 7.69 (m, 5H), 6.98 (d, *J* = 11.1 Hz, 1H), 6.88 (d, *J* = 9.4 Hz, 1H), 6.73 (d, *J* = 1.7 Hz, 1H), 6.08 (s, 1H), 1.88 (s, 1H), 1.30 (s, 2H), 1.00 (d, *J* = 6.1 Hz, 1H). MS (ESI): m/z 439.13 $[M + H]^+$. Mp 150-151°C.

Example 98

Synthesis of (*E*)-9-[2-ethoxycarbonyl-vinyl]-2-oxo-1-[3-(trifluoromethyl)phenyl]-1,2-dihydrobenzo[*h*][1,6]naphthyridine (HTL-7-26)

The synthesis was carried out according to the synthetic method of HTL-7-24 to obtain a yellow solid, with a yield of 81%. $^1$H-NMR (400 MHz, DMSO-$D_6$) δ 9.14 (s, 1H), 8.30 (d, *J* = 9.5 Hz, 1H), 8.09 (s, 1H), 8.05 - 7.93 (m, 3H), 7.89 (t, *J* = 7.9 Hz, 1H), 7.77 (d, *J* = 8.1 Hz, 1H), 7.09 (d, *J* = 16.0 Hz, 1H), 6.93 (d, *J* = 9.4 Hz, 1H), 6.68 (s, 1H), 6.04 (d, *J* = 16.0 Hz, 1H), 4.21 - 4.02 (m, 2H), 1.24 (t, *J* = 7.1 Hz, 3H). MS (ESI): m/z 439.12 $[M + H]^+$. Mp 210-212°C.

Example 99: Experiment of in vitro anti-EV71 activity of the compound of the present application

Experimental method:
According to the mass and molecular weight of the compound to be tested, the compound to be tested was dissolved to 100 mM (mmol/L) with DMSO.
Detection of antiviral activity:

① First, the compound to be tested was diluted to 800 μM with cell maintenance solution (DMEM+2% FBS, Gibco, catalog numbers: 11995-065, 1600-044), and three-fold gradient dilution was performed to prepare 10 concentrations in total. The diluted compound was added to a 96-well plate with white wall and transparent bottom, 50μl per well. To both the cell control group and the virus control group, an equal volume of the cell maintenance solution was added.

② EV71 virus (purchased from ATCC) was taken out from the -80°C refrigerator and equilibrated to room temperature.

③ The virus seed was diluted with a virus growth solution (DMEM+2%FBS, Gibco, catalog numbers: 11995-065, 1600-044) to 100$TCID_{50}$, the diluted virus seed was added to the 96-well plate of ①, with 50μl per well. The same volume of the virus growth solution was added to the cell control group.

④ RD cells (purchased from ATCC) were inoculated at a concentration of $1*10^5$/mL into the 96-well plate of ①, with 100 μL per well, to reach a final volume of 200 μL per well. The final concentration of drug was 0.25 times the initial concentration.

⑤ RD cells were cultured at 37°C for 4 days for testing.

⑥ The Buffer of CellTiter-Glo® luminescent cell viability assay reagent (Promega) was mixed with the substrate in the dark to prepare a working solution.

⑦ The culture medium in the plate was discarded, the plate was dried by patting, 100 μL of the detection reagent was added to each well, and the 96-well plate was shaken on an orbital shaker for 4 minutes to induce cell lysis. After keeping in the dark and subjecting to signal stabilization for 15 minutes, the chemiluminescence unit was detected by using MD5 microplate reader (Molecular Devices), and the plate reading program was performed according to the CellTiter-Glo preset program.

The inhibition rate-concentration curve was fitted to S-curve by using origin8.0 software to calculate the $IC_{50}$ value of the compound to be tested.
Detection of cytotoxicity:

① The compound to be tested was diluted to a concentration of 400 μM with the cell maintenance solution, and 3-fold gradient dilution was carried out to obtain 10 concentrations in total.

② The diluted compound was added to a 96-well plate with white wall and transparent bottom, with 100 μL per well. An equal volume of the cell maintenance solution was added to the cell control group.

③ RD cells were inoculated into the above 96-well plate at a concentration of $1*10^5$/mL, with 100 μL per well, to reach a final volume of 200 μL per well, and the final concentration of drug was 0.5 times the pretreatment concentration.

④ The RD cells were cultured at 37°C for 4 days for testing.

⑤ The Buffer of CellTiter-Glo® luminescent cell viability assay reagent was mixed with the substrate in the dark to prepare a working solution.

⑥ The culture medium in the plate was discarded, the plate was dried by patting, 100 μL of the detection reagent was added to each well, and the 96-well plate was shaken on an orbital shaker for 4 minutes to induce cell lysis. After keeping in the dark and subjecting to signal stabilization for 15 minutes, the chemiluminescence unit was detected, and the plate reading program was performed according to the CellTiter-Glo preset program.

The formula for calculating the inhibition rates of drug at different dilution degrees was as follows: inhibition

rate (%) = (average value of cell control group - value of experimental group) / (average value of cell control group - minimum value of experimental group) * 100

Data analysis:

The inhibition rate-concentration curve was fitted to S-curve by using origin8.0 software, and the $IC_{50}$ value of the compound to be tested was calculated. The same method was used to calculate the $TD_{50}$ value, and the selection index $SI=TD_{50}/IC_{50}$ was calculated according to the half maximal inhibitory concentration $IC_{50}$ and the median toxic dose $TD_{50}$.

The results of inhibitory activity of the compounds of Formula I, Formula II and Formula III against Enterovirus 71 (EV71) H strain were shown in the following tables:

Table 1: Inhibitory activity of compounds of Formula I against EV71

| Code | $IC_{50}$(μM) | $TD_{50}$(μM) | SI | Code | $IC_{50}$(μM) | $TD_{50}$(μM) | SI |
|---|---|---|---|---|---|---|---|
| HTL-2-34 | 1.78±0.03 | 4.99±1.55 | 2.80 | HTL-3-26 | 0.85±0.03 | 1.43±1.62 | 1.68 |
| HTL-2-35 | 3.70±0.01 | 5.14±1.92 | 1.39 | HTL-3-32 | >200 | 1.52±0.87 | - |
| HTL-2-38 | 2.85±1.20 | 8.71±1.53 | 3.06 | HTL-3-33 | >200 | 0.72±0.66 | - |
| HTL-2-42 | >200 | >200 | - | HTL-3-34 | >200 | 3.02±0.91 | - |
| HTL-3-04 | >200 | >200 | - | HTL-3-36 | >200 | 4.65±3.50 | - |
| HTL-3-07 | >200 | >200 | - | HTL-3-37 | >200 | 1.88±0.46 | - |
| HTL-3-11 | >200 | >200 | - | HTL-3-39 | >200 | 1.75±0.73 | - |
| HTL-3-12 | >200 | >200 | - | HTL-3-40 | >200 | 0.83±0.03 | - |
| HTL-3-15 | >200 | >200 | - | HTL-3-41 | >200 | 0.99±0.24 | - |
| HTL-3-18 | >200 | >200 | - | HTL-3-42 | >200 | 0.96±0.15 | - |
| HTL-3-16 | >200 | 0.83±0.79 | - | HTL-3-43 | >200 | 2.23±0.08 | - |
| HTL-3-22 | >200 | 1.55±1.03 | - | HTL-3-45 | >200 | 2.42±0.06 | - |
| HTL-3-24 | >200 | 1.35±1.16 | - | HTL-3-46 | >200 | 3.59±2.46 | - |
| HTL-3-25 | >200 | 0.68±0.21 | - | HTL-7-22 | >200 | 9.4±8.0 | - |

The in vitro anti-EV71 activity test results of 28 compounds represented by Formula I showed that, except for HTL-2-34, HTL-2-35, HTL-2-38 and HTL-3-26 which had moderate anti-EV71 activity, other 3-butenone quinoline compounds didn't show inhibitory activity against EV71.

Table 2: Inhibitory activity of compounds of Formula II against EV71

| Code | $IC_{50}$(μM) | $TD_{50}$(μM) | SI | Code | $IC_{50}$(μM) | $TD_{50}$(μM) | SI |
|---|---|---|---|---|---|---|---|
| HTL-4-32 | 5.57±1.53 | 14.5±6.4 | 2.60 | HTL-5-35 | >200 | 69.36±0.28 | - |
| HTL-5-21 | 2.00±0.30 | 6.95±1.23 | 3.48 | HTL-5-36 | >200 | 13.41±0.18 | - |
| HTL-5-23 | 4.78±1.73 | 16.88±5.46 | 3.53 | HTL-6-30 | 3.70±0.21 | 7±0.09 | 1.89 |
| HTL-5-25 | >200 | >200 | - | HTL-6-31 | >200 | 9.20±2.34 | - |
| HTL-5-26 | >200 | >200 | - | HTL-6-32 | >200 | >200 | - |
| HTL-5-27 | >200 | >200 | - | HTL-6-33 | >200 | 71.99±0.78 | - |
| HTL-5-28 | >200 | >200 | - | HTL-6-34 | >200 | 66.64±1.17 | 1.36 |
| HTL-5-29 | >200 | >200 | - | HTL-6-35 | >200 | 76.02±11.52 | - |
| HTL-5-30 | >200 | 68.52±0.42 | - | HTL-6-38 | >200 | 22.74±0.01 | - |
| HTL-5-32 | >200 | 68.88±0.13 | - | WSX-1-13 | 100±2.34 | 73.88±3.52 | 0.74 |
| HTL-5-33 | >200 | >200 | - | WSX-1-24 | >200 | 4.27±2.83 | - |
| HTL-5-34 | >200 | >200 | - | HTL-7-23 | >200 | >200 | - |

The in vitro anti-EV71 activity test results of 24 compounds represented by Formula II showed that HTL-4-32, HTL-5-21, HTL-5-23 and HTL-6-30 showed moderate inhibitory activity against EV71, WSX-1-13 had weak inhibitory activity against EV71, and other compounds did not show inhibitory activity.

Table 3: Inhibitory activity of compounds of Formula III agaist EV71

| Code | $IC_{50}$(μM) | $TD_{50}$(μM) | SI | Code | $IC_{50}$(μM) | $TD_{50}$(μM) | SI |
|---|---|---|---|---|---|---|---|
| HTL-6-11 | 0.89 | 7.58±1.24 | 8.52 | HTL-7-03 | 0.04±0.01 | 0.13±0.03 | 3.25 |
| HTL-6-12 | 1.23±0.22 | 5.19±0.56 | 4.22 | HTL-7-04 | 10.53±0.56 | 10.27±7.81 | 0.98 |
| HTL-6-16 | >200 | 7.06±0.23 | - | HTL-7-05 | 0.13±0.06 | 0.75±0.36 | 5.77 |
| HTL-6-17 | >200 | 6.81±1.03 | - | HTL-7-06 | 0.05±0.01 | 0.17±0.06 | 3.40 |
| HTL-6-18 | >200 | 7.04±0.68 | - | HTL-7-07 | 0.38±0.39 | 0.46±0.03 | 1.21 |
| HTL-6-19 | >200 | 22.62±2.15 | - | HTL-7-08 | 0.20±0.02 | 0.70±0.48 | 3.50 |
| HTL-6-20 | >200 | 59.31±3.98 | - | HTL-7-10 | 0.39±0.18 | 0.74±0.72 | 1.90 |
| HTL-6-21 | >200 | 20.75±2.11 | - | HTL-7-11 | 0.095±0.007 | 0.19±0.10 | 2.00 |
| HTL-6-22 | >200 | 6.95±0.86 | - | HTL-7-12 | 36.93±0.41 | >200 | >5.42 |
| HTL-6-23 | >200 | 7.22±0.47 | - | HTL-7-13 | 26.34±12.92 | 200±0 | 7.59 |
| HTL-6-24 | >200 | 7.2±0.33 | - | HTL-7-14 | 23.72±1.02 | 1.00±0.81 | 0.04 |
| HTL-6-25 | >200 | 4.56±0.26 | - | HTL-7-15 | 0.09±0.02 | 0.20±0.09 | 2.22 |
| HTL-6-26 | >200 | >200 | - | HTL-7-16 | 0.09±0.01 | 0.17±0.06 | 1.89 |
| HTL-6-27 | 7.14±3.21 | 10.65±0.57 | 1.49 | HTL-7-17 | 0.10±0.03 | 0.18±0.15 | 1.80 |
| HTL-6-28 | 3.70±0.03 | 8.67±0.97 | - | HTL-7-18 | 1.80±1.17 | 2.91±1.16 | 1.62 |
| HTL-6-29 | 1.75±0.41 | 28.65±1.67 | 16.37 | HTL-7-19 | 0.045±0.01 | 0.06±0.02 | 1.33 |
| HTL-6-45 | 0.027±0.01 | 0.04±0.02 | 1.48 | HTL-7-20 | 0.10±0.01 | 1.79±0.67 | 17.90 |
| HTL-6-47 | 0.25±0.23 | 0.03±0.02 | 0.12 | HTL-7-21 | 2.08±2.29 | 0.69±0.50 | 0.33 |
| HTL-6-48 | 0.059±0 | 1.23±1.20 | 20.85 | HTL-7-24 | 0.09±0.01 | 0.15±0.01 | 1.67 |
| HTL-6-49 | 0.07±0.01 | 0.07±0.00 | 1.00 | HTL-7-25 | 0.41±0.03 | 4.42±0.45 | 10.78 |
| HTL-6-50 | >200 | 0.17±0.05 | - | HTL-7-26 | 0.28±0.20 | 1.29±0.57 | 4.61 |
| HTL-7-01 | 0.07±0.01 | 0.23±0.33 | 3.29 | HTL-7-28 | 0.09±0.01 | 0.23±0.01 | 2.56 |
| HTL-7-02 | >200 | >200 | - | HTL-7-29 | 0.09±0.00 | 0.29±0.03 | 3.22 |

Among the 46 compounds represented by Formula III, some of the compounds showed strong in vitro anti-EV71 activity, in which aromatic hydrocarbon-substituted compounds represented by Formula III, such as HTL-6-45, HTL-6-48, HTL-6-49, HTL-7-1, HTL-7-3, HTL-7-6, HTL-7-11, HTL-7-15, HTL-7-16, HTL-7-19, HTL-7-28, and HTL-7-29, showed similar activity to that of the positive control compound, their activities were in the same order of magnitude, and their selection index SI values were all greater than 1. Among them, the SI value of HTL-6-48 was greater than 20, and it showed high activity and relatively low toxicity. Among the imine-substituted compounds represented by Formula III, HTL-6-11, HTL-6-12, HTL-6-27, HTL-6-28 and HTL-6-29 showed moderate activity. Among the alkene -substituted compounds represented by Formula III, HTL-7-24 showed strong inhibitory activity, while HTL-7-25 and HTL-7-26 had moderate inhibitory activity on EV71.

Example 100: Experiment of in vitro inhibitory activity of the compounds of the present application on mTOR kinase

Experimental method:
Preparation of reaction buffers:
Basic buffer composition: 50 mM (mmol/L) HEPES (pH 7.5), 1 mM EGTA, 0.01% Tween-20, 10 mM $MnCl_2$, 2 mM DTT (diluted from 500 mM before used).

① Substrate buffer solution: 1650 μL of 2.5× substrate buffer solution was composed of 1559.6 μL of 1× basic buffer, 89.2 μL of GFP-4E-BP1 (18.5 μM stock solution, purchased from Thermo Fisher, catalog number: PV4759) and 1.2 μL of ATP (10 mM), the final concentrations were 0.4 μM GFP-4E-BP1, 3 μM ATP.

② mTOR kinase buffer solution: 1650 μL of 2.5× mTOR kinase buffer solution was composed of 1640.2 μL of 1× basic buffer and 9.8 μL of mTOR (0.21 mg/mL stock solution), and the final concentration was 0.5 μg/mL.

③ Detection buffer solution: 3960 μL of 2× detection buffer solution was composed of 3797.1 μL of TR-FRET buffer diluent (purchased from Thermo Fisher, catalog number: PV3574), 4.5 μL of Tb-anti-p4E-BP1 antibody (3.49 μM stock solution, purchased from Thermo Fisher, catalog number: PV4757) and 158 μL of EDTA (500 mM stock solution), and the final concentrations were 2 nM Tb-anti-p4E-BP1 antibody and 10 mM EDTA.

Experimental steps:

① 20 μL of 100% DMSO solution containing 5 mM compound to be tested was added to a 96-well plate.

② The compound was serially diluted 3 times in DMSO.

③ 1 μL of the compound in the previous step was taken, diluted with 19 μL of mTOR kinase buffer, and transferred to another 96-well plate.

④ 4 μL of mTOR kinase solution (purchased from Thermo Fisher, catalog number: PV4753) was added to a 384-well plate.

⑤ 2 μL of the compound in ③ was taken out and added to the 384-well plate with mTOR kinase, and incubated at room temperature for 15 minutes.

⑥ 4 μL of substrate solution was added to initiate the reaction.

The final concentrations of mTOR reaction solution were: 0.5 μg/mL mTOR, 0.4 μM GFP-4E-BP1, 3 μM ATP.
The final concentrations of the test compound were: 50000, 16666, 5555, 1851, 617.3, 205.8, 68.58, 22.86, 7.62, 2.54 and 0.85 nM.
The final concentration of the DMSO solution was 1%.

⑦ Incubation was performed for 60 minutes at room temperature.

⑧ 10 μL of detection buffer was added. The final concentrations were 2 nM Tb-anti-p4E-BP1 antibody and 10 mM EDTA.

⑨ Incubation was performed for 30 minutes at room temperature.

⑩ TR-FRET value was read on MD5 multi-mode plate reader (Molecular Devices). The excitation wavelength was 340 nm, the emission wavelength 1 was 495 nm, and the emission wavelength 2 was 520 nm. The ratio of 520nm/495nm readings was calculated as TR-FRET value.

Data processing:
$IC_{50}$ of compound was fitted by nonlinear regression equation:

$$Y = Bottom+(Top-Bottom)/(1+10\text{^}((LogIC_{50}-X)*HillSlope));$$

X: common logarithm value of compound concentration; Y: TR-FRET value (520nm/495nm).

Table 4: Inhibitory activity of some compounds on mTOR kinase

| Compound name | $IC_{50}$(nM) | Compound name | $IC_{50}$(nM) |
|---|---|---|---|
| HTL-2-35 | 7766 | HTL-7-06 | 4.82 |

(continued)

| Compound name | $IC_{50}$(nM) | Compound name | $IC_{50}$(nM) |
|---|---|---|---|
| HTL-2-38 | 1013 | HTL-7-07 | 30.17 |
| HTL-5-21 | 15.05 | HTL-7-08 | 76.60 |
| HTL-5-23 | 256.80 | HTL-7-10 | 34.79 |
| HTL-6-30 | 47.57 | HTL-7-11 | 32.51 |
| HTL-6-34 | 848.60 | HTL-7-12 | 1206 |
| HTL-6-11 | 255.30 | HTL-7-13 | 86.13 |
| HTL-6-12 | 68.16 | HTL-7-14 | 306.30 |
| HTL-6-45 | 29.24 | HTL-7-15 | 25.03 |
| HTL-6-47 | 6.17 | HTL-7-16 | 8.91 |
| HTL-6-48 | 31.46 | HTL-7-17 | 1.15 |
| HTL-6-49 | 11.94 | HTL-6-50 | 158.60 |
| HTL-7-01 | 28.76 | HTL-7-18 | 22.72 |
| HTL-7-02 | 974.20 | HTL-7-19 | 7.58 |
| HTL-7-03 | 7.25 | HTL-7-20 | 11.29 |
| HTL-7-04 | 239.90 | HTL-7-21 | 11.88 |
| HTL-7-05 | 39.03 | | |

In this example, the inhibitory activity on mTOR kinase of some compounds of Formula I, Formula II and Formula III that had in vitro EV71 inhibitory activity was tested. The results showed that, similar to the experimental results of the in vitro inhibitory activity against EV71, the compounds represented by Formula I HTL-2-35 and HTL-2-38 had weak inhibitory activity on mTOR kinase; among the 4 compounds represented by Formula II, HTL-5-21 and HTL-6-30 had better inhibitory activity, while HTL-5-23 and HTL-6-34 merely showed moderate inhibitory activity; among the 27 compounds represented by Formula III, except for HTL-6-11, HTL-7-02, HTL-7-04, HTL-7-14 and HTL-6-50 which had moderate inhibitory activity, other compounds showed good enzyme inhibitory activity, in which HTL-6-47, HTL-7-03, HTL-7-06, HTL-7-16, HTL-7-17 and HTL-7-19 showed excellent inhibitory activity, $IC_{50}$ of which reached nM level.

Example 101: Molecular mechanism experiment of the compounds of the present application

In order to test the inhibitory activity of the synthesized compounds on the two mTOR complexes, the inhibitory activity experiments of mTORC1 and mTORC2 were carried out. Since mTORC1 and mTORC2 exerted their function by activating the phosphorylation of downstream substrates, the inhibitory activity of the compounds on mTORC1 and mTORC2 could be determined by detecting the phosphorylation levels of the Thr389 site of mTORC1 downstream substrate p70S6K1 and the Ser473 site of mTORC2 downstream substrate Akt.

Experimental method:

Pretreatment of compound:

① RD cells were cultured in DMEM medium containing 10% FBS and 1×PS (penicillin and streptomycin were at concentrations 100 IU and 100 μg/mL, respectively) at 37°C and 5% $CO_2$.

② The RD cells ($5\times10^5$ cells/2 mL medium) were inoculated into a 6-well plate and incubated at 37°C and 5% $CO_2$ for 24 hours.

③ The cells were washed once with PBS, and the cells were cultured in serum-free medium without nutrients overnight.

④ Formulation of compounds:

Preparation of insulin medium: Insulin was diluted in DMEM medium containing 10% FBS and 1×PS so that the final concentration of insulin was 167 nM.

Pretreatment of compound to be tested: The compound was dissolved in DMSO so that the concentration of the test compound was 20 mM, and the compound was diluted in 167 nM insulin medium to reach a concentration of 20 .

Preparation of rapamycin solution: Rapamycin was dissolved in DMSO to a concentration of 10 mM, and the rapamycin was diluted in 167 nM insulin medium to reach a concentration of 20 .

⑤ The serum-free medium in each well was removed.

⑥ 2 mL of complete medium containing DMSO was added to each well as a control carrier. The final concentration of DMSO was 0.2%.

⑦ 2 mL of 20 μM rapamycin solution and 20 μM the compound to be tested were added to the designated wells, respectively. The final concentration of DMSO was 0.2%.

⑧ The cells were incubated for 2 hours at 37°C and 5% $CO_2$.

Protein extraction and concentration determination:

① The cells were washed once with refrigerated PBS, and then the PBS was discarded.

② 150 μL of cell extraction buffer (RIPA, APPLYGEN, catalog number: C1053) was transferred into each well to lyse the cells, and then the resulting cell solution were incubated on ice for 30 minutes.

③ Centrifugation was performed at 14000 rpm (13000× g) for 30 minutes at 4°C.

④ The supernatant was transferred into a new eppendorf tube, and the cell lysate was stored at -80°C before testing.

⑤ The protein concentration was determined by BCA method.

Preparation of buffer solutions:

① 100× Protease inhibitor (Beyotime, catalog number: P1005): 1 mL of redistilled water was added to the protease inhibitor and stirred gently until the solid was completely dissolved.

② Lysis Buffer: 2 mL of 100× protease inhibitor and 2× phosphatase inhibitor Cocktails PhosSTOP (Beyotime, catalog number: P1082) were added to 100 mL of the cell extract, and stirred gently until it was completely dissolved.

③ Electrophoresis Running Buffer:
10× MOPS buffer: 52.33 g of MOPS, 30.29 g of Tris base, 10 mL of 0.5 mol/L EDTA (pH 8.5) and 5 g of SDS was dissolved in 400 mL of redistilled water, stirred to dissolve, and the pH was adjusted to 7.5, then redistilled water was added again to reach a volume of 500 mL; 1× MOPS: 100 mL of 10× MOPS was diluted with redistilled water to 1000 mL.

④ 1× Transfer Buffer: 100 mL of 10× transfer buffer (144g of glucine, 30.3g of trisbase, and distilled water were mixed to reach a volume of 1L) and 400 mL of methanol were dissolved in 1500 mL of redistilled water, and then the redistilled water was added to reach a volume of 2000 mL.

⑤ 10× PBS Buffer (0.1M): 5 bags of PBS powder (Solarbio, catalog number: P1010) was added into 800 mL of redistilled water, stirred to dissolve, adjusted to have a pH of 7.6, and then the redistilled water was added to reach a volume of 1000 mL.

⑥ 1× PBS Buffer: 100 mL of 10× PBS buffer was diluted to 1000 mL with redistilled water.

⑦ 10% Tween-20: 20 mL of Tween-20 was added to 180 mL of redistilled water, and stirred well.

⑧ 1× PBST Buffer: 100 mL of 10× PBST buffer and 10 mL of Tween-20 were diluted to 1000 mL with re-distilled water.

⑨ Primary antibody incubation: the primary antibodies (Thermo Fisher, catalog numbers: B2H9L2 and PA5-85513) were diluted with 0.1% Tween-20 in the blocking solution (5% skimmed milk) at a ratio of 1:1000.

⑩ Secondary antibody incubation: IRDye 800CW Goat anti-Rabbit IgG (Abcam, catalog number: ab216773) was diluted with 0.1% Tween-20 in the blocking buffer at a ratio of 1:500.

Western Blot experiment:

① 12 μg of total protein was added to the sample well of SDS-PAGE. Electrophoresis were performed at constant voltage of 120V until the blue marker reached the end of the gel.

② At 120 V, the protein on the gel was transferred to the PVDF membrane for 40 minutes by using the BIO-RAD Trans-Blot.

③ After transferring, the blocking was carried out with blocking buffer at room temperature for 2 hours.

④ The membrane was incubated with the corresponding primary antibody solution on a constant temperature shaker at 4°C overnight.

⑤ The membrane was rinsed with 1× PBST Buffer for 3×10 min, and then incubated with the secondary antibody solution at room temperature for 1 hour.

⑥ The membrane was washed with 1× PBST Buffer for 3×10 min, and scanned and developed with Odyssey Infrared Imaging System.

The RD cells were treated with rapamycin and 33 compounds of Formula I, Formula II and Formula III at concentration of 20 μM for 2 hours, respectively, and the Western blot results were shown in Figure 1.

The RD cells were treated with 33 compounds of Formula I, Formula II and Formula III of the present application and 2 positive drugs to investigate their effects on the phosphorylation levels of p70 and Akt, the downstream substrates of mTORC1 and mTORC2, to evaluate the inhibitory activity of the compounds on mTORC1 and mTORC2. Through the observation of the results of the Western blot experiment, it was found that under the same detection conditions, the p70 phosphorylation expression level of cells treated with rapamycin was significantly reduced. Among the synthesized compounds, HTL-2-38, HTL-5-21, HTL-6-30, HTL-6-11, HTL-6-12, HTL-6-45, HTL-6-47, HTL- 6-48, HTL-6-49, HTL-7-01, HTL-7-03, HTL-7-04, HTL-7-05, HTL-7-06, HTL-7-07, HTL-7- 08, HTL-7-10, HTL-7-11, HTL-7-13, HTL-7-14, HTL-7-15, HTL-7-16, HTL-7-17, HTL-6-50, HTL-7-18, HTL-7-19, HTL-7-20 and HTL-7-21 could significantly down-regulate the p70 phosphorylation expression level, indicating that the above compounds could inhibit mTORC1. In addition, the positive control drug rapamycin could not significantly down-regulate the phosphorylation level of Akt, indicating that it could not inhibit the phosphorylation of Akt. Among the synthesized compounds, HTL-5-21, HTL-6-11, HTL-6-12, HTL-6-45, HTL-6-47, HTL-6-48, HTL-6-49, HTL-7 -01, HTL-7-03, HTL-7-04, HTL-7-05, HTL-7-06, HTL-7-07, HTL-7-08, HTL-7-10, HTL-7-11, HTL-7-13, HTL-7-14, HTL-7-15, HTL-7-16, HTL-7-17, HTL-6-50, HTL-7-18, HTL-7-19, HTL -7-20 and HTL-7-21 could significantly down-regulate the phosphorylation expression level of Akt, indicating that the above compounds could inhibit mTORC2.

Example 102: Experiment of drug metabolism property of the compounds of the present application

The metabolic properties of drug are also important indicators for evaluating the pros and cons of the drug. In the in vitro anti-EV71 activity evaluation, some of the compounds represented by Formula III showed strong inhibitory activity against EV71. Among them, HTL-6-45, HTL-6-48, HTL-7-01, HTL-7-03, HTL-7-17 with excellent activity were selected, and their in vivo drug metabolism properties were evaluated.

Experimental method:

Experimental animals: C57 male mice (purchased from Beijing Vital River Laboratory Animal Technology Co., Ltd.), bred for 6-8 weeks and weighing 20-30 g, 3 mice in each group of iv/po groups, a total of 6 groups.

Preparation of sample solutions:

① Preparation of sample solution (1 mg/kg, 5 mL/kg) for injection (iv): 1 mg of the sample to be tested was dissolved in 0.5 mL DMSO, mixed by a vortex mixer, and treated ultrasonically to obtain a stock solution (2 mg/mL); 0.10 mL of the stock solution (2 mg/mL) was added to a vial, then 0.4 mL of PEG400 and 0.5 mL of water were added, and mixed by a vortex shaker, treated ultrasonically to obtain a sample solution with a concentration of 0.2 mg/mL.

② Preparation of sample solution (10 mg/kg, 10 mL/kg) for oral administration (po): 1 mg of the sample to be tested was dissolved in 1 mL of "0.5% CMC/0.1% Tween-80 aqueous solution", mixed by a vortex mixer, and treated ultrasonically to obtain a suspension of the sample to be tested with a concentration of 1 mg/mL.

Liquid chromatography method:

① Chromatographic column: Waters XSELECT CSH C18, 2.5 μm 2.1x50 mm chromatographic column.

② Mobile phase: Phase A: 5% acetonitrile in aqueous solution (0.1% formic acid); Phase B: 95% acetonitrile in aqueous solution (0.1% formic acid).

③ Flow rate: 0.6 mL/min.

④ Sampling volume: 20 μL.

For each compound, po group and iv group were set for administration. All experimental animals were fasted overnight before administration, and all administration was performed at room temperature.

Sampling time: for iv group, sampling was performed at 0.083, 0.25, 0.5, 1, 2, 4, 8 and 24 hours after injection administration (iv); for po group, sample was performed at 0.25, 0.5, 1, 2, 4, 8 and 24 hours after oral administration (po).

Sampling method:

① At each time point, about 0.03 mL of blood was collected. The blood of each sample was transferred to a plastic microcentrifuge tube containing heparin sodium anticoagulant, mixed well with the anticoagulant, and then cooled on ice and centrifuged.

② The blood sample was centrifuged at 4000 rpm for 5 minutes at 4°C to obtain plasma.

③ The samples was stored in a refrigerator at -75±15°C for later test.

Analysis and identification:
The mixed solution of acetonitrile and water (1:1) was used to dilute the stock solution to prepare a series of working solutions.

3 μL of working solution (5, 10, 20, 50, 100, 500, 1000, 5000, 10000 ng/mL) was added to 30 μL of C57 mouse blank plasma to obtain a standard solution with a concentration of 0.5-1000 ng/mL (0.5, 1, 2, 5, 10, 50, 100, 500, 1000 ng/mL), in total of 33 μL. Four quality control samples of 1 ng/mL, 2 ng/mL, 50 ng/mL and 800 ng/mL were used to calibrate the standard curve.

② The quality control samples were prepared on the day of analysis, and the method was the same as the standard solution.

③ 200 μL of acetonitrile containing internal standard solution was added to 33 μL of standard samples, 33 μL of quality control samples and 33 μL of unknown sample (30 μL of plasma and 3 μL of blank solution) to precipitate protein, respectively. The samples were mixed by a vortex mixer for 30 seconds and mixed well. The precipitated samples were centrifuged for 15 minutes at 4°C and 4000 rpm.

④ The supernatant was pipetted quantitatively and diluted by 3 times with water, then the diluted supernatant was loaded and quantitatively analyzed by liquid chromatography-mass spectrometry technology.

⑤ Detection data were obtained, and pharmacokinetic parameters such as $T_{1/2}$, Cmax, AUC, AUC, CL, Vss, F were calculated.

Through experiments, the drug metabolism data of 5 compounds of the present application were obtained, and the specific information was shown in Table 5:

Table 5: In vivo drug metabolism data of the compounds of the present application

| Compd. | Co(ng/mL) iv/$C_{max}$ (ng/mL) po | $T_{max}$ (h) po | AUC (h.ng/mL) iv/po | $T_{1/2}$ (h) iv/po | CL ((mL/min) /kg) iv | Vss (L/kg) iv | F (%) po |
|---|---|---|---|---|---|---|---|
| HTL-6-45 | 398/42.9 | 0.25 | 95/3.54 | 0.324/0.84 | 176 | 3.25 | 3.89 |
| HTL-6-48 | 651/1290 | 0.25 | 651/133 | 0.840/0.99 | 25.6 | 1.59 | 20.2 |
| HTL-7-01 | 574/298 | 0.5 | 592/69.9 | 1.05/2.01 | 27.8 | 2.29 | 12.2 |
| HTL-7-03 | 596/565 | 0.667 | 668/164 | 1.83/1.24 | 24.1 | 3.24 | 24.0 |
| HTL-7-17 | 364/144 | 0.333 | 168/35.7 | 0.718/1.58 | 99 | 3.59 | 21.8 |

Through the investigation of several drug metabolism properties, it was found that on the highest blood concentration $C_0/C_{max}$ index, the oral $C_{max}$ of compound HTL-6-48 was relatively high, while the $C_0/C_{max}$ values of several other compounds were relatively low. Comparing the peak time $T_{max}$, HTL-6-45 and HTL-6-48 both reached the highest plasma concentration quickly in 0.25 hours and were absorbed relatively quickly, while the $T_{max}$ of HTL-7-17 was slightly lower, being 0.33 hours; the $T_{max}$ values of HTL-7-01 and HTL-7-03 were both above 0.5 hours. In terms of area under curve (AUC), the AUC values of HTL-6-48, HTL-7-01 and HTL-7-03 were relatively high through injection administration route, but the AUC of HTL-7-01 through oral administration was lower. In terms of half-life $T_{1/2}$, except for HTL-7-03 that had relatively longer half-life through injection administration, all other compounds had relatively lower $T_{1/2}$. In terms of clearance rate (CL), except for HTL-6-45 and HTL-7-17 that had relatively higher clearance rate, the other three compounds had relatively lower clearance rates, indicating that they were less liable to be cleared in the body. Comparing the apparent volume of distribution (Vss), the Vss values of HTL-6-48 and HTL-7-01 were lower, indicating that they were less liable to tend to tissue distribution, while the Vss values of HTL-6-45, HTL-7-03 and HTL-7-17 were slightly higher, indicating that they were more liable to tend to tissue distribute. As a therapeutic drug of anti-enterovirus EV71, oral bioavailability (F) was an important drug metabolism index that was focused in this text. Generally speaking, a compound having a bioavailability greater than 20% indicates it had a certain potential to be developed as drugs . Among the experimental data, the F values of HTL-6-48, HTL-7-03 and HTL-7-17 were all greater than 20%, indicating that they were candidate compounds that could possibility be further developed into drugs.

Example 103: Water solubility experiment of the compounds of the application

Water solubility is important physical and chemical property that affects the oral absorption of drugs. Therefore, we selected five compounds HTL-6-45, HTL-6-48, HTL-7-01, HTL-7-03 and HTL-7-17 which had certain potential to be developed as drugs among the compounds of the present application and tested their water solubility.
Experimental method:
By measuring the solubility of compound in a saturated aqueous solution, the water solubility of the compound was further investigated.
Preparation of supersaturated solution:

① About 1 mg of the compound to be tested was weighed and added to a 25 mL tube;

② 5 mL of deionized water was added, shaken well, placed in a vortex shaker and mixed for 30 seconds, then treated ultrasonically for 30 seconds;

③ The tube was placed in a constant temperature water bath, and allowed to stand in a constant temperature water bath at 25°C for 1 hour;

④ The operation of ② was repeated, and the standing at constant temperature of 25°C was continued for 1 hour;

⑤ The prepared supersaturated solution supernatant was taken out and placed in a centrifuge tube, centrifuged at 15000 rpm for 5 minutes, and the upper centrifugate was taken out for later use.

Liquid chromatography method:

① Chromatographic column: Agilent XDB Eclipse C18, 25cm×4.6mm×5μm liquid chromatography column.

② Mobile phase: Phase A: water (0.1% trifluoroacetic acid); Phase B: acetonitrile.

③ Flow rate: 1.0 mL/min.

④ Sampling volume: 10 μl.

Preparation of standard solution: 1.0 mg of the sample to be tested was accurately weighed, added to a 10 mL volumetric flask, dissolved in methanol as solvent, metered to a constant volume of 10 mL, the concentration of solution was calculated, and it was used as a standard solution for later use.

By using the one-point method, the chromatographic peak area of the standard solution of the sample to be tested was determined, and then the peak area of the supersaturated compound was determined, and the solubility of the compound to be tested in water was calculated accordingly.

Through experiments, the solubility values of 5 compounds of the present invention in water were measured, and the results were shown in Table 6:

Table 6: Water solubility of the compounds of the present application

| Compound name | HTL-6-45 | HTL-6-48 | HTL-7-01 | HTL-7-03 | HTL-7-17 |
|---|---|---|---|---|---|
| Water solubility (μg/mL) | 0.39 | 5.88 | 0.42 | 0.61 | 18.64 |

The water solubility test results showed that among the 5 compounds in the present application, HTL-6-48 and HTL-7-17 had relatively high water solubility and were compounds with high biological activity, and good drug metabolism and water solubility, and had the potential to be further developed as drugs.

## Claims

1. A compound represented by Formula I, a pharmaceutically acceptable salt, a stereoisomer, a hydrate or a solvate thereof,

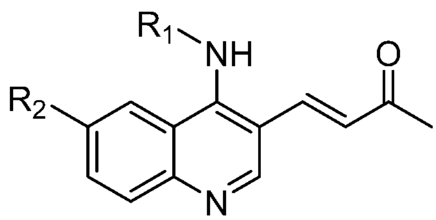

Formula I

wherein:

$R_1$ is $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3-to 14-membered substituted or unsubstituted heterocyclyl;
$R_2$ is hydrogen, or $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 7- to 12-membered substituted or unsubstituted bridged ring group, amino, 6- to 14-membered substituted or unsubstituted arylimino.

2. A compound represented by Formula II, a pharmaceutically acceptable salt, a stereoisomer, a hydrate or a solvate thereof,

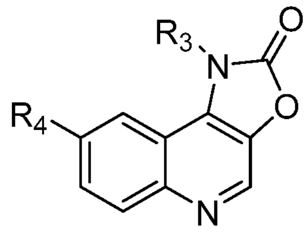

Formula II

wherein:

$R_3$ is $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3-to 14-membered substituted or unsubstituted heterocyclyl;

$R_4$ is hydrogen, or $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 7- to 12-membered substituted or unsubstituted bridged ring group, amino, 6- to 14-membered substituted or unsubstituted arylimino.

**3.** A compound represented by the general Formula III, a pharmaceutically acceptable salt, a stereoisomer, a hydrate or a solvate thereof,

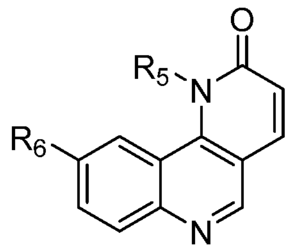

Formula III

wherein:

$R_5$ is $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3-to 14-membered substituted or unsubstituted heterocyclyl;

$R_6$ is hydrogen, or $C_{1-10}$ alkyl, 3- to 14-membered cycloalkyl, $C_{2-12}$ alkenyl or polyenyl, $C_{2-12}$ alkenoyl or polyenoyl, 2- to 10-membered alkanoyl, 6- to 14-membered substituted or unsubstituted aryl, 3- to 14-membered substituted or unsubstituted heterocyclyl, 7- to 12-membered substituted or unsubstituted bridged ring group, amino, 6- to 14-membered substituted or unsubstituted arylimino.

**4.** The compound, a pharmaceutically acceptable salt, a stereoisomer, a hydrate or a solvate thereof according to any one of claims 1 to 3, wherein:

$R_1$ is 5- to 6-membered cycloalkyl, 5- to 6-membered heterocyclyl, 5- to 6-membered aryl or 5- to 6-membered heteroaryl, $R_1$ can be optionally substituted by one or more $R^a$, each $R^a$ is independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen or amino;

preferably, $R_1$ is phenyl, $R_1$ can be optionally substituted by one or more $R^a$, each $R^a$ is independently hydrogen, trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino;

preferably, $R_3$ is 5- to 6-membered cycloalkyl, 5- to 6-membered heterocyclyl, 5- to 6-membered aryl or 5- to

6-membered heteroaryl, $R_3$ can be optionally substituted by one or more $R^b$, each $R^b$ is independently hydrogen, $C_{1-6}$ alkyl, $C_{1-6}$ haloalkyl, $C_{1-6}$ alkoxy, alkyl-amino (e.g., $C_{1-6}$ alkyl-amino-), hydroxy, nitro, cyano, $C_{1-6}$ alkylthio, dialkyl-amino (e.g., di($C_{1-6}$ alkyl)-amino-), halogen or amino;

preferably, $R_3$ is phenyl, $R_3$ can be optionally substituted with one or more $R^b$, and each $R^b$ is independently hydrogen, trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino;

preferably, $R_5$ is 5- to 6-membered cycloalkyl, 5- to 6-membered heterocyclyl, 5- to 6-membered aryl or 5- to 6-membered heteroaryl, and $R_5$ can be optionally substituted by one or more $R^c$, each $R^c$ is independently $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen or amino;

preferably, $R_5$ is phenyl, $R_5$ can be optionally substituted by one or more $R^c$, each $R^c$ is independently trifluoromethyl, methyl, ethyl, n-propyl, isopropyl, n-butyl, methoxy, ethoxy, propoxy, methylamino, ethylamino, dimethylamino, diethylamino, hydroxyl, nitro, cyano, methylthio, ethylthio, fluorine, chlorine, bromine, iodine, or amino.

**5.** The compound, a pharmaceutically acceptable salt, a stereoisomer, a hydrate or a solvate thereof according to any one of claims 1 to 3, wherein:

preferably, $R_2$ is pyridyl, phenyl, furyl, pyrazolyl, thienyl, quinolyl, $R_2$ is optionally substituted by one or more $R^d$, and each $R^d$ is independently $C_{1-6}$ alkyl, $C_{1-6}$ haloalkyl, $C_{1-6}$ alkoxy, alkyl-amino (e.g., $C_{1-6}$ alkyl-amino), hydroxyl, nitro, cyano, $C_{1-6}$ alkylthio, dialkyl-amino (e.g., di($C_{1-6}$ alkyl)-amino), halogen, amino, $NH_2C(O)$-, R'OC(O)NH-, wherein R' is benzyl, phenyl or $C_{1-6}$ alkyl;

preferably, $R_4$ is pyridyl, phenyl, furyl, pyrazolyl, thienyl, quinolyl, vinyl, propenyl, or butenyl, $R_4$ is optionally substituted by one or more $R^e$, each $R^e$ is independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxy, nitro, cyano, $C_{1-6}$ alkylthio, dialkyl-amino (e.g., di($C_{1-6}$ alkyl)-amino), halogen, amino, $NH_2C(O)$-, $NH_2C(O)NH$-, R'OC(O)- (wherein R' is benzyl, phenyl, or $C_{1-6}$ alkyl), R"OC(O)NH- (wherein R" is benzyl, phenyl, or $C_{1-6}$ alkyl), R'"C(O)NH- (wherein R'" is benzyl, phenyl, or $C_{1-6}$ alkyl);

preferably, $R_6$ is

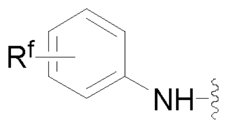,
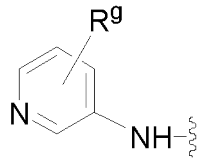,
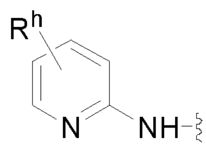,

or

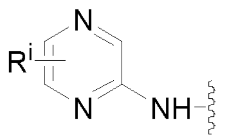,

wherein $R^f$, $R^g$, $R^h$, $R^i$ are each independently hydrogen, $C_{1-6}$ alkyl, $C_{1-6}$ haloalkyl, $C_{1-6}$ alkoxy, alkyl-amino (e.g., $C_{1-6}$ alkyl-amino), hydroxyl, nitro, cyano, $C_{1-6}$ alkylthio, dialkyl-amino (e.g., di($C_{1-6}$ alkyl)-amino), halogen, amino, $C_{1-6}$ alkyl-C(O)NH-; or

$R_6$ is pyridyl, phenyl, quinolyl, 2-oxoindolyl, pyrimidyl, isoxazolyl, 1,4-dioxaspiro[4.5]dec-7-ene group, or pyrazolyl, $R_6$ is optionally substituted by one or more $R^j$, each $R^j$ is independently hydrogen, $C_{1-6}$ alkyl, $C_{1-6}$ alkoxy, halogen, alkyl-amino (e.g., $C_{1-6}$ alkyl-amino), dialkyl-amino (e.g., di($C_{1-6}$ alkyl)-amino), 4-methyl-piperazinyl, morpholinyl, amino, $R^k$-C(O)NH- (wherein $R^k$- is benzyl, phenyl, p-methoxybenzyl, phenoxy or $C_{1-6}$ alkyl), pyrrolidinyl, allylamino or propargylamino; or

$R_6$ is $C_{2-6}$ alkenyl, $R_6$ is optionally substituted by one or more $R^m$, each $R^m$ is independently $NH_2C(O)$-, $NH_2C(O)NH$-, $R^n$-OC(O)- (wherein $R^n$ is $C_{1-6}$ alkyl).

**6.** The compound represented by Formula I, a pharmaceutically acceptable salt, a stereoisomer, a hydrate or a solvate thereof according to claim 1, wherein

preferably, $R_1$ is

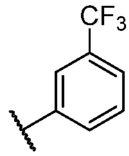;

preferably, $R_2$ is

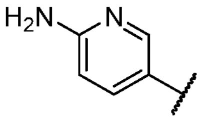, 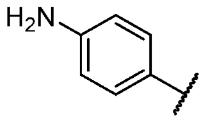, 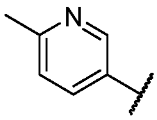, 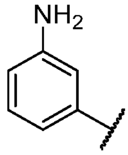, 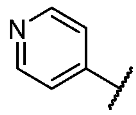,

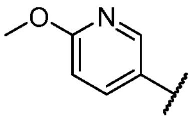, 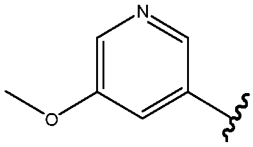 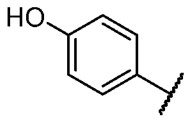, 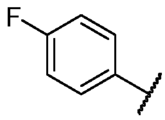, 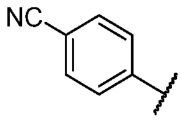,

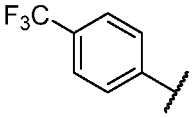, 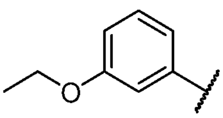, 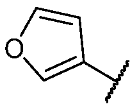, 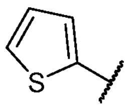, 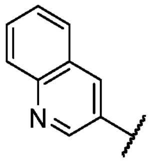, 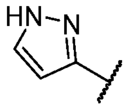, 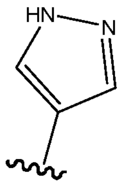,

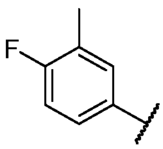, 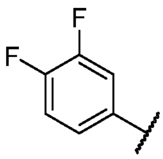, 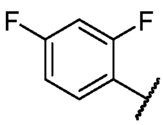, 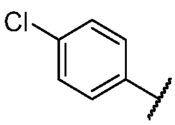, 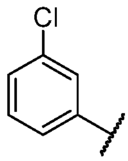, 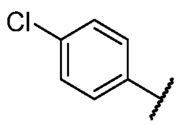,

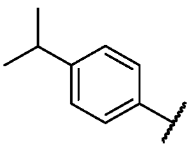, 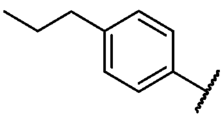, 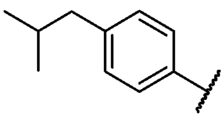, 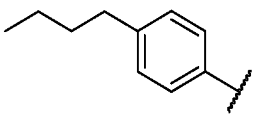, 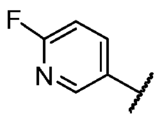,

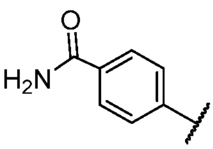, 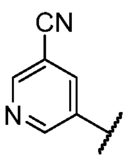, or 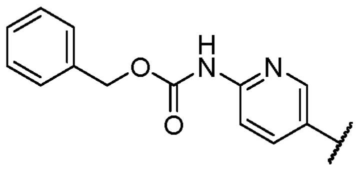.

**7.** The compound represented by Formula II, a pharmaceutically acceptable salt, a stereoisomer, a hydrate or a solvate thereof according to claim 2, wherein

preferably, $R_3$ is

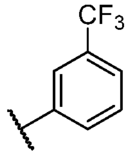;

preferably, $R_4$ is

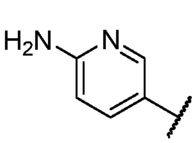, 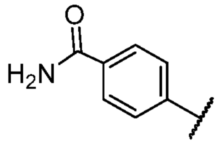, 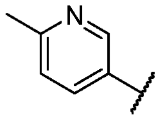, 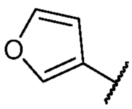, 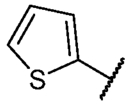,

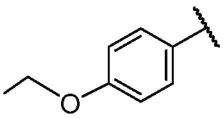, 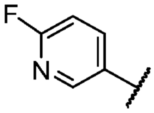, 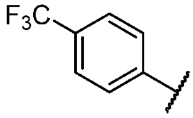, 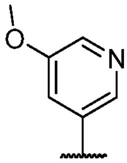, 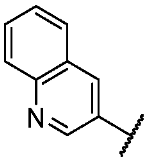, 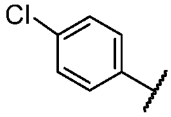,

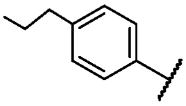, 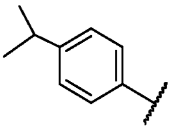, 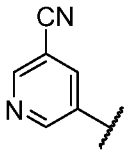, 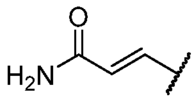, 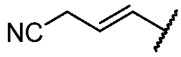, 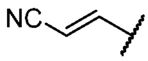,

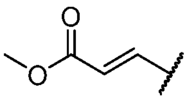, 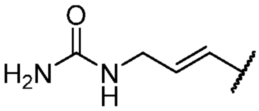, 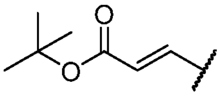, 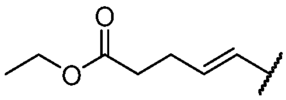,

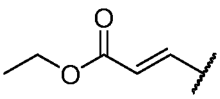, 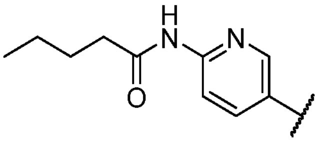, or 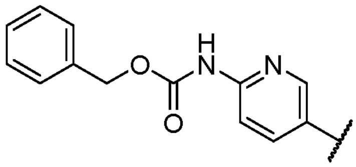.

**8.** The compound represented by Formula III, a pharmaceutically acceptable salt, a stereoisomer, a hydrate or a solvate thereof according to claim 3, wherein

preferably, $R_5$ is

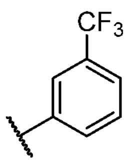;

preferably, $R_6$ is

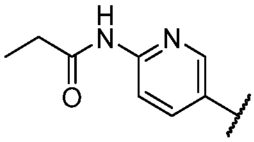, 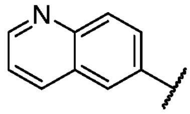, 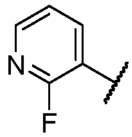, 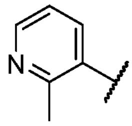, 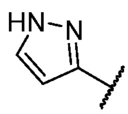,

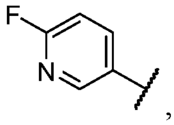, 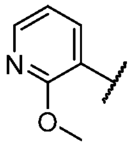, 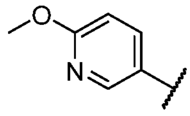, 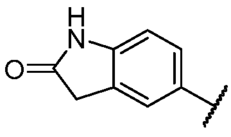, 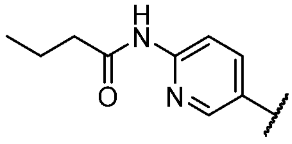,

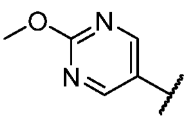, 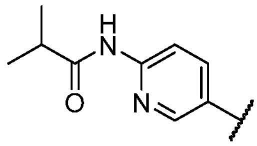, 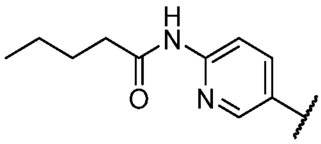, 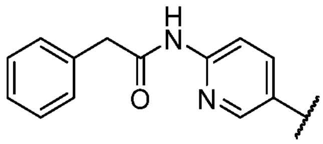,

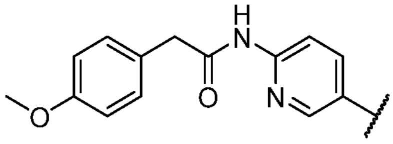, 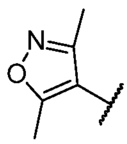, 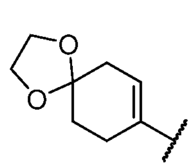, 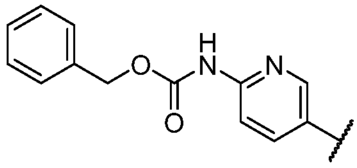,

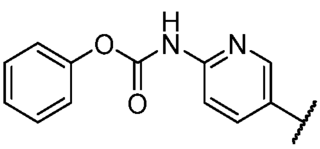, 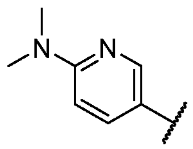, 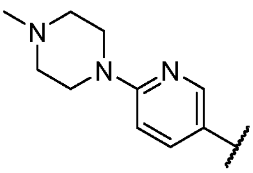, 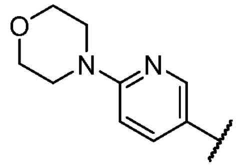,

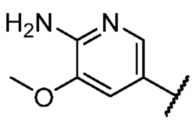, 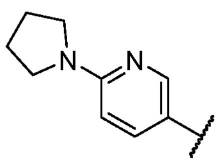, 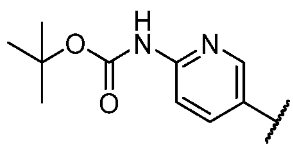, 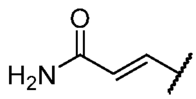,

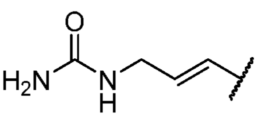, 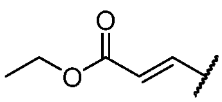, 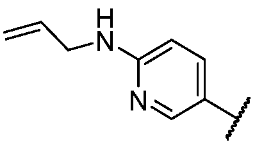, 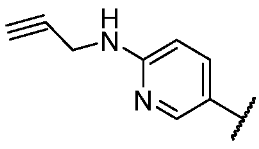,

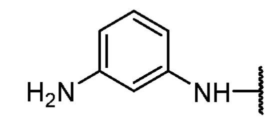, 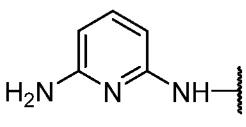, 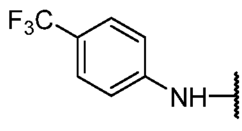, 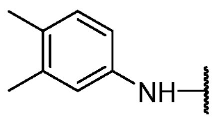,

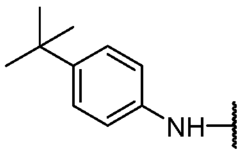, 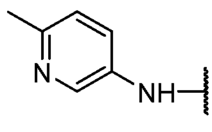, 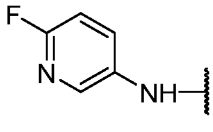, 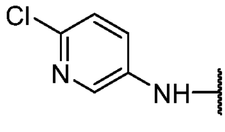, 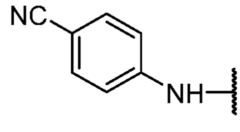,

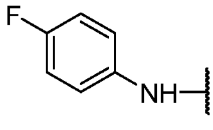, 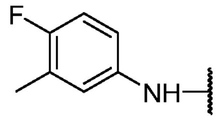, 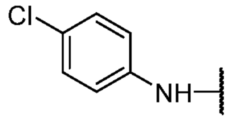, 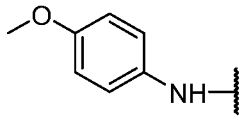,

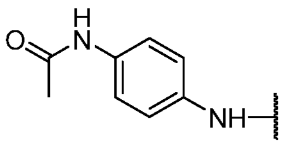, 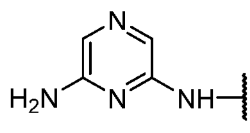, or 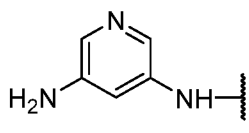.

**9.** The compound represented by Formula I, Formula II and Formula III, a pharmaceutically acceptable salt, a stereoisomer, a hydrate or a solvate thereof according to claim 1, 2 and 3, wherein the compound is preferably selected from the group consisting of:

(*E*)-6-[3-(6-amino)pyridyl]-4-[3-(trifluoromethyl)phenyl]aminoquinoline-3-butenone (HTL-2-34);
(*E*)-6-(4-amino)phenyl-4-[3-(trifluoromethyl)phenyl]aminoquinoline-3-butenone (HTL-2-35);
(*E*)-6-[3-(6-methyl)pyridyl]-4-[3-(trifluoromethyl)phenyl]aminoquinoline-3-butenone (HTL-2-38);
(*E*)-6-(3-aminophenyl)-4-[3-(trifluoromethyl)phenyl]aminoquinoline-3-butenone (HTL-2-42);
(*E*)-6-(4-pyridyl)-4-[3-(trifluoromethyl)phenyl]aminoquinoline-3-butenone (HTL-3-04);
(*E*)-6-(5-methoxy)pyridyl-4-[3-(trifluoromethyl)phenyl]aminoquinoline-3-butenone (HTL-3-07);
(*E*)-6-(4-hydroxy)phenyl-4-[3-(trifluoromethyl)phenyl]aminoquinoline-3-butenone (HTL-3-11);
(*E*)-6-(4-fluoro)phenyl-4-[3-(trifluoromethyl)phenyl]aminoquinoline-3-butenone (HTL-3-12);
(*E*)-6-(4-cyano)phenyl-4-[3-(trifluoromethyl)phenyl]aminoquinoline-3-butenone (HTL-3-15);
(*E*)-6-[4-(trifluoromethyl)phenyl]-4-[3-(trifluoromethyl)phenyl]aminoquinoline-3-butenone (HTL-3-18);
(*E*)-6-(3-ethoxy)phenyl-4-[3-(trifluoromethyl)phenyl]aminoquinoline-3-butenone (HTL-3-16);
(*E*)-6-(3-furyl)-4-[3-(trifluoromethyl)phenyl]aminoquinoline-3-butenone (HTL-3-22);
(*E*)-6-(2-thienyl)-4-[3-(trifluoromethyl)phenyl]aminoquinoline-3-butenone (HTL-3-24);
(*E*)-6-(3-quinolyl)-4-[3-(trifluoromethyl)phenyl]aminoquinoline-3-butenone (HTL-3-25);
(*E*)-6-(1H-4-pyrazolyl)-4-[3-(trifluoromethyl)phenyl]aminoquinoline-3-butenone (HTL-3-26);
(*E*)-6-(4-fluoro-3-methyl)phenyl-4-[3-(trifluoromethyl)phenyl]aminoquinoline-3-butenone (HTL-3-32);
(*E*)-6-(2,4-difluoro)phenyl-4-[3-(trifluoromethyl)phenyl]aminoquinoline-3-butenone (HTL-3-33);
(*E*)-6-(3,4-difluoro)phenyl-4-[3-(trifluoromethyl)phenyl]aminoquinoline-3-butenone (HTL-3-34);
(*E*)-6-(3-chloro)phenyl-4-[3-(trifluoromethyl)phenyl]aminoquinoline-3-butenone (HTL-3-36);
(*E*)-6-(4-chloro)phenyl-4-[3-(trifluoromethyl)phenyl]aminoquinoline-3-butenone (HTL-3-37);
(*E*)-6-(4-isopropyl)phenyl-4-[3-(trifluoromethyl)phenyl]aminoquinoline-3-butenone (HTL-3-39);
(*E*)-6-(4-propyl)phenyl-4-[3-(trifluoromethyl)phenyl]aminoquinoline-3-butenone (HTL-3-40);
(*E*)-6-(4-isobutyl)phenyl-4-[3-(trifluoromethyl)phenyl]aminoquinoline-3-butenone (HTL-3-41);
(*E*)-6-(4-butyl)phenyl-4-[3-(trifluoromethyl)phenyl]aminoquinoline-3-butenone (HTL-3-42);
(*E*)-6-[3-(6-fluoro)pyridyl]-4-[3-(tiifluoromethyl)phenyl]aminoquinoline-3-butenone (HTL-3-43);
(*E*)-6-(4-carbamoyl)phenyl-4-[3-(trifluoromethyl)phenyl]aminoquinoline-3-butenone (HTL-3-45);
(*E*)-6-[3-(5-cyano)pyridyl]-4-[3-(trifluoromethyl)phenyl]aminoquinoline-3-butenone (HTL-3-46);
(*E*)-6-[3-(N-6-benzyloxyamido)pyridyl]-4-[3-(trifluoromethyl)phenyl]aminoquinoline-3-butenone (HTL-7-22);
8-[3-(6-amino)pyridyl]-1-[3-(trifluoromethyl)phenyl]oxazolo[5,4-*c*]quinolin-2(1*H*)-one (HTL-4-32);
8-(4-carbamoyl)phenyl-1-[3-(trifluoromethyl)phenyl]oxazolo[5,4-*c*]quinolin-2(1*H*)-one (HTL-5-21);
8-[3-(6-methyl)pyridyl]-1-[3-(trifluoromethyl)phenyl]oxazolo[5,4-*c*]quinolin-2(1*H*)-one (HTL-5-23);
8-(3-furyl)-1-[3-(trifluoromethyl)phenyl]oxazolo[5,4-*c*]quinolin-2(1*H*)-one (HTL-5-25);
8-(2-thienyl)-1-[3-(trifluoromethyl)phenyl]oxazolo[5,4-*c*]quinolin-2(1*H*)-one (HTL-5-26);
8-(4-ethoxy)phenyl-1-[3-(trifluoromethyl)phenyl]oxazolo[5,4-*c*]quinolin-2(1*H*)-one (HTL-5-27);
8-[3-(6-fluoro)pyridyl]-1-[3-(trifluoromethyl)phenyl]oxazolo[5,4-*c*]quinolin-2(1*H*)-one (HTL-5-28);

8-(4-trifluoromethyl)phenyl-1-[3-(trifluoromethyl)phenyl]oxazolo[5,4-*c*]quinolin-2(1*H*)-one (HTL-5-29);
8-[3-(5-methoxy)pyridyl]-1-[3-(trifluoromethyl)phenyl]oxazolo[5,4-*c*]quinolin-2(1*H*)-one (HTL-5-30);
8-(3-quinolyl)-1-[3-(trifluoromethyl)phenyl]oxazolo[5,4-*c*]quinolin-2(1*H*)-one (HTL-5-32);
8-(4-chloro)phenyl-1-[3-(trifluoromethyl)phenyl]oxazolo[5,4-*c*]quinolin-2(1*H*)-one (HTL-5-33);
8-(4-propyl)phenyl-1-[3-(trifluoromethyl)phenyl]oxazolo[5,4-*c*]quinolin-2(1*H*)-one (HTL-5-34);
8-(4-isopropyl)phenyl-1-[3-(trifluoromethyl)phenyl]oxazolo[5,4-*c*]quinolin-2(1*H*)-one (HTL-5-35);
8-[3-(5-cyano)pyridyl]-1-[3-(trifluoromethyl)phenyl]oxazolo[5,4-*c*]quinolin-2(1*H*)-one (HTL-5-36);
8-[3-(-6-valerylamino)pyridyl]-1-[3-(trifluoromethyl)phenyl]oxazolo[5,4-*c*]quinolin-2(1*H*)-one (WSX-1-24);
8-[3-(-6-benzyloxyamido)pyridyl]-1-[3-(trifluoromethyl)phenyl]oxazolo[5,4-*c*]quinolin-2(1*H*)-one (HTL-7-23);
(*E*)-8-(2-carbamoyl-vinyl)-1-[3-(trifluoromethyl)phenyl]oxazolo[5,4-*c*]quinolin-2(1*H*)-one (HTL-6-30);
(*E*)-8-(3-cyanopropenyl)-1-[3-(trifluoromethyl)phenyl]oxazolo[5,4-*c*]quinolin-2(1*H*)-one (HTL -6-31);
(*E*)-8-(2-cyanovinyl)-1-[3-(trifluoromethyl)phenyl]oxazolo[5,4-*c*]quinolin-2(1*H*)-one (HTL-6-32);
(*E*)-8-(2-methoxycarbonyl-vinyl)-1-[3-(trifluoromethyl)phenyl]oxazolo[5,4-*c*]quinolin-2(1*H*)-one (HTL-6-33);
(*E*)-8-(3-ureido-propenyl)-1-[3-(trifluoromethyl)phenyl]oxazolo[5,4-*c*]quinolin-2(1*H*)-one (HTL-6-34);
(*E*)-8-(2-tert-butoxycarbonyl-vinyl)-1-[3-(trifluoromethyl)phenyl]oxazolo[5,4-*c*]quinolin-2(1*H*)-one (HTL-6-35);
(*E*)-8-(4-ethoxycarbonyl-but-1-enyl)-1-[3-(trifluoromethyl)phenyl]oxazolo[5,4-*c*]quinolin-2(1*H*)-one (HTL-6-38);
(*E*)-8-(2-ethoxycarbonyl-vinyl)-1-[3-(trifluoromethyl)phenyl]oxazolo[5,4-*c*]quinolin-2(1*H*)-one (WSX-1-13);
9-[3-(6-propionylamino)pyridyl]-2-oxo-1-[3-(trifluoromethyl)phenyl]-1,2-dihydrobenzo[*h*][1,6]naphthyridine (HTL-6-45);
9-(6-quinolyl)-2-oxo-1-[3-(trifluoromethyl)phenyl]-1,2-dihydrobenzo[*h*][1,6]naphthyridine (HTL-6-47);
9-[3-(2-fluoro)pyridyl]-2-oxo-1-[3-(trifluoromethyl)phenyl]-1,2-dihydrobenzo[*h*][1,6]naphthyridine (HTL-6-48);
9-[3-(2-methyl)pyridyl]-2-oxo-1-[3-(trifluoromethyl)phenyl]-1,2-dihydrobenzo[*h*][1,6]naphthyridine (HTL-6-49);
9-[(1*H*)-3-pyrazolyl]-2-oxo-1-[3-(trifluoromethyl)phenyl]-1,2-dihydrobenzo[*h*][1,6]naphthyridine (HTL-6-50);
9-[3-(6-fluoro)pyridyl]-2-oxo-1-[3-(trifluoromethyl)phenyl]-1,2-dihydrobenzo[*h*][1,6]naphthyridine (HTL-7-01);
9-[3-(2-methoxy)pyridyl]-2-oxo-1-[3-(trifluoromethyl)phenyl]-1,2-dihydrobenzo[*h*][1,6]naphthyridine (HTL-7-02);
9-[3-(6-methoxy)pyridyl]-2-oxo-1-[3-(trifluoromethyl)phenyl]-1,2-dihydrobenzo[*h*][1,6]naphthyridine (HTL-7-03);
9-[5-(2-oxo)indolyl]-2-oxo-1-[3-(trifluoromethyl)phenyl]-1,2-dihydrobenzo[*h*][1,6]naphthyridine (HTL-7-04)
9-[3-(6-butyrylamino)pyridyl]-2-oxo-1-[3-(trifluoromethyl)phenyl]-1,2-dihydrobenzo[*h*][1,6]naphthyridine (HTL-7-05);
9-[5-(2-methoxy)pyrimidyl]-2-oxo-1-[3-(trifluoromethyl)phenyl]-1,2-dihydrobenzo[*h*][1,6]naphthyridine (HTL-7-06);
9-[3-(2-isobutyrylamino)pyridyl]-2-oxo-1-[3-(trifluoromethyl)phenyl]-1,2-dihydrobenzo[*h*][1,6]naphthyridine (HTL-7-07);
9-[3-(6-valerylamino)pyridyl]-2-oxo-1-[3-(trifluoromethyl)phenyl]-1,2-dihydrobenzo[*h*][1,6]naphthyridine (HTL-7-08);
9-[3-(6-phenylacetamide)pyridyl]-2-oxo-1-[3-(trifluoromethyl)phenyl]-1,2-dihydrobenzo[*h*][1,6]naphthyridine (HTL-7-10);
9-{3-[6-(4-methoxy)phenylacetylamino]pyridyl}-2-oxo-1-[3-(trifluoromethyl)phenyl]-1,2-dihydrobenzo[*h*][1,6]naphthyridine (HTL-7-1 1);
9-[4-(3,5-dimethyl)isoxazolyl]-2-oxo-1-[3-(trifluoromethyl)phenyl]-1,2-dihydrobenzo[*h*][1,6]naphthyridine (HTL-7-12);
9-(1,4-dioxa-spiro[4.5]dec-7-en-8-yl)-2-oxo-1-[3-(trifluoromethyl)phenyl]-1,2-dihydrobenzo[*h*][1,6]naphthyridine (HTL-7-13);
9-[3-(6-benzyloxyamido)pyridyl]-2-oxo-1-[3-(trifluoromethyl)phenyl]-1,2-dihydrobenzo[*h*][1,6]naphthyridine (HTL-7-14);
9-[3-(-6-phenoxyamido)pyridyl]-2-oxo-1-[3-(trifluoromethyl)phenyl]-1,2-dihydrobenzo[*h*][1,6]naphthyridine(HTL-7-15);
9-[3-(6-N,N-dimethyl)pyridyl]-2-oxo-1-[3-(trifluoromethyl)phenyl]-1,2-dihydrobenzo[h][1,6]naphthyridine (HTL-7-16);
9-{3-[6-(4-methylpiperazin-1-yl)pyridyl]}-2-oxo-1-[3-(trifluoromethyl)phenyl]-1,2-dihydrobenzo[*h*][1,6]naphthyridine (HTL-7-17);
9-[3-(6-morpholin-4-yl)pyridyl]-2-oxo-1-[3-(trifluoromethyl)phenyl]-1,2-dihydrobenzo[*h*] [1,6]naphthyridine (HTL-7-18);
9-[3-(6-amino-5-methoxy)pyridyl]-2-oxo-1-[3-(trifluoromethyl)phenyl]-1,2-dihydrobenzo[h ][1,6]naphthyridine (HTL-7-19);
9-[3-(6-pyrrolidinyl)pyridyl]-2-oxo-1-[3-(trifluoromethyl)phenyl]-1,2-dihydrobenzo[*h*][1,6]naphthyridine (HTL-7-20);
9-[3-(6-tert-butoxycarbonylamino)pyridyl]-2-oxo-1-[3-(trifluoromethyl)phenyl]-1,2-dihydrobenzo[*h*][1,6]naphthyridine (HTL-7-21);
9-[3-(N-6-allylamino)pyridyl]-2-oxo-1-[3-(trifluoromethyl)phenyl]-1,2-dihydrobenzo[*h*][1,6]naphthyridine (HTL-7-28);
9-[3-(6-propargylamino)pyridyl]-2-oxo-1-[3-(trifluoromethyl)phenyl]-1,2-dihydrobenzo[*h*][1,6]naphthyridine (HTL-7-29);
9-(3-aminophenyl)imino-2-oxo-1-[3-(trifluoromethyl)phenyl]-1,2-dihydrobenzo[*h*][1,6]naphthyridine (HTL-6-11);
9-[2-(6-aminopyridyl)]imino-2-oxo-1-[3-(trifluoromethyl)phenyl]-1,2-dihydrobenzo[*h*][1,6]naphthyridine (HTL-6-12);
9-[4-(trifluoromethyl)phenyl]imino-2-oxo-1-[3-(trifluoromethyl)phenyl]-1,2-dihydrobenzo[*h*][1,6]naphthyridine (HTL-6-16);
9-(3,4-dimethylphenyl)imino-2-oxo-1-[3-(trifluoromethyl)phenyl]-1,2-dihydrobenzo[*h*][1,6]naphthyridine (HTL-6-17);
9-(4-tert-butylphenyl)imino-2-oxo-1-[3-(trifluoromethyl)phenyl]-1,2-dihydrobenzo[*h*][1,6]naphthyridine (HTL-6-18);
9-[3-(6-methylpyridyl)]imino-2-oxo-1-[3-(trifluoromethyl)phenyl]-1,2-dihydrobenzo[*h*][1,6]naphthyridine (HTL-6-19);
9-[3-(6-fluoropyridyl)]imino-2-oxo-1-[3-(trifluoromethyl)phenyl]-1,2-dihydrobenzo[*h*][1,6]naphthyridine (HTL-6-20);
9-[3-(6-chloropyridyl)]imino-2-oxo-1-[3-(trifluoromethyl)phenyl]-1,2-dihydrobenzo[*h*][1,6]naphthyridine (HTL-6-21);
9-(4-cyanophenyl)imino-2-oxo-1-[3-(trifluoromethyl)phenyl]-1,2-dihydrobenzo[*h*][1,6]naphthyridine (HTL-6-22);
9-(4-fluorophenyl)imino-2-oxo-1-[3-(trifluoromethyl)phenyl]-1,2-dihydrobenzo[*h*][1,6]naphthyridine (HTL-6-23);
9-[(4-fluoro-3-methyl)phenyl]imino-2-oxo-1-[3-(trifluoromethyl)phenyl]-1,2-dihydrobenzo[*h*][1,6]naphthyridine (HTL-6-24);
9-(4-chlorophenyl)imino-2-oxo-1-[3-(trifluoromethyl)phenyl]-1,2-dihydrobenzo[*h*][1,6]naphthyridine (HTL-6-25);
9-(4-methoxyphenyl)imino-2-oxo-1-[3-(trifluoromethyl)phenyl]-1,2-dihydrobenzo[*h*][1,6]naphthyridine (HTL-6-26);
9-(N-4-acetylaminophenyl)imino-2-oxo-1-[3-(trifluoromethyl)phenyl]-1,2-dihydrobenzo[*h*][1,6]naphthyridine (HTL-6-27);
9-[2-(6-aminopyrazinyl)]imino-2-oxo-1-[3-(trifluoromethyl)phenyl]-1,2-dihydrobenzo[*h*][1,6]naphthyridine (HTL-6-28);
9-[3-(5-aminopyridyl)]imino-2-oxo-1-[3-(trifluoromethyl)phenyl]-1,2-dihydrobenzo[*h*][1,6]naphthyridine (HTL-6-29);
(*E*)-9-(2-carbamoyl-vinyl)-2-oxo-1-[3-(trifluoromethyl)phenyl]-1,2-dihydrobenzo[*h*][1,6]naphthyridine (HTL-7-24);
(*E*)-9-(3-ureido-propenyl)-2-oxo-1-[3-(trifluoromethyl)phenyl]-1,2-dihydrobenzo[*h*][1,6]naphthyridine (HTL-7-25); and
(*E*)-9-(2-ethoxycarbonyl-vinyl)-2-oxo-1-[3-(trifluoromethyl)phenyl]-1,2-dihydrobenzo[*h*][1,6]naphthyridine (HTL-7-26).

**10.** The compound represented by Formula I, Formula II and Formula III, a pharmaceutically acceptable salt, a stereoisomer, a hydrate or a solvate thereof according to any one of claims 1 to 9, wherein the pharmaceutically acceptable salt is preferably one of inorganic acid salt of the compound such as hydrochloride, sulfate, phosphate, or organic acid salt such as methanesulfonate, trifluoromethanesulfonate, acetate, trifluoroacetate, benzoate.

**11.** A pharmaceutical composition, comprising at least one compound represented by Formula I, Formula II or Formula III, a pharmaceutically acceptable salt, a stereoisomer, a hydrate or a solvate thereof according to any one of claims 1 to 9, and one or more pharmaceutically acceptable carriers or excipients.

**12.** Use of the compound represented by Formula I, Formula II or Formula III, a pharmaceutically acceptable salt, a stereoisomer, a hydrate or a solvate thereof according to any one of claims 1 to 7, or the pharmaceutical composition of the present application in the manufacture of a medicament for the treatment and/or prevention of a disease or condition associated with viral infection, or in the manufacture of a medicament for inhibiting an enterovirus (e.g.,

EV71) in a host cell (e.g., a cell of mammal);
preferably, the viral infection is an infection caused by an enterovirus (e.g., EV71), and preferably, the disease or condition associated with viral infection is hand-foot-and-mouth disease.

**13.** The compound represented by Formula I, Formula II or Formula III, a pharmaceutically acceptable salt, a stereoisomer, a hydrate or a solvate thereof according to any one of claims 1 to 7, or the pharmaceutical composition of the present application, for use in the treatment and/or prevention of a disease or condition associated with viral infections; preferably, the viral infection is an infection caused by an enterovirus (e.g., EV71); preferably, the disease or condition associated with viral infection is hand-foot-and-mouth disease.

**14.** The compound represented by Formula I, Formula II or Formula III, a pharmaceutically acceptable salt, a stereoisomer, a hydrate or a solvate thereof according to any one of claims 1 to 7, or the pharmaceutical composition of the present application, for use in inhibiting the replication of an enterovirus (e.g., EV71) in a host cell (e.g., a cell of mammal).

**15.** A method for the treatment and/or prevention of a disease or condition associated with viral infection, the method comprising administering to a subject in need a therapeutically and/or prophylactically effective amount of at least one compound represented by Formula I, Formula II or Formula III, a pharmaceutically acceptable salt, a stereoisomer, a hydrate or a solvate thereof according to any one of claims 1 to 7, or the pharmaceutical composition according to claim 11,
preferably, the viral infection is an infection caused by an enterovirus (e.g., EV71), and preferably, the disease or condition associated with viral infection is hand-foot-and-mouth disease.

**16.** A method for inhibiting the replication of an enterovirus (e.g., EV71) in a mammal in need, the method comprising administering to the mammal in need a therapeutically and/or prophylactically effective amount of at least one compound represented by Formula I, Formula II or Formula III, a pharmaceutically acceptable salt, a stereoisomer, a hydrate or a solvate thereof according to any one of claims 1 to 7, or the pharmaceutical composition according to claim 11.

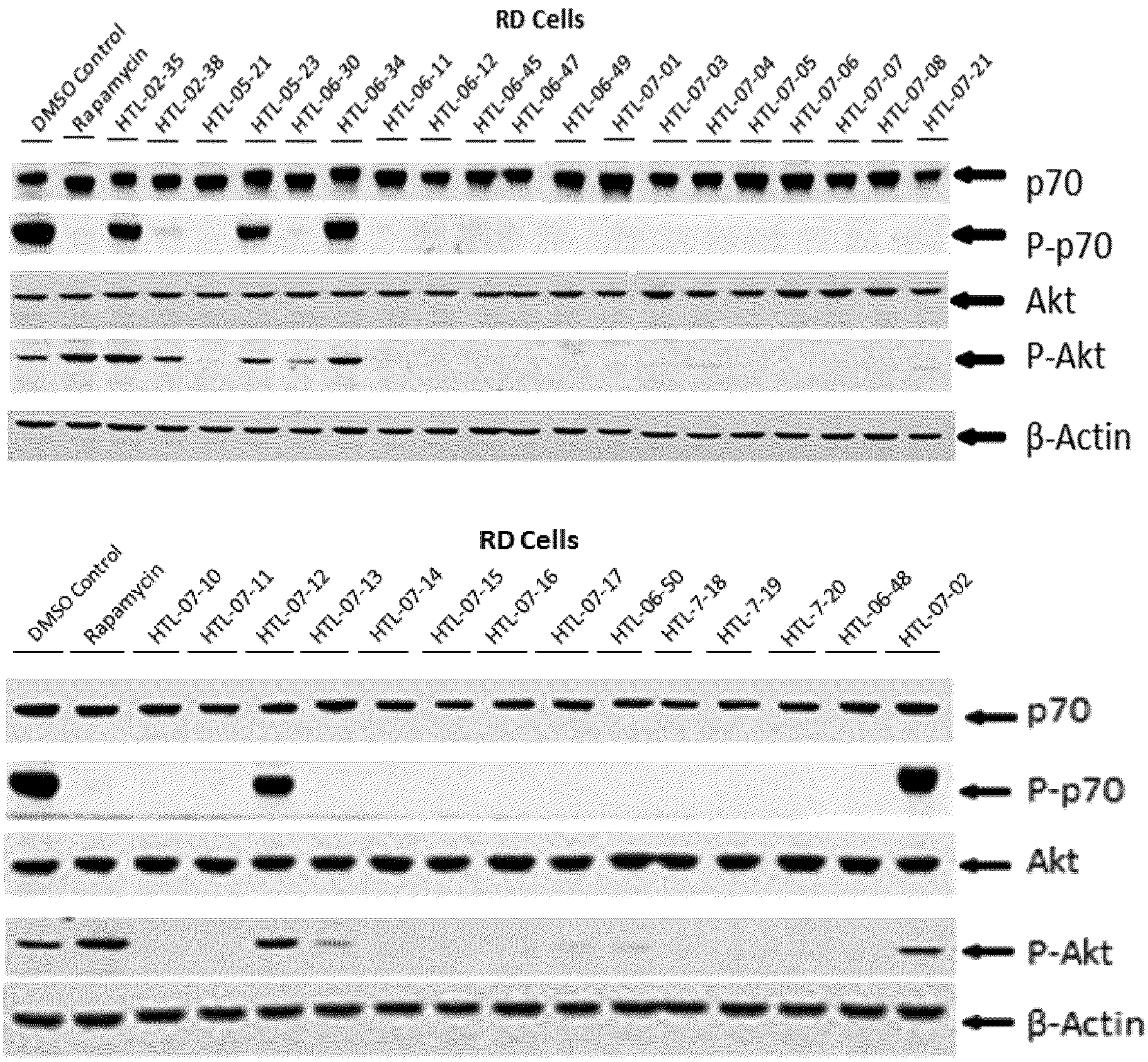
RD Cells
DMSO Control
Rapamycin
HTL-02-35
HTL-02-38
HTL-05-21
HTL-05-23
HTL-06-30
HTL-06-34
HTL-06-11
HTL-06-12
HTL-06-45
HTL-06-47
HTL-06-49
HTL-07-01
HTL-07-03
HTL-07-04
HTL-07-05
HTL-07-06
HTL-07-07
HTL-07-08
HTL-07-21
p70
P-p70
Akt
P-Akt
β-Actin
RD Cells
DMSO Control
Rapamycin
HTL-07-10
HTL-07-11
HTL-07-12
HTL-07-13
HTL-07-14
HTL-07-15
HTL-07-16
HTL-07-17
HTL-06-50
HTL-7-18
HTL-7-19
HTL-7-20
HTL-06-48
HTL-07-02
p70
P-p70
Akt
P-Akt
β-Actin

**Figure 1**

**INTERNATIONAL SEARCH REPORT**

International application No.
**PCT/CN2020/086837**

**A. CLASSIFICATION OF SUBJECT MATTER**

C07D 471/04(2006.01)i; C07D 498/04(2006.01)i; C07D 519/00(2006.01)i; A61K 31/47(2006.01)i; A61K 31/4725(2006.01)i; A61K 31/4745(2006.01)i; A61P 31/12(2006.01)i; A61P 1/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, WPI, EPODOC, CNKI, Caplus(STN), Registry(STN): 中国人民解放军军事科学院军事医学研究院, 钟武, 郝天龙, 曹瑞源, 程通, 李月香, 夏宁邵, 李松, 喹啉, 肠道, 病毒, quinoline, intestinal, virus, EV71, mTOR

**C. DOCUMENTS CONSIDERED TO BE RELEVANT**

| Category* | Citation of document, with indication, where appropriate, of the relevant passages | Relevant to claim No. |
|---|---|---|
| X | HAO, Tianlong et al. "Design, Synthesis and Pharmacological Evaluation of a Novel mTOR-Targeted Anti-EV71 Agent" *European Journal of Medicinal Chemistry,* Vol. 175, 24 April 2019 (2019-04-24), pp. 172-186 | 1-16 |
| X | CN 103044446 A (SHANGHAI HENGRUI PHARMACEUTICAL CO., LTD. et al.) 17 April 2013 (2013-04-17) description, embodiments 1, 4-6, 20, 21 and 23 | 2 |
| A | CN 108658974 A (OCEAN UNIVERSITY OF CHINA) 16 October 2018 (2018-10-16) entire document | 1-16 |
| A | CN 108658972 A (OCEAN UNIVERSITY OF CHINA) 16 October 2018 (2018-10-16) entire document | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 July 2020** | **22 July 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2020/086837**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-16**
because they relate to subject matter not required to be searched by this Authority, namely:

   [1] Claims 15 and 16 relate to a method of treatment of a disease, and the search report is made on the basis of the use of a compound in the preparation of a medicine.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/086837**

| Patent document cited in search report | Publication date (day/month/year) | Patent family member(s) | Publication date (day/month/year) |
|---|---|---|---|
| CN 103044446 A | 17 April 2013 | None | |
| CN 108658974 A | 16 October 2018 | US 2020030312 A1 | 30 January 2020 |
| | | WO 2018177296 A1 | 04 October 2018 |
| CN 108658972 A | 16 October 2018 | WO 2018177297 A1 | 04 October 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201910348714